(19) Europäisches Patentamt / European Patent Office / Office européen des brevets

(11) **EP 4 134 968 A1**

(12) **EUROPEAN PATENT APPLICATION**
published in accordance with Art. 153(4) EPC

(43) Date of publication:
**15.02.2023 Bulletin 2023/07**

(21) Application number: **21774849.0**

(22) Date of filing: **23.03.2021**

(51) International Patent Classification (IPC):
**G16H 20/60** (2018.01)      **A23L 33/00** (2016.01)
**A23L 33/10** (2016.01)

(52) Cooperative Patent Classification (CPC):
**A23L 33/40; A23L 33/125; A23L 33/15;**
**A23L 33/16; G16H 20/60**

(86) International application number:
**PCT/JP2021/012053**

(87) International publication number:
**WO 2021/193658 (30.09.2021 Gazette 2021/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.03.2020 JP 2020055099**
**05.03.2021 JP 2021035701**

(71) Applicant: NISSIN FOODS HOLDINGS CO., LTD.
**Yodogawa-ku**
**Osaka-shi, Osaka 532-8524 (JP)**

(72) Inventors:
• **ANDO, Noritaka**
**Osaka-shi Osaka 532-8524 (JP)**
• **NAKAMURA, Futoshi**
**Osaka-shi Osaka 532-8524 (JP)**
• **HIRANO, Yukio**
**Osaka-shi Osaka 532-8524 (JP)**
• **SAKURAGI, Takanori**
**Osaka-shi Osaka 532-8524 (JP)**

(74) Representative: **Mewburn Ellis LLP**
**Aurora Building**
**Counterslip**
**Bristol BS1 6BX (GB)**

(54)    **COMPLETE NUTRITIONAL FOOD PRODUCT AND PROVISION SYSTEM FOR SAME**

(57)    Different nutritional intake standards are set according to age, gender, and physical activity level in the Dietary Reference Intakes (DRIs) set in each country, for example, the "Japanese DRIs". Therefore, in order to provide meals satisfying the DRIs to a plurality of users with different attributes, for example, in a company staff canteen or the like, not only adjustment of the amounts of meals but also fine adjustment of nutritional components of meals for each category has been required. The present invention proposes a complete nutritious food product that eliminates the need for adjustment of nutrients according to each category of age, gender, and physical activity level, and enables eating of a complete nutritious food product satisfying the DRIs only by adjusting the amount (calories) of foods, and a providing system thereof. As a result, users who receive meals can easily take the complete nutritious food product, are freed from the trouble of nutrition management, and can improve their health.

FIG. 1

**Description**

Technical Field

The present invention relates to a complete nutritious food product and a providing system thereof.

Background Art

In recent years, increasing medical and long-term care costs due to aging has become a problem, and it is an important issue for reducing medical costs and spending a rich old age to extend "healthy life expectancy" in which daily life is not restricted for health reasons.

The Ministry of Health, Labour and Welfare of Japan revises the standards for the amounts of energy and nutrients that are desirably taken for maintaining and enhancing the health of the people every five years and has published a Development Study Group Report, the "Dietary Reference Intakes (DRIs) for Japanese (2020 edition)" (which will be hereinafter referred to as "Japanese DRIs") on December 24, 2019.

In the "Japanese DRIs", the estimated energy requirement (EER) (kcal/day) is calculated for each category of age, gender, and physical activity level (low (I), average (II), and high (III)), the tentative dietary goal for preventing life-style related diseases (DG) (proportion of each nutrient in the total energy intake) of each of the three major nutrients, protein, lipid, and carbohydrate is stipulated according to the EER, and any of the recommended dietary allowance (RDA), the adequate intake (AI), the tolerable upper intake level (UL), and the DG, or a combination of these is stipulated for fat-soluble vitamins such as vitamin A and vitamin D, water-soluble vitamins such as vitamin $B_1$, vitamin $B_2$, and niacin, and minerals such as sodium, potassium, calcium, magnesium, phosphorus, iron, zinc, and manganese.

The standard values such as the RDA, the AI, the UL, and the DG of each nutrient described in the "Japanese DRIs" are set for each category of gender, age, and physical activity level. Trial and error is required to calculate nutrition so that all nutrients meet the standard values. Therefore, the nutrition is often calculated so that only the three major nutrients and the amount of salt, or major vitamins and major minerals in addition to these fall within the standard values. Further, the nutrition is often calculated also so that the average of the nutritional intake of a subject within a predetermined period such as one week satisfies the standard values, instead of calculating the nutrition so that the nutrients of each meal fall within the standard values. In the "Dietary Reference Intakes (DRIs)" in the United States (which will be hereinafter referred to as "US DRIs") and the "Dietary Reference Intakes for Chinese" in China (which will be hereinafter referred to as "Chinese DRIs"), the standard values such as the RDA, the AI, the UL, and the DG of each nutrient are set for each category of gender, age, and physical activity level. However, the category settings of age and physical activity level are not necessarily the same as the category settings of Japanese DRIs.

Patent Literature 1 discloses an invention relating to a nutrition adjustment food for oral intake containing at least 2 to 75% of carbohydrate, 10% or more of protein, and 15 to 70% of lipid in terms of energy percentages and designed so that the intake of vitamins and minerals shown in the Japanese DRIs published by the Ministry of Health, Labour and Welfare of Japan falls between the required amount or more and the upper-limit amount or less, in the case of taking the EER shown in the Japanese DRIs published by the Ministry of Health, Labour and Welfare of Japan.

In Table 2 to Table 4 of Patent Literature 1, the target contents of carbohydrate, lipid, carbohydrate, vitamins, and minerals per 100 kcal and the contents (g) of the nutrients in foods as Examples 1 to 10 are stipulated, but the specific method for determining the contents is not explicitly described. For minerals and vitamins, the Japanese DRIs are satisfied by adjusting each content to the recommended standard to 50% to 250%. Further, in order to satisfy the intake of vitamins and minerals shown in the Japanese DRIs, the EER shown in the Japanese DRIs published by the Ministry of Health, Labour and Welfare of Japan need to be taken. Further, the invention disclosed in Patent Literature 1 is a nutrition adjustment food provided by dissolving powder in water as an alternative to a normal food. However, since nutrients vary depending on the recipe in a normal food, the contents of nutrients need to be adjusted so as to satisfy the Japanese DRIs each time when providing meals to a plurality of users with different attributes.

Citation List

Patent Literature

Patent Literature 1: Japanese Patent Laid-Open No. 2019-140952

Summary of Invention

Technical Problem

Thus, different nutritional intake standards are set according to age, gender, and physical activity level in the DRIs set in each country, for example, the "Japanese DRIs". Therefore, in order to provide meals satisfying the DRIs to a plurality of users with different attributes, for example, in a company staff canteen or the like, not only adjustment of the amounts of meals but also fine adjustment of nutritious components of meals for each category has been required.

Solution to Problem

The present invention has been accomplished for solving the aforementioned problems and enables providing a complete nutritious food product to a plurality of users with different ages, genders, and physical activity levels only by adjusting the amounts of meals.

The complete nutritious food product-providing system according to the present invention comprises: an information acquisition unit configured to acquire information on user attributes; a user information storage unit (user information database) configured to store the information on user attributes; a DRI-storing unit (DRI database) configured to store DRIs of a complete nutritious food product in which intake standards for first and second nutrients are set for each user attribute; and a nutrition calculation unit configured to calculate the intake standard for the first nutrient in a standard food forming the complete nutritious food product so that the standard food can satisfy the intake standard for the first nutrient of all users. Examples of the "DRIs of a complete nutritious food product in which intake standards for first and second nutrients are set" include the "Japanese DRIs", but any DRIs may be used as long as they are DRIs in which intake standards for different nutrients are set according to user attributes.

In the complete nutritious food product-providing system of the present invention, the range of the intake standard for the first nutrient in the standard food is narrower than the range of the DRI for the first nutrient in the complete nutritious food product.

In the complete nutritious food product-providing system of the present invention, the first nutrient is vitamins and/or minerals.

In the complete nutritious food product-providing system of the present invention, the second nutrient is protein, lipid, and/or carbohydrate.

In the complete nutritious food product-providing system of the present invention, the complete nutritious food product is prepared by adjusting the amount of the standard food, the energy amount of the complete nutritious food product is the EER in the category of the DRIs to which each user belongs, and the energy amount of the standard food is the EER in the category with the lowest EER among the categories of DRIs to which all users belong.

In the complete nutritious food product-providing system of the present invention, the ratio of the amount of the complete nutritious food product to the amount of the standard food is equal to the ratio of the energy amount of the complete nutritious food product to the energy amount of the standard food. That is, since the energy amount of the complete nutritious food product is the EER of each user, the amount of the complete nutritious food product is the amount of the standard food multiplied by "the EER of each user/the energy amount of the standard food".

In the complete nutritious food product-providing system of the present invention, in the case where UL of the first nutrient is set in the DRIs, a normalized nutrient amount in each category to which the user belongs is calculated by the formula: "Normalized nutrient amount = UL of first nutrient × (energy amount of standard food/EER in each category)", and the smallest normalized nutrient amount is set as the upper limit of first nutrient intake in the standard food.

In the complete nutritious food product-providing system of the present invention, in the case where AI or RDA of the first nutrient is set in the DRIs, the maximum value of the AI or the RDA of the first nutrient in each category of the DRIs is set as the lower limit of first nutrient intake in the standard food.

In the complete nutritious food product-providing system of the present invention, the upper limit of the DG of protein in the category with the lowest EER among the categories of the food reference intakes to which the users belong is set as the upper limit of protein intake in the standard food, and the RDA with the largest value among the categories to which the users belong is set as the lower limit of protein intake in the standard food that is provided to the users.

In the complete nutritious food product-providing system of the present invention, the upper limit of the DG of lipid in the category of standard energy amount with the lowest EER among the categories of the food reference intakes to which the users belong is set as the upper limit of lipid intake in the standard food, and the lower limit of the DG of lipid for the users belonging to the category of the standard energy amount is set as the lower limit of lipid intake in the standard food.

In the complete nutritious food product-providing system of the present invention, the upper limit of the DG of carbohydrate in the category of standard energy amount with the lowest EER among the categories of the food reference intakes to which the users belong is set as the upper limit of carbohydrate intake in the standard food, and the lower limit of the DG of carbohydrate for the users belonging to the category of the standard energy amount is set as the lower limit of carbohydrate intake in the standard food.

In the complete nutritious food product-providing system of the present invention, the information on user attributes includes at least information on gender, age, or physical activity level.

In the complete nutritious food product-providing system of the present invention, the vitamins include one or more of vitamin A, vitamin D, vitamin E, vitamin K, vitamin $B_1$, vitamin $B_2$, niacin, vitamin $B_6$, vitamin $B_{12}$, folic acid, pantothenic acid, biotin, and vitamin C, and the minerals include one or more of sodium, potassium, calcium, magnesium, phosphorus, iron, zinc, copper, manganese, iodine, selenium, chromium, and molybdenum.

In the complete nutritious food product-providing system of the present invention, the DRIs of the complete nutritious food product are any of the "Japanese DRIs" published by the Ministry of Health, Labour and Welfare of Japan, the US DRIs, and the Chinese DRIs.

The complete nutritious food product-providing system of the present invention further comprises a menu-selecting unit, wherein the nutrition calculation unit outputs the energy amount of the standard food, the upper or lower limit of first nutrient intake, and the upper or lower limit of second nutrient intake to the menu-selecting unit.

The complete nutritious food product according to the present invention is a complete nutritious food product nutritionally calculated by the aforementioned complete nutritious food product-providing system.

The complete nutritious food product of the present invention is composed of any one selected from the group consisting of breakfast, lunch, and supper or a combination of two or three of these.

The complete nutritious food product of the present invention is a processed food product, a cooked food product, or a dish cooked based on a specific menu.

The nutrition calculation device for a complete nutritious food product according to the present invention comprises: an information acquisition unit configured to acquire information on user attributes; a user information storage unit configured to store the information on user attributes; a DRI-storing unit configured to store DRIs of a complete nutritious food product in which intake standards for first and second nutrients are set for each user attribute; and a nutrition calculation unit configured to calculate the intake standard for the first nutrient, which is satisfied regardless of attributes of each user, in the standard food forming the complete nutritious food product.

The nutrition calculation program for a complete nutritious food product according to the present invention comprises: a step of acquiring information on user attributes; a step of storing the information on user attributes; a step of storing DRIs of the complete nutritious food product in which the intake standards for the first and second nutrients are set for each user attribute; and a step of calculating the intake standard for the first nutrient, which is satisfied regardless of attributes of each user, in the standard food forming the complete nutritious food product.

Advantageous Effects of Invention

The present invention proposes a complete nutritious food product that enables a complete nutritious food product that eliminates the need for adjustment of nutrients according to each category of age, gender, and physical activity level and satisfies the DRIs set in each country such as the "Japanese DRIs" to be provided, and a providing system, a providing device, and a providing program thereof. As a result, users who receive meals can easily take the complete nutritious food product, are freed from the trouble of nutrition management, and can improve their health without awareness. Further, businesses that provide nutritious foods can provide a complete nutritious food product to all users only by adjusting the amount of a "standard food", even if the user attributes vary, and can reduce the labor and cost of nutrition calculation.

There is a problem that, if the dietary intake, that is, the energy intake is reduced for the purpose of losing weight, the necessary nutrients cannot be taken. However, the present invention enables necessary amounts of nutrients to be taken through the intake of the "standard food".

Brief Description of Drawings

[Figure 1] Figure 1 is a block diagram illustrating the configuration of a complete nutritious food product-providing system in the present embodiment.
[Figure 2] Figure 2 is a flowchart showing a processing operation in a nutrition calculation unit of a food information-providing device in this embodiment.
[Figure 3] Figure 3 is an example of the calculation table of protein, lipid, saturated fatty acid, n-3 fatty acid, n-6 fatty acid, carbohydrate, and dietary fibers to be used in the complete nutritious food product-providing system based on the "Japanese DRIs".
[Figure 4] Figure 4 is an example of the calculation table of sodium, calcium, iron, phosphorus, magnesium, potassium, and copper to be used in the complete nutritious food product-providing system based on the "Japanese DRIs".

[Figure 5] Figure 5 is an example of the calculation table of iodine, selenium, zinc, chromium, manganese, and molybdenum to be used in the complete nutritious food product-providing system based on the "Japanese DRIs".

[Figure 6] Figure 6 is an example of the calculation table of vitamins (vitamin A, vitamin D, vitamin E, vitamin K, vitamin B 1, vitamin B2, niacin, vitamin B6, folic acid, vitamin B12, biotin, pantothenic acid, and vitamin C) to be used in the complete nutritious food product-providing system based on the "Japanese DRIs".

[Figure 7] Figure 7 is an example of the calculation table of protein, lipid, saturated fatty acid, n-3 fatty acid, n-6 fatty acid, carbohydrate, and dietary fibers to be used in the complete nutritious food product-providing system based on the US DRIs.

[Figure 8] Figure 8 is an example of the calculation table of sodium, potassium, calcium, magnesium, phosphorus, iron, zinc, and copper to be used in the complete nutritious food product-providing system based on the US DRIs.

[Figure 9] Figure 9 is an example of the calculation table of manganese, iodine, selenium, chromium, molybdenum, nickel, vanadium, boron, fluoride, and chloride to be used in the complete nutritious food product-providing system based on the US DRIs.

[Figure 10] Figure 10 is an example of the calculation table of vitamins (vitamin A, vitamin D, vitamin E, vitamin K, vitamin B 1, vitamin B2, niacin, vitamin B6, vitamin B12, folic acid, pantothenic acid, biotin, vitamin C, and choline) to be used in the complete nutritious food product-providing system based on the US DRIs.

[Figure 11] Figure 11 is an example of the calculation table of protein, lipid, saturated fatty acid, $\alpha$-linolenic acid, linoleic acid, carbohydrate, and added sugar to be used in the complete nutritious food product-providing system based on the Chinese DRIs.

[Figure 12] Figure 12 is an example of the calculation table of sodium, potassium, calcium, magnesium, phosphorus, iron, zinc, and copper to be used in the complete nutritious food product-providing system based on the Chinese DRIs.

[Figure 13] Figure 13 is an example of the calculation table of manganese, iodine, selenium, chromium, molybdenum, fluoride, and chloride to be used in the complete nutritious food product-providing system based on the Chinese DRIs.

[Figure 14] Figure 14 is an example of the calculation table of vitamins (vitamin A, vitamin D, vitamin E, vitamin K, vitamin B 1, vitamin B2, niacin, vitamin B6, vitamin B12, folic acid, pantothenic acid, biotin, vitamin C, and choline) to be used in the complete nutritious food product-providing system based on the Chinese DRIs.

[Figure 15] Figure 15 is a table showing the results of anthropometry before and after the modified food in the examples of the present invention is continuously taken for a certain period.

[Figure 16] Figure 16 is a table showing the results of analyzing the triglyceride in blood lipid before and after the modified food in the examples of the present invention is continuously taken for a certain period.

[Figure 17] Figure 17 is a table showing the results of analyzing 8-OHdG (8-hydroxy-2'-deoxyguanosine) of metabolites in blood (blood plasma) of subjects using a CE-TOFMS (capillary electrophoresis time-of-flight mass spectrometer) for blood metabolome analysis before and after the modified food in the examples of the present invention is continuously taken for a certain period.

[Figure 18] Figure 18 is a table showing the results of measuring the blood pressure before and after the modified food in the examples of the present invention is continuously taken for a certain period.

[Figure 19] Figure 19 is a table showing the results of measuring the bone density before and after the modified food in the examples of the present invention is continuously taken for a certain period.

[Figure 20] Figure 20 is a table showing the results of analyzing the intestinal flora before and after the modified food in the examples of the present invention is continuously taken for a certain period.

Description of Embodiments

Hereinafter, embodiments for implementing a complete nutritious food product-providing system according to the present invention will be described in detail with reference to the drawings. The present invention is not limited to the following embodiments and examples.

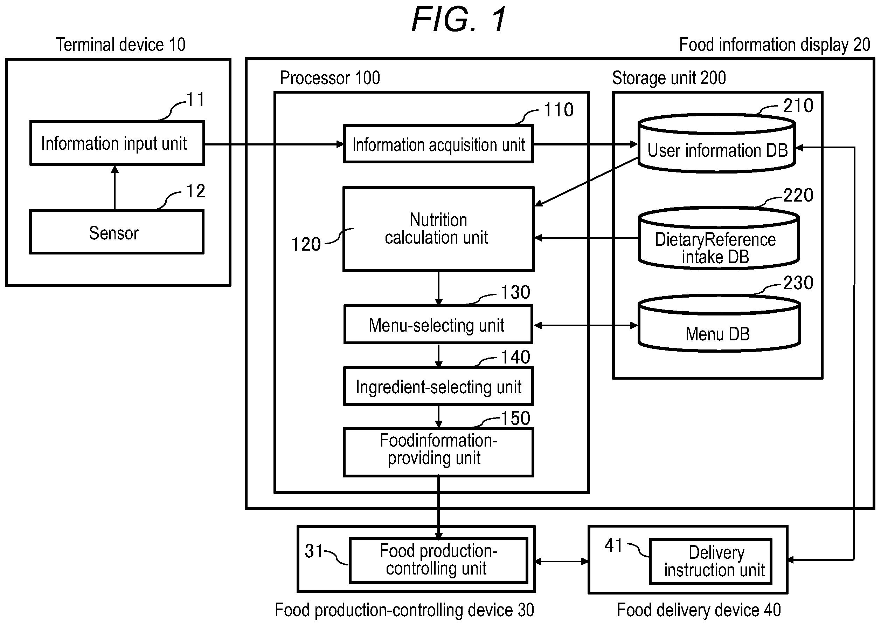

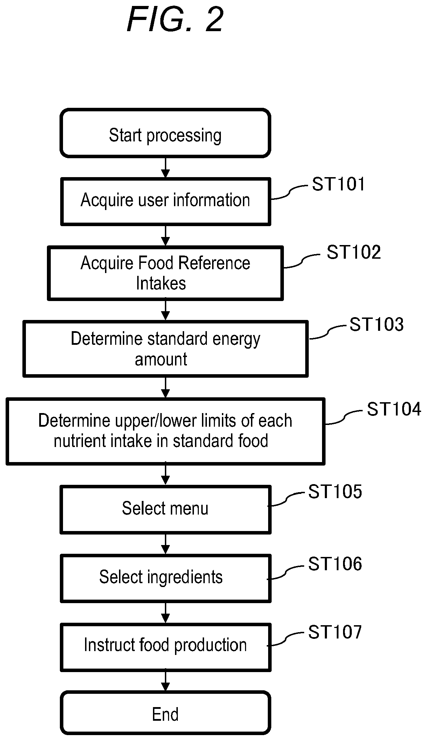

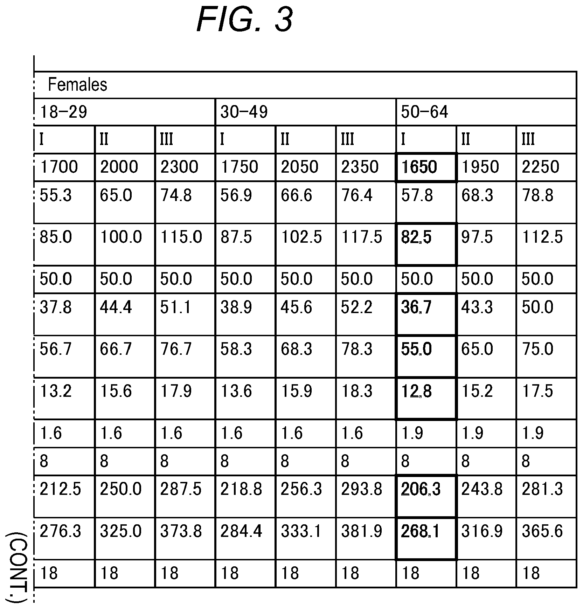

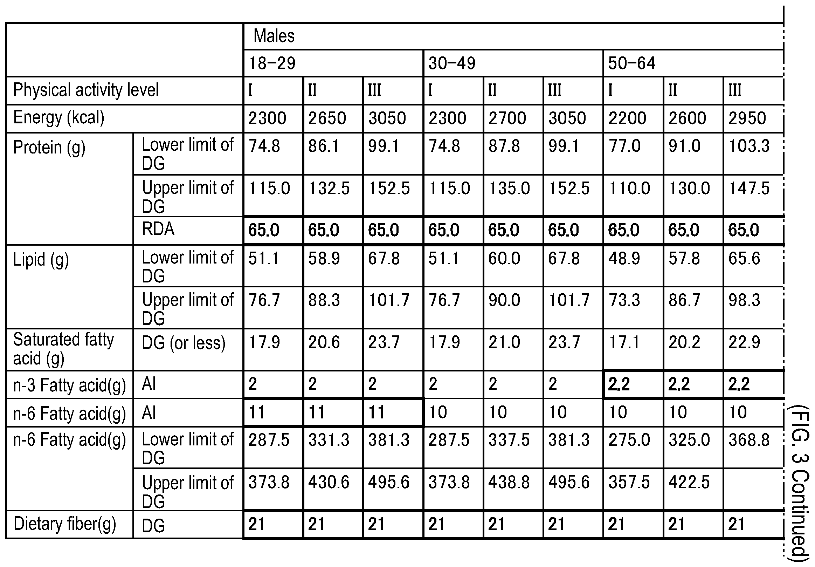

Referring to Figure 1 to Figure 14, the configuration of the complete nutritious food product-providing system in the present embodiment will be described. Figure 1 is a diagram illustrating the outline of the complete nutritious food product-providing system in this embodiment. Figure 2 is a diagram illustrating the outline of the processing operation of a nutrition calculation unit of a food information display in this embodiment. Figure 3 to Figure 14 are each a table showing an example of the nutrition calculation table to be used in the nutrition calculate system of the complete nutritious food product in this embodiment.

The "complete nutritious food product" in this embodiment refers to a nutritious food satisfying the intake standards for protein, lipid, carbohydrate, vitamins (such as vitamin A, vitamin D, vitamin E, vitamin K, vitamin B1, vitamin B2, niacin, vitamin B6, folic acid, vitamin B 12, biotin, pantothenic acid, and vitamin C), and minerals (such as sodium, calcium, iron, phosphorus, magnesium, potassium, copper, iodine, selenium, zinc, chromium, manganese, and molybdenum) stipulated, for example, in the DRIs set in each country such as the "Japanese DRIs".

The "standard food" in this embodiment refers to a nutritious food satisfying conditions (1) and (2) below.

(1) A nutritious food that enables all users who are provided with the nutritious food from businesses to satisfy intake standards for vitamins and minerals stipulated, for example, in the DRIs set in each country such as the "Japanese DRIs", regardless of their attributes (such as categories of gender, age, and physical activity level).
(2) A nutritious food that enables intake standards for PFC (protein, lipid, carbohydrate) stipulated in the DRIs to be satisfied, in addition to those for vitamins and minerals described in (1) above by taking the nutritious food in an amount corresponding to the EER (kcal/day) stipulated, for example, in the categories (age, gender, and physical activity level (low (I), average (II), and high (III)) in the "Japanese DRIs") of the DRIs set in each country to which the user belongs, such as the "Japanese DRIs".

Further, the "nutritious food" in this embodiment is a meal in which ingredients are cooked by a method such as "raw", "baking", "simmering", "steaming", and "frying", and seasoning, as required.

In the US DRIs, vitamins include choline, and minerals include nickel, vanadium, boron, fluoride, and chloride. Meanwhile, minerals do not include sodium, and lipid does not include saturated fatty acid.

As shown in Figure 1, a complete nutritious food product-providing system 1 includes a terminal device 10 of each user, a food information display 20, a food production-controlling device 30, and a food delivery device 40.

The terminal device 10 includes an information input unit 11 and a sensor 12.

Further, the food information display 20 includes a processor 100 and a storage unit 200.

Further, the food production-controlling device 30 includes a food production-controlling unit 31.

Further, the food delivery device 40 includes a delivery instruction unit 41.

The terminal device 10, the food information display 20, the food production-controlling device 30, and the food delivery device 40 each include a processor such as a CPU and a storage device that stores various programs. The processor executes various functions by executing each program.

The terminal device 10 of each user is connected to the food information display 20 so as to be communicable with each other via a communication network such as the internet. Further, the food production-controlling device 30 and the food delivery device 40 are connected to the food information display 20 via a communication network N.

The terminal device 10 includes the information input unit 11 and the sensor 12. The information transmitted from the terminal device 10 toward the food information display 20 includes personal information of the user (user ID, gender, date of birth, height, weight, and address) and information such as vital data including user activity information output from the sensor 12, desired diet foods, and desired dates of eating. The terminal device 10 of each user may be any information terminal capable of communication such as a smartphone, a tablet terminal, and a personal computer (PC).

The sensor 12 may be any sensor such as an accelerometer, a gyroscope, a magnetometer, and a GPS machine and may be built in the terminal device 10 or in an external terminal capable of communicating with the terminal device 10 such as a wearable device.

The processor 100 of the food information display 20 includes an information acquisition unit 110, a nutrition calculation unit 120, a menu-selecting unit 130, an ingredient-selecting unit 140, and a food information-providing unit 150.

The storage unit 200 of the food information display 20 includes a user information database (DB) 210, a DRI database (DB) 220, and a menu database (DB) 230.

The information acquisition unit 110 of the food information display 20 receives the information output from the terminal device 10 of each user and stores the information received in the user information database (DB) 210.

The nutrition calculation unit 120 acquires the personal information and the vital data of each user from the user information database (DB) 210 and determines the physical activity level of each user based on the vital data. Three types of physical activity levels, "low (I)", "average (II)", and "high (III)" are set in the "Japanese DRIs", but physical activity levels other than above may be defined.

Then, the categories of gender, age, and physical activity level to which each user belongs are specified, and the EER (kcal/day), the DRIs for protein, lipid, and carbohydrate, the DRIs for fat-soluble vitamins and water-soluble vitamins, and the DRIs for high and trace minerals of the applicable categories are acquired according to the DRIs (for example, the "Japanese DRIs" published by the Ministry of Health, Labour and Welfare of Japan) stored in the DRI database (DB) 220.

The nutrition calculation unit 120 specifies the lowest EER among those in the categories extracted according to the later-described processing operation and defines it as the energy amount of the standard food. The upper and lower limits of each nutrient intake in the standard food are calculated, so that a complete nutritious food product satisfying the "Japanese DRIs" can be taken by eating an adjusted amount of the standard food so as to satisfy the EER of the category to which the user belongs. As a result, users who are provided with meals can easily take the complete nutritious food product, are freed from the trouble of nutrition management, and can improve their health without awareness. Further, businesses that provide meals can provide a complete nutritious food product to each user only by adjusting the amount (calories) of the standard food and can reduce the labor and cost of nutrition calculation.

Although the "Japanese DRIs (2020 edition)" published by the Ministry of Health, Labour and Welfare are used for calculating the nutrition of foods in this embodiment, the calculation is not necessarily based on the above standards and may be, for example, based on the latest "Japanese DRIs" which are revised every five years, other nutritional intake standards, or other guidelines.

Examples of the other nutritional intake standards can include the "Dietary Reference Intakes (DRIs)", Food and Nutrition Board of the Institute of Medicine in the US, the "Dietary Reference Values for nutrients", European Food Safety Authority, 2017 in Europe, the "Dietary Reference Intakes for Chinese", Chinese Nutrition Society, 2013 in China, the "Dietary Reference Intakes for Koreans", Ministry of Health and Welfare and The Korean Nutrition Society, 2015 in Korea, the "Dietary Guidelines for Indians", National Institute of Nutrition, India, 2011 in India, and the "Nutritional Requirements during Spaceflight and on Earth", Aerospace Environmental Medicine, Vol. 45, No. 3, 75-97, 2008 for astronauts. In the case of using other nutritional intake standards or other guidelines, it is necessary to calculate the amounts of nutrients for which standards are not shown in the Japanese DRIs. The nutrition calculation for the standard food in this embodiment can be applied thereto.

The menu-selecting unit 130 refers to the menus and cooking procedures stored in the menu database (DB) 230 and selects a menu satisfying the upper and lower limits of each nutrient intake in the standard food acquired from the nutrition calculation unit 120. Even in the case of a menu that does not satisfy the upper and lower limits of each nutrient intake in the standard food, the menu can be selected by preparing several kinds of foods in advance to compensate for the lack of nutrients. The ingredient-selecting unit 140 selects ingredients (meat, vegetables, seasonings, etc.) necessary for cooking the nutritious food of the selected menu.

The food information-providing unit 150 acquires information on the EER of each user and the standard energy amount of the standard food from the nutrition calculation unit 120 and information on the cooking procedure and ingredients from the ingredient-selecting unit 140, and transmits the information to the food production-controlling unit 31 of the food production-controlling device 30.

The food production-controlling device 30 instructs a food production unit (not shown) to produce a food that is a complete nutritious food product based on the instruction from the food information display 20.

The food delivery device 40 instructs a delivery unit (not shown) to deliver the complete nutritious food product based on the instructions from the food information display 20 and the food production-controlling device 30. For example, in the case where the delivery of the complete nutritious food product to the user is not needed, such as a staff canteen, the terminal device 10 of the user may be notified of the completion of the complete nutritious food product, or the completion of the complete nutritious food product may be displayed on a monitor (not shown) installed in the staff canteen.

Here, the outline of the processing operation of the nutrition calculation unit 120 of the food information display 20 will be described with reference to Figure 2.

<Acquisition of user information and food reference intakes>

In step ST 101, the personal information and the vital data of each user are acquired from the user information database (DB) 210, and the physical activity level of each user is determined based on the vital data. Three types of physical activity levels, "low (I)", "average (II)", and "high (III)" are set in the "Japanese DRIs", but physical activity levels other than above may be used. In step ST102, the categories of gender, age, and physical activity level to which each user belongs are specified based on the gender, age, and physical activity level of each user, and the EER (kcal/day), the DRIs for protein, lipid, and carbohydrate, the DRIs for fat-soluble vitamins and water-soluble vitamins, and the DRIs for high and trace minerals in the applicable categories are acquired according to the DRIs stored in the DRI database (DB) 220. The DRIs include the "RDA", "AI", "DG", and "UL" in the "Japanese DRIs". The "target value" corresponds to the CDRR (Chronic Disease Risk Reduction Intake) in the US DRIs.

<Dietary Reference Intakes (DRIs)>

The Ministry of Health, Labour and Welfare of Japan formulates the "Japanese DRIs" every five years as the intake standards for energy and nutrients to be referred to for maintaining and enhancing the health of the people and preventing lifestyle-related diseases.

The Japanese DRIs (2020 edition) stipulate energy indicators and nutrient indicators. The nutrient indicators set an "estimated average requirement", which is met by half of the people, and "RDA", which is met by most people (97% to 98%), for the purpose of avoiding insufficient intake. In the case where sufficient scientific evidence is not available, and the estimated average requirement and the RDA cannot be set, "AI" is set as an amount sufficient to maintain a certain nutritional status, "UL" is set for the purpose of avoiding health problems due to overdose, and a "DG" is set for the purpose of preventing the onset of lifestyle-related diseases.

In order to provide a complete nutritious food product that can be eaten by all users with different genders, ages, and physical activity levels, the upper and lower limits of each nutrient intake in the standard food are calculated, as follows in this embodiment, based on the "RDA", the "AI", the "DG", and the "UL" set in the DRIs for each nutrient in each country. This enables each user to be provided with a complete nutritious food product satisfying the DRIs of each country by adjusting the amount of the standard food according to the EER of the user. In order to satisfy the DRIs for vitamins and minerals of all users, the range of intake standards stipulated by the upper and lower limits of each nutrient intake for minerals and vitamins in the standard food is narrower than the range of intake standards stipulated by the upper-limit (UL) and the lower-limit (RDA, AI, or DG) for minerals and vitamins in the DRIs of each country such as the "Japanese DRIs".

Further, the aforementioned concept is also applicable to the DRIs of each country such as the US DRIs and the Chinese DRIs.

<Determination of standard energy amount>

In step ST103, the standard energy amount of the standard food is determined as follows.

According to "Japanese DRIs (2020 edition)" published by the Ministry of Health, Labour and Welfare of Japan, the EER (kcal/day) for males and females aged 18 to 64 years is 1650 kcal/day to 3050 kcal/day as shown in Figure 3. In this embodiment, it is premised that the complete nutritious food product is provided to users belonging to the categories stipulated by the age, gender, and physical activity level shown in Figure 3.

The lowest EER is 1650 kcal/day for females aged 50 to 64 years with physical activity level I (low), and the highest is 3050 kcal/day for males aged 18 to 29 years and 30 to 49 years with physical activity level III (high). In the case of providing the complete nutritious food product to users belonging to the categories stipulated by the age, gender, and physical activity level shown in Figure 3, the EER of 1650 kcal/day in the category with the lowest EER is set as the energy amount of the standard food (which will be hereinafter referred to as "standard energy amount"). The standard food satisfies the nutritional intake standards for minerals and vitamins of all users shown in Figure 3 and enables each user to be provided with the complete nutritious food product satisfying the "Japanese DRIs" by adjusting the amount of the standard food according to the EER of the user.

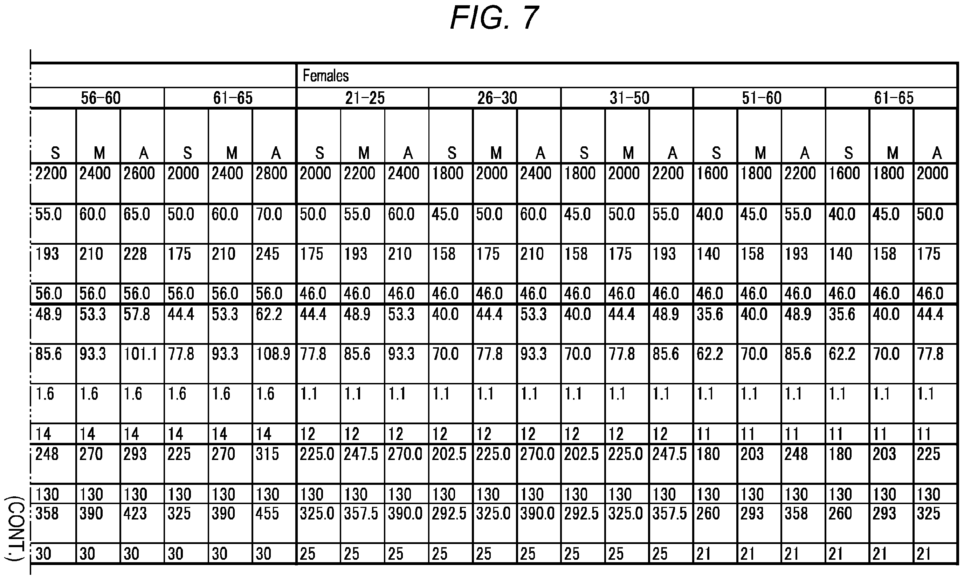

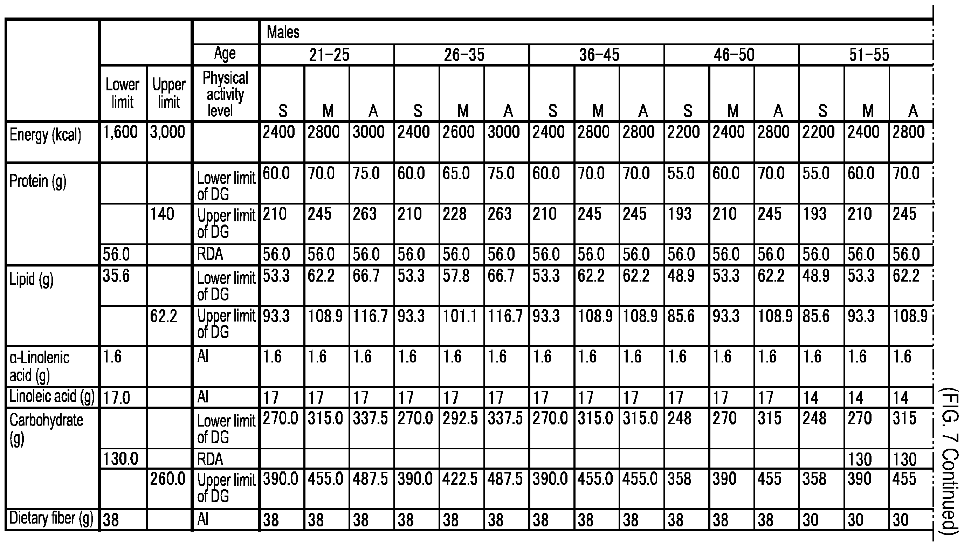

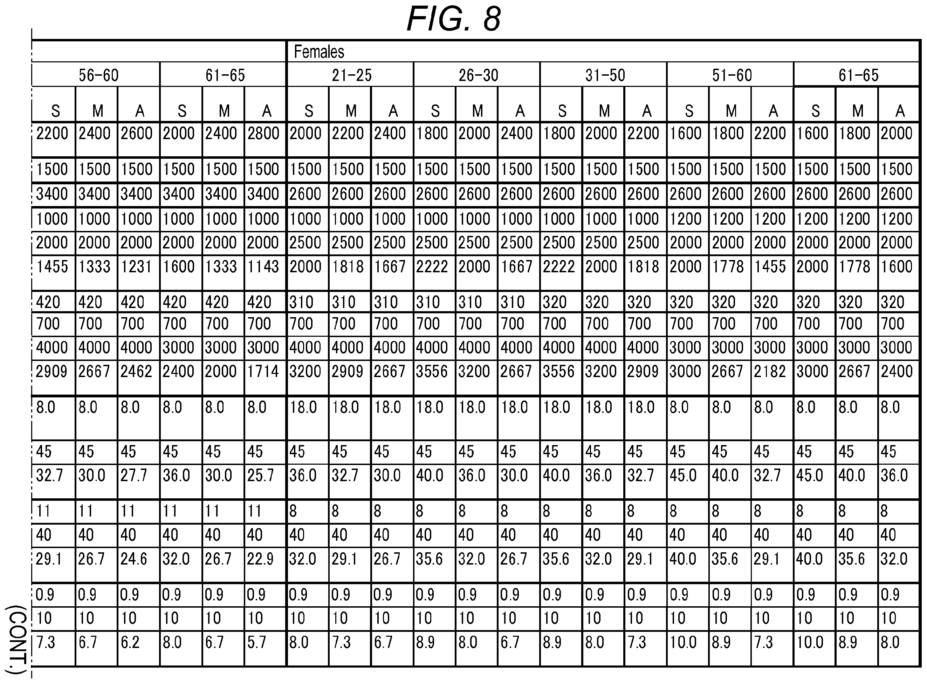

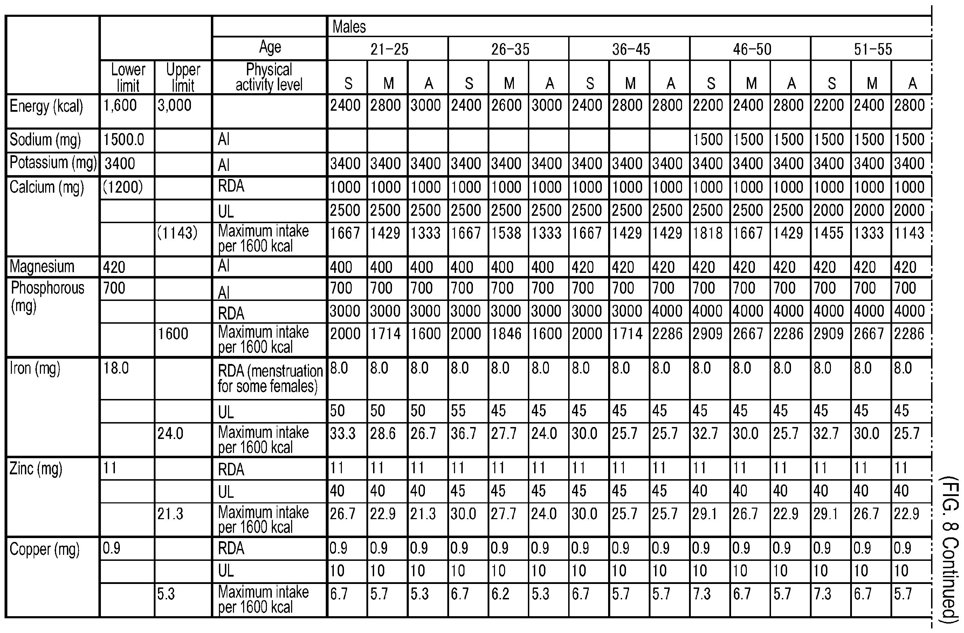

The US DRIs provide formulas for calculating the EER based on age, gender, height, weight, and physical activity level, but do not stipulate the EER for each category of age, gender, and physical activity level, unlike the Japanese DRIs. Therefore, the EERs (EER) for the reference positions of Americans stipulated by the US Department of Health and Human Services (HHS) are applied to the US DRIs in this embodiment and are referred to as "US DRIs". As shown in Figure 7, the EER (kcal/day) for males and females aged 21 to 65 years is 1600 kcal/day to 3000 kcal/day, and the energy amount of the standard food is 1600 kcal/day. The standard food satisfies the nutritional intake standards for minerals and vitamins of all users shown in Figure 8 to Figure 10 and enables each user to be provided with a complete nutritious food product satisfying the US DRIs by adjusting the amount of the standard food according to the EER of the user. In Figure 7 to Figure 10, the physical activity level S means "Sedentary", M means "Moderately active", and A means "Active".

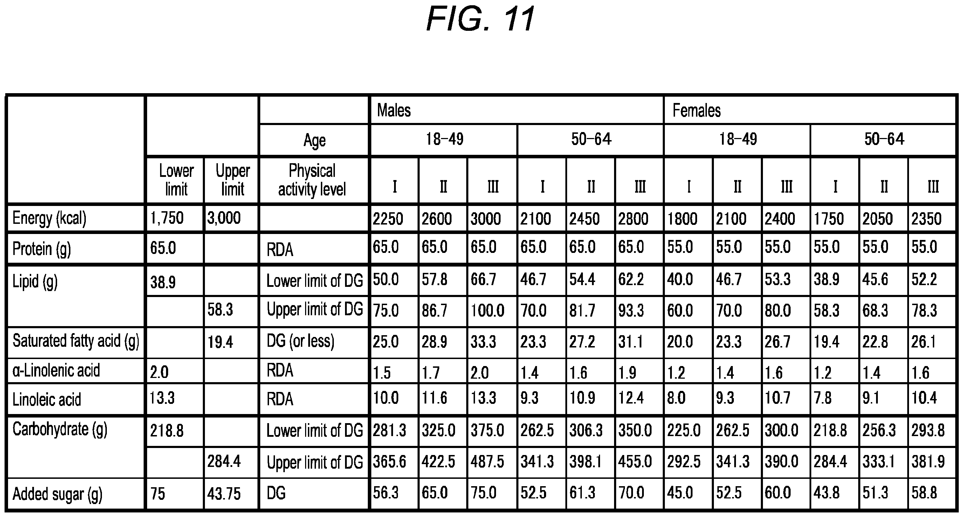

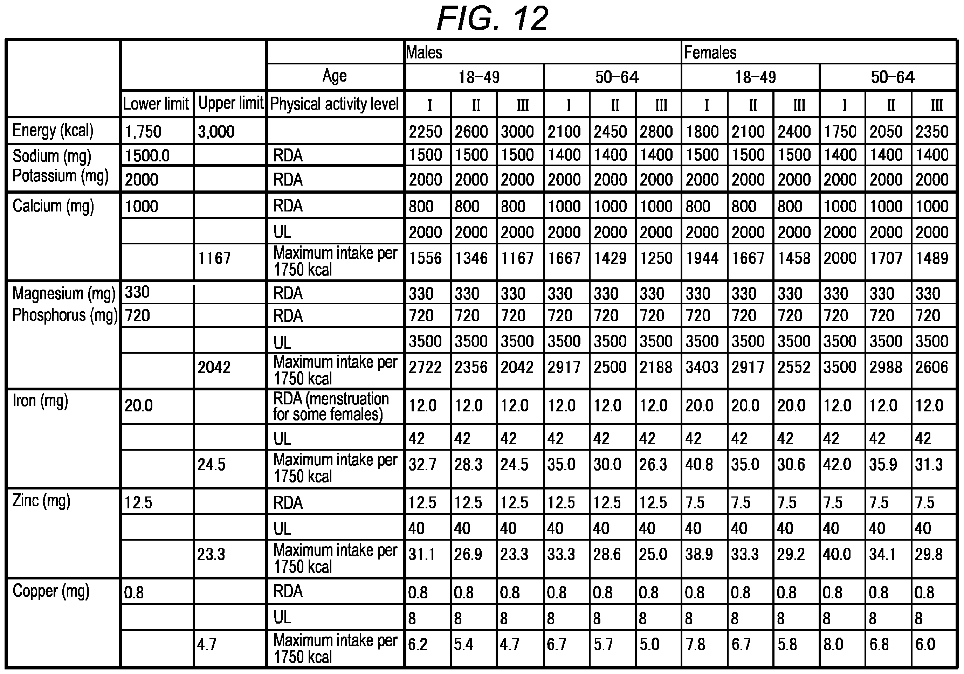

According to the Chinese DRIs (Chinese Nutrition Society) published by the Chinese Nutrition Society, the EER (kcal/day) for males and females aged 18 to 65 years is 1750 kcal/day to 3000 kcal/day as shown in Figure 11, and the energy amount of the standard food is 1750 kcal/day. The standard food satisfies the nutritional intake standards for minerals and vitamins of all users shown in Figure 12 to Figure 14 and enables each user to be provided with a complete nutritious food product satisfying the Chinese DRIs by adjusting the amount of the standard food according to the EER of the user.

The aforementioned setting of the standard energy amount is an example, and similar complete nutrition can be provided also using other DRIs such as the meal balance guide by the Ministry of Agriculture, Forestry and Fisheries of Japan, the dietary guideline by the US Department of Health and Human Services, and the guideline for Chinese diet by the Chinese Nutrition Society. In addition, different DRIs may be used in combination. The DRIs of Japan, the US, and China are mentioned above as examples, but a standard food and a complete nutritious food product can be provided based on the DRIs set by other countries or regions, or international organizations such as the World Health Organization (WHO). Further, it is possible to provide a complete nutritious food product for special environments such as DRIs for space foods. In addition, it is also possible to provide a complete nutritious food product by formulating the DRIs that are limited to specific ages and purposes, such as "standards for the elderly", "standards for males or females for the purpose of dieting ", "standards for both males and females" based on the DRIs formulated.

<Determination of upper/lower limit of each nutrient, menu selection, ingredient selection, and instruction of food production>

The upper and lower limits of each nutrient intake in the standard food in this embodiment is calculated in step ST104, a menu satisfying the upper and lower limits of each nutrient intake in the standard food is selected in step ST105, and ingredients (such as meat, vegetables, and seasonings) necessary for cooking the nutritious food of the selected menu are selected based on the EER of each user and the standard energy amount of the standard food in step ST106. Further, information on the EER of each user, the standard energy amount of the standard food, the cooking procedure, and ingredients is transmitted to the food production-controlling unit 31 of the food production-controlling device 30 in step ST107, and the food production-controlling device 30 instructs the food production unit to produce a food product that is a complete nutritious food product based on the instruction from the food information display 20.

Here, the upper and lower limits of each nutrient intake in the standard food are determined in step ST104, as follows.

1. Protein

The upper and lower limits of protein intake in the standard food of this embodiment are calculated, as follows.

According to the "Japanese DRIs", the DRI (DG) of protein for males and females aged 18 to 64 years is set to 13 to 20% or 14% to 20% energy/day. Further, the RDA is set to 65 g/day for males and 50 g/day for females. For those with low energy intake required, the lower limit may fall below the RDA, but the lower limit is desirably to be the RDA or more even in such a case.

Since the EER differs depending on each category of gender, age, and physical activity level, the upper and lower limits of the DG of protein when calculated in terms of g/day differ depending on each category of the DRIs as shown in Figure 3. Therefore, the upper limit of the DG of protein in the category of the "standard energy amount" with the lowest EER is set as the upper limit of protein intake in the standard food, so that users with different genders, ages, and physical activity levels can eat the complete nutritious food product satisfying the Japanese DRIs. Further, the largest RDA among the categories to which the users belong is set as the lower limit of protein intake in the standard food.

In the example of Figure 3, the upper limit of the DG of protein in the category with the lowest EER (females aged 50 to 64 years with physical activity level I (low)) is set as the upper limit of protein intake in the standard food. In this case, since 1 g of protein has 4 kcal of energy in the body, the upper limit of protein intake in the standard food is 82.5 g/day (= 1650 kcal/4 kcal × 20%). Meanwhile, the lower limit of protein intake is 65 g, which is the largest RDA in all categories.

In the case of using the aforementioned "US DRIs" as DRIs, the upper limit of the macronutrient distribution range of protein in the category with the lowest EER (females aged 60 to 65, Sedentary) is set as the upper limit of protein intake in the standard food, as shown in Figure 7. In this case, since 1 g of protein has 4 kcal of energy in the body, the upper limit of protein intake in the standard food is 140 g/day (= 1600 kcal/4 kcal × 35%). Meanwhile, the lower limit of protein intake is 56 g, which is the largest RDA in all categories.

In the Chinese DRIs, only the lower limit of protein intake is set as the RDA, as shown in Figure 11, and the lower limit of intake is 65 g, which is the largest RDA in all categories.

2. Lipid

The upper and lower limits of intake of lipid, and saturated fatty acid, n-6 fatty acid, n-3 fatty acid, etc. included in the lipid in the standard food of this embodiment are calculated, as follows.

<Lipid>

According to the "Japanese DRIs", the DRI (DG) of lipid for males and females aged 18 to 64 years is set to 20% to 30% energy/day. Since the EER differs depending on each category of gender, age, and physical activity level, the upper and lower limits of the DG of lipid differ depending on the category, as shown in Figure 3. Therefore, the upper limit of the DG of lipid in the category of the "standard energy amount" with the lowest EER is set as the upper limit of lipid intake in the standard food, so that users with different genders, ages, and physical activity levels can eat the complete nutritious food product satisfying the Japanese DRIs.

Meanwhile, since a RDA of lipid is not set in the "Japanese DRIs", the lower limit of the DG of lipid for the users belonging to the category of the "standard energy amount" is set as the lower limit of lipid intake in the standard food. In the example of Figure 3, the upper and lower limits of the DG of lipid in the category with the lowest EER (category of females aged 50 to 64 years with physical activity level I (low)) are set as the upper and lower limits of lipid intake in the standard food. In this case, since 1 g of lipid has 9 kcal of energy in the body, the upper limit of lipid intake is 55.0 g/day (= 1650 kcal/9 kcal × 30%), and the lower limit of lipid intake is 36.7 g/day (= 1650 kcal/9 kcal × 20%), in the standard food.

According to the US DRIs, the DRI for lipid for males and females aged 21 to 65 years (Acceptable Macronutrient Distribution Ranges) is set to 20% to 35% energy/day. Since the EER differs depending on each category of gender, age, and physical activity level, the upper limit of the macronutrient distribution range and the lower limit of the macronutrient distribution range of lipid differ depending on the category, as shown in Figure 7. Therefore, the upper limit of the macronutrient distribution range of lipid in the category of "standard energy amount" with the lowest EER is set as the upper limit of lipid intake in the standard food, so that users with different genders, ages, and physical activity levels can eat the complete nutritious food product satisfying the "US DRIs".

Further, since the US DRIs do not set the RDA of lipid, the lower limit of the macronutrient distribution range of lipid of users belonging to the category of the "standard energy amount" is set as the lower limit of lipid intake in the standard food. In the example of Figure 7, the upper limit of the macronutrient distribution range and the lower limit of the macronutrient distribution range of lipid in the category with the lowest EER (category of females aged 61 to 65 years with physical activity level S (Sedentary)) are set as the upper and lower limits of lipid intake in the standard food. In this case, since 1 g of lipid has 9 kcal of energy in the body, the upper limit of lipid intake is 62.2 g/day (= 1600 kcal/9 kcal × 35%), and the lower limit of lipid intake is 35.6/day (= 1600 kcal/9 kcal × 20%), in the standard food.

In the Chinese DRIs, the DRI for lipid for males and females aged 18 to 64 years (macronutrient distribution range) is set to 20% to 30% energy/day. Since the EER differs depending on each category of gender, age, and physical activity level, the upper limit of the macronutrient distribution range and the lower limit of the macronutrient distribution range of lipid differ depending on the category, as shown in Figure 11. Therefore, the upper limit of the macronutrient distribution range of lipid in the category of "standard energy amount" with the lowest EER is set as the upper limit of lipid intake in the standard food, so that users with different genders, ages, and physical activity levels can eat the complete nutritious food product satisfying the Chinese DRIs. Meanwhile, since a RDA of lipid is not set in the Chinese DRIs, the lower limit of the macronutrient distribution range of lipid of users belonging to the category of the "standard energy amount" is set as the lower limit of lipid intake in the standard food. In the example of Figure 11, the upper limit of the macronutrient distribution range and the lower limit of the macronutrient distribution range of lipid in the category with the lowest EER (category of females aged 50 to 64 years with physical activity level I (low)) are set as the upper and lower limits of lipid intake in the standard food. In this case, since 1 g of lipid has 9 kcal of energy in the body, the upper limit of lipid intake is 58.3 g/day (= 1750 kcal/9 kcal × 35%), and the lower limit of lipid intake is 38.9/day (= 1750 kcal/9 kcal × 20%), in the standard food.

<Saturated fatty acid>

According to the "Japanese DRIs", the DRI (DG) for saturated fatty acid for males and females aged 18 years or older is set to 7% energy/day. Since the EER differs depending on each category of gender, age, and physical activity level, the upper limit and the lower limit differ depending on the category when the upper limit of saturated fatty acid is calculated in terms of g/day, as shown in Figure 3.

Therefore, the upper limit of the DG of saturated fatty acid in the category of "standard energy amount" with the lowest EER is set as the upper limit of saturated fatty acid intake in the standard food, so that users with different genders, ages, and physical activity levels can eat the complete nutritious food product satisfying the Japanese DRIs. In the example of Figure 3, the upper limit of the DG of saturated fatty acid in the category with the lowest EER (category of females aged 50 to 64 years with physical activity level I (low)) is set as the upper limit of saturated fatty acid intake in the standard food. In this case, since 1 g of saturated fatty acid has 9 kcal of energy in the body, the upper limit of saturated fatty acid intake in the standard food is 12.8 g/day (= 1650/9 × 0.07).

In the US DRIs, a standard value is not set for the amount of saturated fatty acid, and the upper limit of intake is not set in the standard food based on the US DRIs.

In the Chinese DRIs, the macronutrient distribution range of saturated fatty acid for males and females aged 18 to 64 years is set to less than 10% of the energy intake. In the example of Figure 11, the upper limit of the macronutrient distribution range of saturated fatty acid in the category with the lowest EER (category of females aged 50 to 64 years with physical activity level I (low)) is set as the upper limit of saturated fatty acid intake in the standard food. In this case, since 1 g of saturated fatty acid has 9 kcal of energy in the body, the upper limit of saturated fatty acid intake in the standard food is 19.4 g/day (= 1750/9 × 0.1).

<n-3 Fatty acid and n-6 fatty acid>

According to the "Japanese DRIs", the DRIs for n-3 fatty acid and n-6 fatty acid are set as AIs, where n-3 fatty acid for those aged 18 to 29 years is set to 2.0 g/day for males and 1.6 g/day for females, for those aged 30 to 49 years, 2.0 g/day for males and 1.6 g/day for females, and for those aged 50 to 64 years, 2.2 g/day for males and 1.9 g/day for females, and n-6 fatty acid for those aged 18 to 29 years is set to 11 g/day for males and 8 g/day for females, for those aged 30 to 49 years, 10 g/day for males and 8 g/day for females, and for those aged 50 to 64 years, 10 g/day for males and 8 g/day for females.

In this embodiment, the maximum values of the AIs of n-3 fatty acid and n-6 fatty acid in each category (2.2 g/day for n-3 fatty acid and 11 g/day for n-6 fatty acid) are set as the lower limits of intake in the standard food in this embodiment, so that all users can take the AIs of n-3 fatty acid and n-6 fatty acid by taking the standard food.

In the US DRIs, AI of linoleic acid representing n-6 fatty acid and AI of α-linolenic acid representing n-3 fatty acid are set. In the case of using the US DRIs as DRIs, the maximum values of the AIs of linoleic acid and α-linolenic acid in each category (17 g/day for linoleic acid and 1.6 g/day for α-linolenic acid) are set as the lower limits of intake in the standard food, so that all users can take the AIs of n-6 fatty acid and n-3 fatty acid by taking the standard food, as shown in Figure 7.

In the Chinese DRIs, a ratio of the amount of linoleic acid representing n-6 fatty acid to the energy intake is set as RDA. Further, a ratio of the amount of α-linolenic acid representing n-3 fatty acid to the energy intake is set as RDA. In the case of using the Chinese DRIs as DRIs, the maximum values of the RDAs of linoleic acid and α-linolenic acid in each category (13.3 g/day for linoleic acid and 2.0 g/day for α-linolenic acid) are set as the lower limits of intake in the standard food, so that all users can take the RDA of n-6 fatty acid and the RDA of n-3 fatty acid by taking the standard food.

3. Carbohydrate

The upper and lower limits of intake of carbohydrate and dietary fibers contained in the carbohydrate in the standard food of this embodiment are calculated, as follows.

<Carbohydrate>

According to the "Japanese DRIs", the DRI (DG) for carbohydrate for males and females aged 18 to 64 years is set to 50% to 65% energy/day. Since the EER differs depending on each category of gender, age, and physical activity level, the upper and lower limits of the DG of carbohydrate differ depending on the category, as shown in Figure 3.

Therefore, the upper limit of the DG of carbohydrate in the category of "standard energy amount" with the lowest EER is set as the upper limit of carbohydrate intake in the standard food, so that users with different genders, ages, and physical activity levels can eat the complete nutritious food product satisfying the Japanese DRIs.

Meanwhile, since "RDA" of carbohydrate is not set, the lower limit of the DG of carbohydrate for the users belonging to the category of the "standard energy amount" is set as the lower limit of carbohydrate intake in the standard food. In the example of Figure 3, the upper and lower limits of the DG of carbohydrate for the users belonging to the category with the lowest EER (category of females aged 50 to 64 years with physical activity level I (low)) are set as the upper and lower limits of carbohydrate intake in the standard food. In this case, since 1 g of carbohydrate has 4 kcal of energy in the body, the upper limit of carbohydrate intake is 268.1 g/day (= 1650/4 $\times$ 0.65), and the lower limit of carbohydrate intake is 206.3 g/day (= 1650/4 $\times$ 0.5).

In the US DRIs, the RDA of carbohydrate for males and females aged 21 to 65 years is set to 130 g/day, and the RDA, 130 g/day, is set as the lower limit of intake in the case of using the US DRIs as DRIs, as shown in Figure 7. Meanwhile, the upper limit of the macronutrient distribution range is set to 65% energy/day in the US DRIs. Since the EER differs depending on each category of gender, age, and physical activity level, the upper limit of the macronutrient distribution range of carbohydrate differs depending on the category, as shown in Figure 7. Therefore, the upper limit of the macronutrient distribution range of carbohydrate in the category of "standard energy amount" with the lowest EER is set as the upper limit of carbohydrate intake in the standard food, so that users with different genders, ages, and physical activity levels can take the complete nutritious food product satisfying the "US DRIs". In the example of Figure 7, the upper limit of the macronutrient distribution range of carbohydrate for the users belonging to the category with the lowest EER (category of females aged 61 to 65 with physical activity level S (Sedentary)) is set as the upper limit of carbohydrate intake in the standard food. In this case, since 1 g of carbohydrate has 4 kcal of energy in the body, the upper limit of carbohydrate intake is 260 g/day (= 1600/4 $\times$ 0.65).

In the Chinese DRIs, the macronutrient distribution range of carbohydrate for males and females aged 18 to 64 years is set to 50% to 65% energy/day. Since the EER differs depending on each category of gender, age, and physical activity level, the upper and lower limits of the macronutrient distribution range of carbohydrate differ depending on the category, as shown in Figure 11. Therefore, the upper limit of the macronutrient distribution range of carbohydrate in the category of "standard energy amount" with the lowest EER is set as the upper limit of carbohydrate intake in the standard food, so that users with different genders, ages, and physical activity levels can eat the complete nutritious food product satisfying the Chinese DRIs. Meanwhile, since RDA of carbohydrate is not set in the Chinese DRIs, the lower limit of the macronutrient distribution range of carbohydrate for the users belonging to the category of the "standard energy amount" is set as the lower limit of carbohydrate intake in the standard food. In the example of Figure 11, the upper and lower limits of the macronutrient distribution range of carbohydrate in the category with the lowest EER (category of females aged 50 to 64 years with physical activity level I (low)) are set as the upper and lower limits of carbohydrate intake in the standard food. In this case, since 1 g of carbohydrate has 4 kcal of energy in the body, the upper limit of carbohydrate intake is 284.4 g/day (= 1750 kcal/4 kcal $\times$ 65%), and the lower limit of carbohydrate intake is 218.8/day (= 1750 kcal/4 kcal $\times$ 50%), in the standard food.

<Dietary fibers>

According to the "Japanese DRIs", a DG is set as the DRI for dietary fibers, and the DG for those aged 18 to 64 years is set to 21 g/day or more for males and 18 g/day or more for females. In this embodiment, the maximum value (21 g/day) of the DG of dietary fibers in each category is set as the lower limit of intake in the standard food in this embodiment, so that all users can take the DG of dietary fibers by taking a standard food in an amount adjusted.

In the US DRIs, AI is set as the DRI for dietary fibers. Therefore, in the case of using the US DRIs as DRIs, the maximum value (38 g/day) of the AI of dietary fibers in each category is set as the lower limit of intake in the standard food, so that all users of males and females aged 21 to 65 years can take the AI of dietary fibers by taking a standard food in an amount adjusted.

In the Chinese DRIs, a standard value is not set for dietary fibers, the standard for China is not set also in the present invention.

<Added sugar>

In the Chinese DRIs, the macronutrient distribution range of added sugar for males and females aged 18 to 64 years is set to less than 10% of the energy intake. Therefore, in the case of using the Chinese DRIs as the DRIs, the upper limit of the macronutrient distribution range of added sugar in the category with the lowest EER (category of females aged 50 to 64 years with physical activity level I (low)) is set as the upper limit of added sugar intake in the standard food, in the example of Figure 11. In this case, since 1 g of added sugar has 9 kcal of energy in the body, the upper limit of added sugar intake in the standard food is 43.8 g/day (= 1750/4 $\times$ 0.1).

4. Sodium (salt equivalent)

The upper and lower limits of sodium (salt equivalent) intake in the standard food of this embodiment are calculated, as follows.

According to the "Japanese DRIs", the DRI (DG) of sodium (salt equivalent) for males and females aged 18 to 64 years is set to less than 7.5 g/day for males and less than 6.5 g/day for females in terms of salt equivalent.

The amount of the standard food in this embodiment is adjusted according to the attributes of each user. Therefore, the salt equivalent contained in an adjusted amount of the standard food needs to be below the DRI in the category with high EER. Thus, a normalized salt equivalent in each category is calculated by the following formula, and the smallest normalized salt equivalent is set as the upper limit of salt equivalent intake in the standard food.

$$\text{Normalized salt equivalent} = \text{DG of salt equivalent} \times (\text{standard energy amount/EER in each category})$$

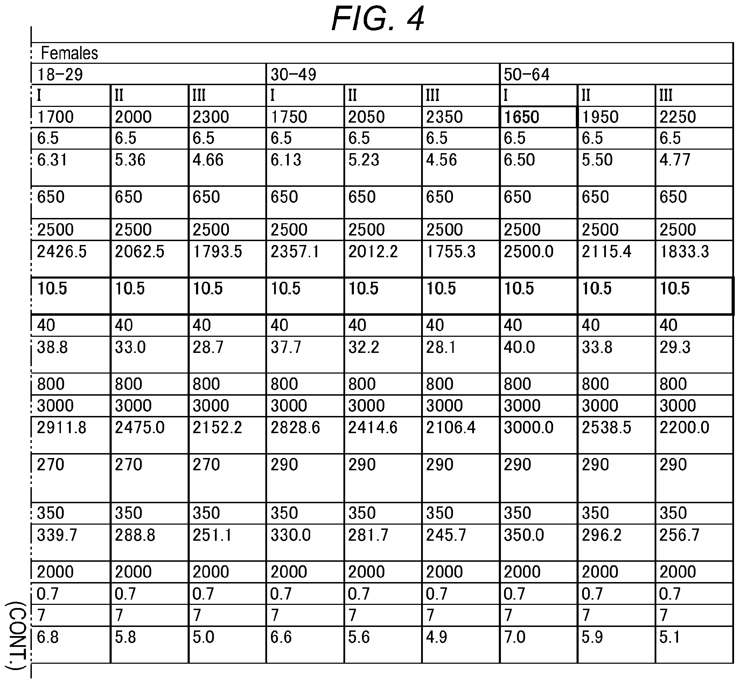

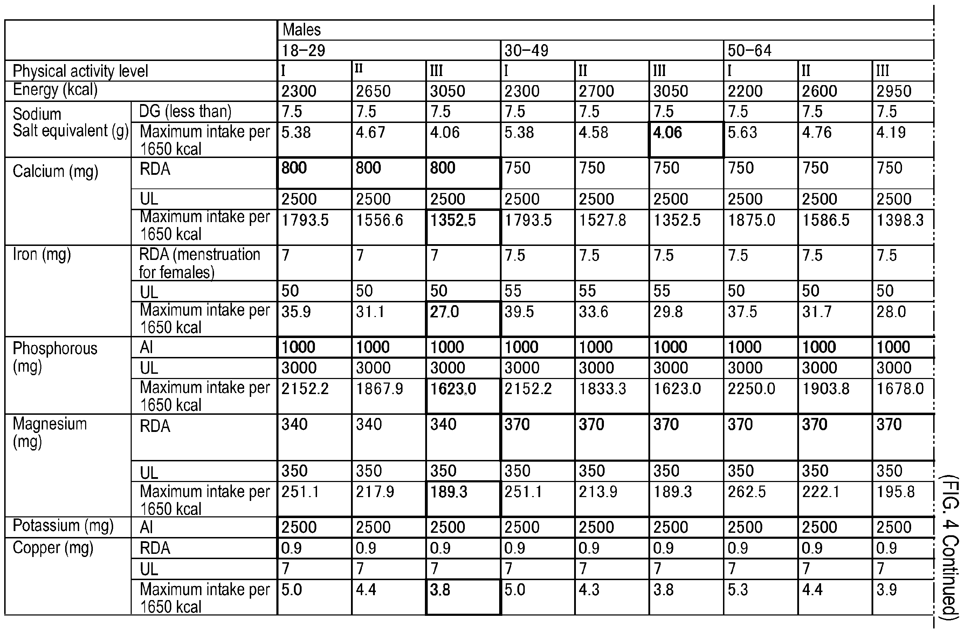

In the example of Figure 4, since the EER in the category of males aged 18 to 29 years with physical activity level III (high) is higher than other categories, the salt equivalent that can be added to the standard food is 4.06 g/day (7.5 g $\times$ 1650 kcal/3050 kcal), which is smallest. Therefore, the upper limit of sodium intake in the standard food is set to less than 4.06 g/day in terms of salt equivalent.

Since sodium in the standard food also affects the taste of the diet, it is also possible to flexibly consider this amount. For example, according to the smart meal standards examined and certified by a consortium such as The Japanese Society of Nutrition and Dietetics, The Nutrition and Food service Management, and The Japanese Society of Hypertension, sodium (salt equivalent) can be "proper" (less than 3.0 g) or "firm" (less than 3.5 g). These standards may be followed. That is, it is possible to adopt a method of setting the salt equivalent for breakfast, lunch, and supper in a day to less than 3.0 g in the case of 450 to 650 kcal and to less than 3.5 g in the case of 650 to 850 kcal to adjust the salt equivalent to be less than the upper limit. Further, other original standards may be set. For example, in the case of less than 450 kcal, the salt equivalent may be less than 2.5 g. Further, in the case of 850 kcal or more, the salt equivalent may be less than 4.0 g.

Further, as other original standards, it is possible to adopt a method of setting the salt intake for breakfast, lunch, and supper in a day to 1.7 g or less in breakfast, less than 3.0 g in lunch, and less than 3.5 g in supper to adjust the salt equivalent to be upper limit. In this way, it is possible to set an upper limit of sodium (salt equivalent) intake.

Further, standards other than the aforementioned setting methods may be followed, or the standards that can be eaten deliciously and reduce salt reasonably may be set.

Within the aforementioned range, the upper and lower limits of sodium (salt equivalent) intake in the "standard food" in this embodiment may be set.

In the US DRIs, the AI of sodium for males and females aged 21 to 65 years is set to 1500 mg/day. Therefore, in the case of using the US DRIs as DRIs, the AI, 1500 mg/day, is set as the lower limit of intake in the standard food.

In the Chinese DRIs, the RDA of sodium for males and females aged 18 to 64 years is set to 1400 mg/day or 1500 mg/day. Therefore, in the case of using the Chinese DRIs as DRIs, the RDA, 1500 mg/day, is set as the lower limit of intake in the standard food.

5. Calcium

The upper and lower limits of calcium intake in the standard food of this embodiment are calculated, as follows.
According to the "Japanese DRIs", RDA and a UL are set for those aged 18 to 64 years as DRIs for calcium. The amount of the standard food in this embodiment is adjusted according to the attributes of each user. Therefore, calcium contained in an adjusted amount of the standard food needs to be below the DRI in the categories with high EERs. Thus, a normalized calcium amount is calculated by the following formula in each category, and the smallest normalized calcium amount is set as the upper limit of calcium intake in the standard food.

$$\text{Normalized calcium amount} = \text{UL of calcium} \times (\text{standard energy amount/EER in each category})$$

Further, the maximum value (800 mg/day) of the RDA of calcium in each category is set as the lower limit of calcium intake in the standard food, so that all users can take the RDA or more of calcium. In the example of Figure 4, the EER in the category of males aged 18 to 29 years with physical activity level III (high) is higher than other categories, the calcium that can be added to the standard food is 1352.5 mg/day (= 2500 mg $\times$ 1650 kcal/3050 kcal), which is smallest. Therefore, this value is set as the upper limit of calcium intake in the standard food.
In the US DRIs, the RDA of calcium for males and females aged 21 to 65 years is set to 1000 to 1200 mg/day. However, if 1200 mg/day that is the maximum value is set as the lower limit of intake in the standard food, the upper limit of calcium for the users belonging to the category of males aged 51 to 65 years physical activity level A (Active) calculated by the aforementioned formula of the normalized calcium amount is 1143 mg, which is below the lower limit of intake, as shown in Figure 8. Therefore, the upper and lower limits of calcium intake are not set. However, it is possible to set the upper and lower limits of intake by limiting the age and gender.
In the Chinese DRIs, the RDA of calcium for males and females aged 18 to 64 years is set to 800 to 1000 mg/day. Therefore, in the case of using the Chinese DRIs as DRIs, the maximum value of the RDA of 1000 mg/day is set as the lower limit of intake in the standard food. Meanwhile, a normalized calcium amount in each category is calculated by the aforementioned formula for the UL, and the smallest normalized calcium amount is set as the upper limit of calcium intake in the standard food. In the example of Figure 12, the upper limit of calcium in the category of males aged 18 to 49 years with physical activity level III (high), 1167 mg/day, is set as the upper limit of calcium intake in the standard food.

6. Iron

The upper and lower limits of iron intake in the standard food of this embodiment are calculated, as follows.
According to the "Japanese DRIs", RDA and UL are set for those aged 18 to 64 years as DRIs for iron. The amount of the standard food in this embodiment is adjusted according to the attributes of each user. Therefore, the iron contained in an adjusted amount of the standard food needs to be below the DRI in the categories with high EERs. Thus, a normalized iron amount is calculated by the following formula in each category, and the smallest normalized iron amount is set as the upper limit of iron intake in the standard food.

$$\text{Normalized iron amount} = \text{UL of iron} \times (\text{standard energy amount/EER in each category})$$

Further, the maximum value (10.5 mg/day) of the RDA of iron in each category is set as the lower limit of iron intake in the standard food, so that all users can take the RDA or more of iron. In the example of Figure 4, since the EER in the category of males aged 18 to 29 years with physical activity level III (high) is higher than other categories, the iron that can be added to the standard food is 27.0 mg/day (= 50 mg $\times$ 1650 kcal/3050 kcal), which is smallest. Therefore, this value is set as the upper limit of iron intake in the standard food.
In the US DRIs, the RDA of iron for males and females aged 21 to 65 years is set to 8 to 18 mg/day. Therefore, in the case of using the US DRIs as DRIs, the maximum value of the RDA, 18 mg/day, is set as the lower limit of intake in the standard food. Meanwhile, a normalized iron amount in each category is calculated by the aforementioned formula for the UL, and the smallest normalized iron amount is set as the upper limit of iron intake in the standard food. In the example of Figure 8, the upper limit of iron in the category of males aged 26 to 35 years with physical activity level A (Active), 24 mg/day, is set as the upper limit of iron intake in the standard food.

In the Chinese DRIs, the RDA of iron for males and females aged 18 to 64 years is set to 12 to 20 mg/day. Therefore, in the case of using the Chinese DRIs as DRIs, the maximum value of the RDA of 20 mg/day is set as the lower limit of intake in the standard food. Meanwhile, a normalized iron amount in each category is calculated by the aforementioned formula for the UL, and the smallest normalized iron amount is set as the upper limit of iron intake in the standard food. In the example of Figure 12, the upper limit of iron in the category of males aged 18 to 49 years with physical activity level III (high), 24.5 mg/day, is set as the upper limit of iron intake in the standard food.

7. Phosphorus

The upper and lower limits of phosphorus intake in the standard food of this embodiment are calculated, as follows.

According to the "Japanese DRIs", AI and UL for those aged 18 to 64 years are set as DRIs for phosphorus. The amount of the standard food in this embodiment is adjusted according to the attributes of each user. Therefore, the phosphorus contained in an adjusted amount of the standard food needs to be below the DRI in the categories with high EERs. Thus, a normalized phosphorus amount in each category is calculated by the following formula, and the smallest normalized phosphorus amount is set as the upper limit of phosphorus intake in the standard food.

$$\text{Normalized phosphorus amount} = \text{UL of phosphorus} \times (\text{standard energy amount/EER in each category})$$

Further, the maximum value (1000 mg/day) of the AI of phosphorus in each category is set as the lower limit of phosphorus intake in the standard food, so that all users can take the AI or more of phosphorus. In the example of Figure 4, since the EER in the category of males aged 18 to 29 years with physical activity level III (high) is higher than other categories, the phosphorus that can be added to the standard food is 1623.0 mg/day (= 3000 mg × 1650 kcal/3050 kcal), which is smallest. Therefore, this value is set as the upper limit of phosphorus intake in the standard food.

In the US DRIs, the RDA of phosphorus for males and females aged 21 to 65 years is set to 700 mg/day. Therefore, in the case of using the US DRIs as DRIs, the RDA, 700 mg/day, is set as the lower limit of intake in the standard food. Meanwhile, a normalized phosphorus amount in each category is calculated by the aforementioned formula for the UL, and the smallest normalized phosphorus amount is set as the upper limit of phosphorus intake in the standard food. In the example of Figure 8, the upper limit of phosphorus in the category of males aged 21 to 35 years with physical activity level A (Active), 1600 mg/day, is set as the upper limit of phosphorus intake in the standard food.

In the Chinese DRIs, the RDA of phosphorus for males and females aged 18 to 64 years is set to 720 mg/day. Therefore, in the case of using the Chinese DRIs as DRIs, 720 mg/day is set as the lower limit of intake in the standard food. Meanwhile, a normalized phosphorus amount in each category is calculated by the aforementioned formula for the UL, and the smallest normalized phosphorus amount is set as the upper limit of phosphorus intake in the standard food. In the example of Figure 12, the upper limit of phosphorus in the category of males aged 18 to 49 years with physical activity level III (high), 2042 mg/day, is set as the upper limit of phosphorus intake in the standard food.

8. Magnesium

The upper and lower limits of magnesium intake in the standard food of this embodiment are calculated, as follows.

According to the "Japanese DRIs", RDA and UL of intake from non-normal foods for those aged 18 to 64 years are set as DRIs for magnesium. The amount of the standard food in this embodiment is adjusted according to the attributes of each user. Therefore, the magnesium to be added to an adjusted amount of the standard food needs to be below the DRI in the categories with high EERs. Thus, a normalized magnesium amount in each category is calculated by the following formula, and the smallest normalized magnesium amount is set as the upper limit of magnesium intake in the standard food.

$$\text{Normalized magnesium amount} = \text{UL of magnesium} \times (\text{standard energy amount/EER in each category})$$

Further, the maximum value (370 mg/day) of the RDA of magnesium in each category is set as the lower limit of magnesium intake in the standard food, so that all users can take the RDA or more of magnesium. In the example of Figure 4, since the EER in the category of males aged 18 to 29 years with physical activity level III (high) is higher than other categories, the magnesium that can be added to the standard food is 189.3 mg/day (350 mg × 1650 kcal/3050 kcal), which is smallest. Therefore, the upper limit of magnesium intake to be added to the standard food is set to less than 189.3 mg/day. According to the "Japanese DRIs", the UL is set only for the intake from non-normal foods, as described above.

In the US DRIs, the RDA of magnesium for males and females aged 21 to 65 years is set to 320 to 420 mg/day. Therefore, in the case of using the US DRIs as DRIs, the RDA, 420 mg/day, is set as the lower limit of intake in the standard food.

In the Chinese DRIs, the RDA of magnesium for males and females aged 18 to 64 years is set to 330 mg/day. Therefore, in the case of using the Chinese DRIs as DRIs, the RDA, 330 mg/day, is set as the lower limit of intake in the standard food.

9. Potassium

The upper and lower limits of potassium intake in the standard food of this embodiment are calculated, as follows.

According to the "Japanese DRIs", AI for those aged 18 to 64 years is set as DRI for potassium.

In this embodiment, the maximum value (2500 mg/day) of the AI of potassium in each category is set as the lower limit of intake in the standard food, so that all users can take the AI or more of potassium.

In the US DRIs, the AI of potassium for males and females aged 21 to 65 years is set to 2600 to 3400 mg/day. Therefore, in the case of using the US DRIs as DRIs, the AI, 3400 mg/day, is set as the lower limit of intake in the standard food.

In the Chinese DRIs, the RDA of potassium for males and females aged 18 to 64 years is set to 2000 mg/day. Therefore, in the case of using the Chinese DRIs as DRIs, 2000 mg/day is set as the lower limit of intake in the standard food.

10. Copper

The upper and lower limits of copper intake in the standard food of this embodiment are calculated, as follows.

According to the "Japanese DRIs", RDA and UL for those aged 18 to 64 years are set as DRIs for copper.

The amount of the standard food in this embodiment is adjusted according to the attributes of each user. Therefore, the copper contained in an adjusted amount of the standard food needs to be below the DRI in the categories with high EERs. Thus, a normalized copper amount is calculated by the following formula in each category, and the smallest normalized copper amount is set as the upper limit of copper intake in the standard food.

$$\text{Normalized copper amount} = \text{UL of copper} \times (\text{standard energy amount/EER in each category})$$

Further, the maximum value (0.9 mg/day) of the RDA of copper in each category is set as the lower limit of copper intake in the standard food, so that all users can take the RDA or more of copper. In the example of Figure 4, since the EER in the category of males aged 18 to 29 years with physical activity level III (high) is higher than other categories, the copper that can be added to the standard food is 3.79 mg/day (7 mg × 1650 kcal/3050 kcal), which is smallest. Therefore, this value is set as the upper limit of copper intake in the standard food.

In the US DRIs, the RDA of copper for males and females aged 21 to 65 years is set to 0.9 mg/day. Therefore, in the case of using the US DRIs as DRIs, the RDA, 0.9 mg/day, is set as the lower limit of intake in the standard food. Meanwhile, a normalized copper amount in each category is calculated by the aforementioned formula for the UL, and the smallest normalized copper amount is set as the upper limit of copper intake in the standard food. In the example of Figure 8, the upper limit of copper in the category of males aged 21 to 35 years with physical activity level A (Active), 5.3 mg/day, is set as the upper limit of copper intake in the standard food.

In the Chinese DRIs, the RDA of copper for males and females aged 18 to 64 years is set to 0.8 mg/day. Therefore, in the case of using the Chinese DRIs as DRIs, the RDA, 0.8 mg/day, is set as the lower limit of intake in the standard food. Meanwhile, a normalized copper amount in each category is calculated by the aforementioned formula for the UL, and the smallest normalized copper amount is set as the upper limit of copper intake in the standard food. In the example of Figure 12, the upper limit of copper in the category of males aged 18 to 49 years with physical activity level III (high), 4.7 mg/day, is set as the upper limit of copper intake in the standard food.

11. Iodine

The upper and lower limits of iodine intake in the standard food of this embodiment are calculated, as follows.

According to the "Japanese DRIs", RDA and UL are set for those aged 18 to 64 years as DRIs for iodine.

The amount of the standard food in this embodiment is adjusted according to the attributes of each user. Therefore, the iodine contained in an adjusted amount of the standard food needs to be below the DRI in the categories with high EERs. Thus, a normalized iodine amount in each category is calculated by the following formula, and the smallest normalized iodine amount is set as the upper limit of iodine intake in the standard food.

$$\text{Normalized iodine amount} = \text{UL of iodine} \times (\text{standard energy amount}/\text{EER in each category})$$

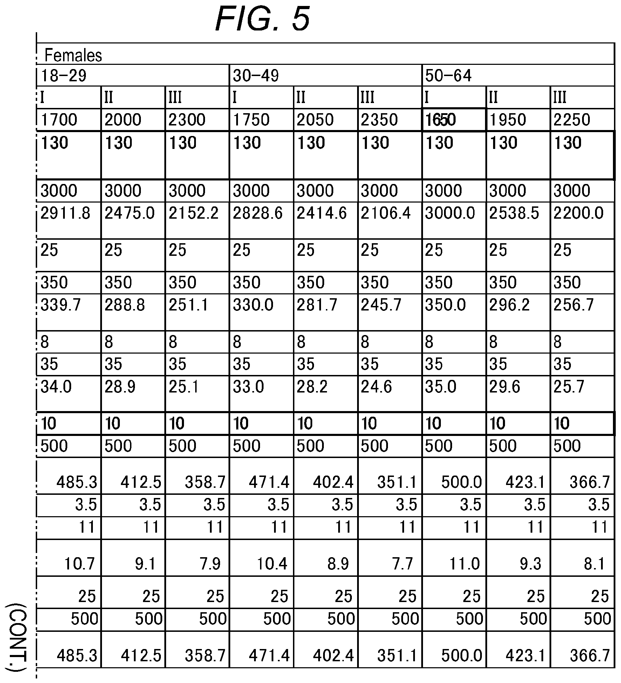

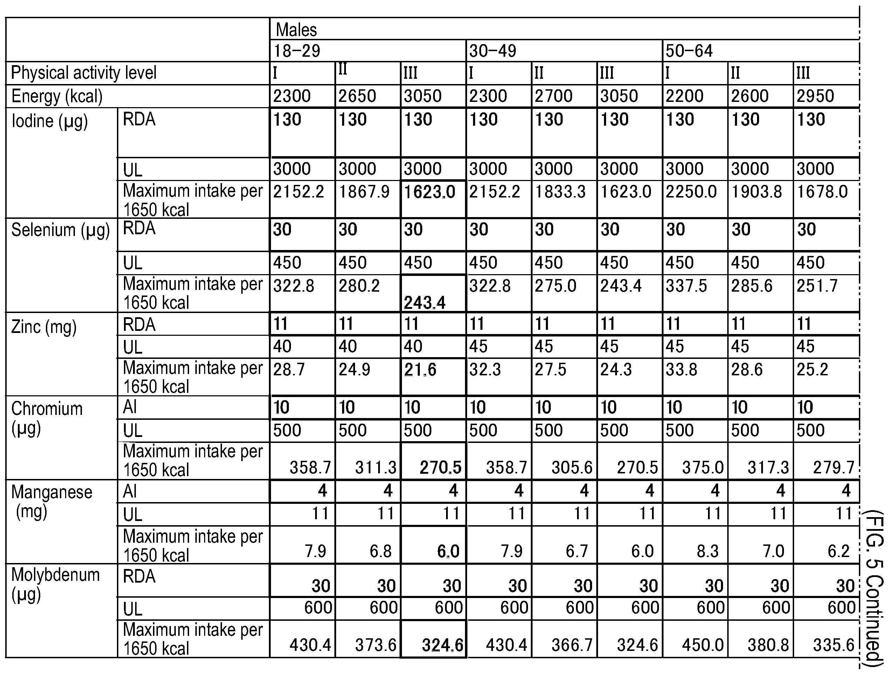

Further, the maximum value (130 μg/day) of the RDA of iodine in each category is set as the lower limit of iodine intake in the standard food, so that all users can take the RDA or more of iodine. In the example of Figure 5, since the EER in the category of males aged 18 to 29 years with physical activity level III (high) is higher than other categories, the iodine that can be added to the standard food is 1623 μg/day (3000 μg × 1650 kcal/3050 kcal), which is smallest. Therefore, this value is set as the upper limit of copper intake in the standard food.

Since the UL in the "Japanese DRIs" is set for habitual intake, it is allowed to exceed the UL intermittently, and the same applies to this embodiment.

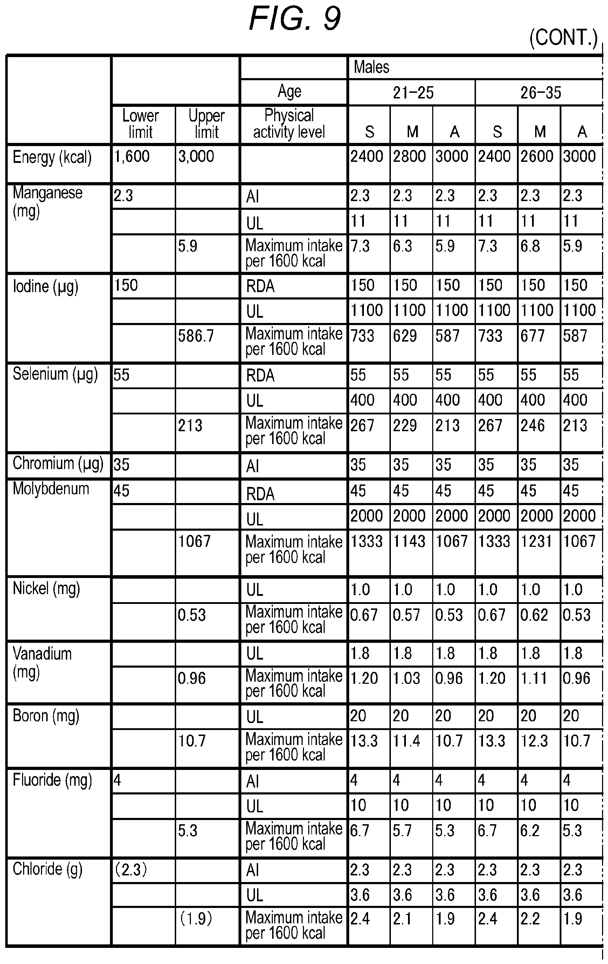

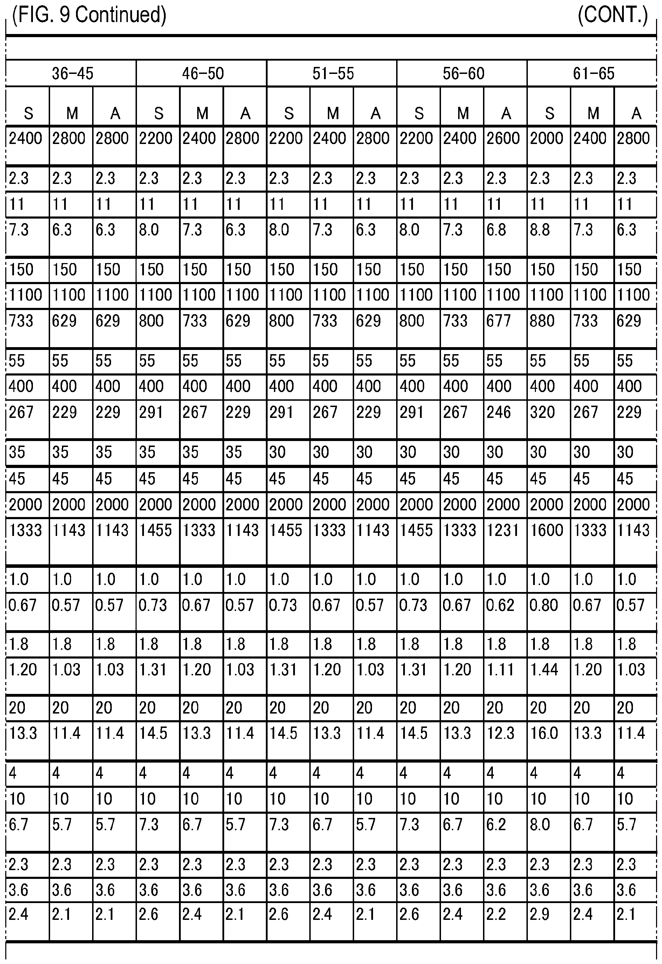

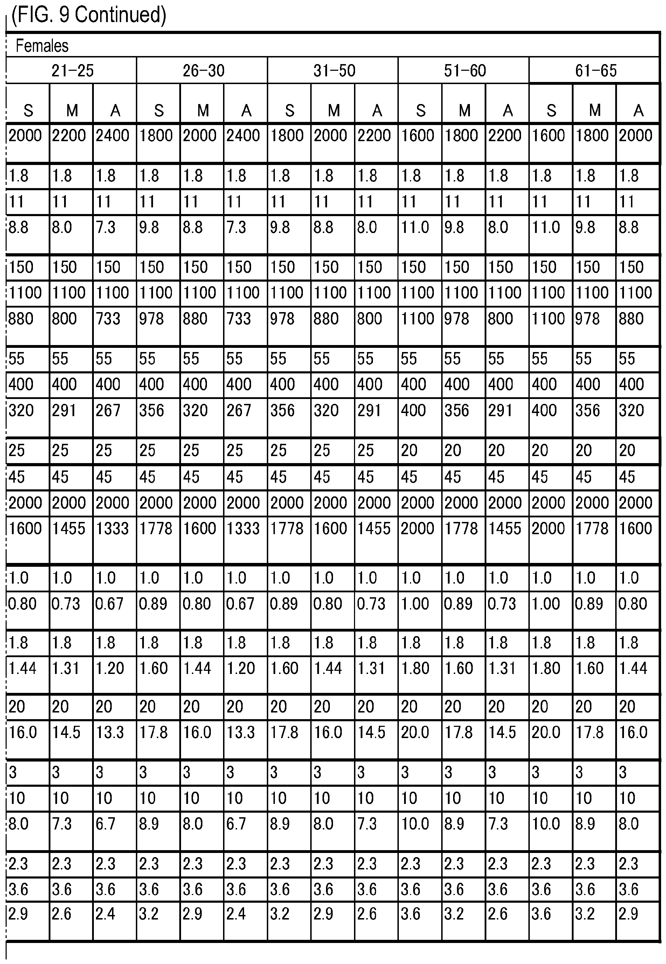

In the US DRIs, the RDA of iodine for males and females aged 21 to 65 years is set to 150 μg/day. Therefore, in the case of using the US DRIs as DRIs, the RDA, 150 μg/day, is set as the lower limit of intake in the standard food. Meanwhile, a normalized iodine amount in each category is calculated by the aforementioned formula for the UL, and the smallest normalized iodine amount is set as the upper limit of iodine intake in the standard food. In the example of Figure 9, the upper limit of iodine in the category of males aged 21 to 35 years with physical activity level A (Active), 586.7 μg/day, is set as the upper limit of iodine intake in the standard food.

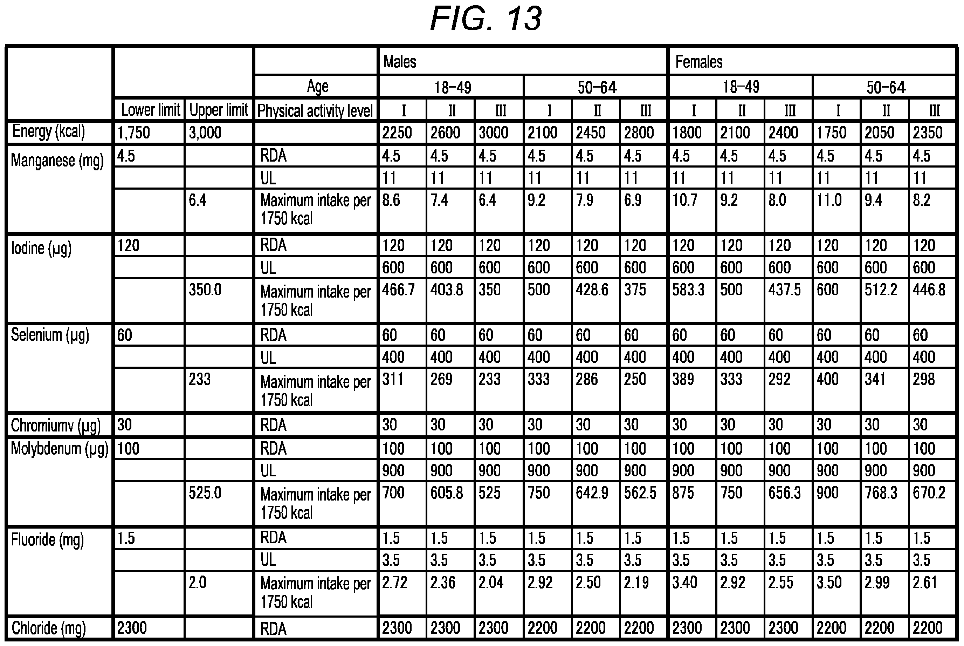

In the Chinese DRIs, the RDA of iodine for males and females aged 18 to 64 years is set to 120 μg/day. Therefore, in the case of using the Chinese DRIs as DRIs, the RDA, 120 μg/day, is set as the lower limit of intake in the standard food. Meanwhile, a normalized iodine amount in each category is calculated by the aforementioned formula for the UL, and the smallest normalized iodine amount is set as the upper limit of iodine intake in the standard food. In the example of Figure 13, the upper limit of iodine in the category of males aged 18 to 49 years with physical activity level III (high), 350 μg/day, is set as the upper limit of iodine intake in the standard food.

12. Selenium

The upper and lower limits of selenium intake in the standard food of this embodiment are calculated, as follows.

According to the "Japanese DRIs", RDA and UL are set for those aged 18 to 64 years as DRIs for selenium. The amount of the standard food in this embodiment is adjusted according to the attributes of each user. Therefore, the selenium contained in an adjusted amount of the standard food needs to be below the DRI in the categories with high EERs. Thus, a normalized selenium amount in each category is calculated by the following formula, and the smallest normalized selenium amount is set as the upper limit of selenium intake in the standard food.

$$\text{Normalized selenium amount} = \text{UL of selenium} \times (\text{standard energy amount}/\text{EER in each category})$$

Further, the maximum value (30 μg/day) of the RDA of selenium in each category is set as the lower limit of selenium intake in the standard food, so that all users can take the RDA or more of selenium. In the example of Figure 5, since the EER in the category of males aged 18 to 29 years with physical activity level III (high) is higher than other categories, the selenium that can be added to the standard food is 243.4 μg/day (450 μg × 1650 kcal/3050 kcal), which is smallest. Therefore, this value is set as the upper limit of selenium intake in the standard food.

In the US DRIs, the RDA of selenium for males and females aged 21 to 65 years is set to 55 μg/day. Therefore, in the case of using the US DRIs as DRIs, the RDA, 55 μg/day, is set as the lower limit of intake in the standard food. Meanwhile, a normalized selenium amount in each category is calculated by the aforementioned formula for the UL, and the smallest normalized selenium amount is set as the upper limit of selenium intake in the standard food. In the example of Figure 9, the upper limit of selenium in the category of males aged 21 to 35 years with physical activity level A (Active), 213 μg/day, is set as the upper limit of selenium intake in the standard food.

In the Chinese DRIs, the RDA of selenium for males and females aged 18 to 64 years is set to 60 μg/day. Therefore, in the case of using the Chinese DRIs as DRIs, the RDA, 60 μg/day, is set as the lower limit of intake in the standard food. Meanwhile, a normalized selenium amount in each category is calculated by the aforementioned formula for the UL, and the smallest normalized selenium amount is set as the upper limit of selenium intake in the standard food. In the example of Figure 13, the upper limit of selenium in the category of males aged 18 to 49 years with physical activity level III (high), 233.3 μg/day, is set as the upper limit of selenium intake in the standard food.

13. Zinc

The upper and lower limits of zinc intake in the standard food of this embodiment are calculated, as follows.

According to the "Japanese DRIs", RDA and UL are set for those aged 18 to 64 years as DRIs for zinc.

The amount of the standard food in this embodiment is adjusted according to the attributes of each user. Therefore, the zinc contained in an adjusted amount of the standard food needs to be below the DRI in the categories with high EERs. Thus, a normalized zinc amount is calculated by the following formula in each category, and the smallest normalized zinc amount is set as the upper limit of zinc intake in the standard food.

$$\text{Normalized zinc amount} = \text{UL of zinc} \times (\text{standard energy amount/EER in each category})$$

Further, the maximum value (11 mg/day) of the RDA of zinc in each category is set as the lower limit of zinc intake in the standard food, so that all users can take the RDA or more of zinc. In the example of Figure 5, since the EER in the category of males aged 18 to 29 years with physical activity level III (high) is higher than other categories, the zinc that can be added to the standard food is 21.6 mg/day (40 mg × 1650 kcal/3050 kcal), which is smallest. Therefore, this value is set as the upper limit of zinc intake in the standard food.

In the US DRIs, the RDA of zinc for males and females aged 21 to 65 years is set to 8 to 11 mg/day. Therefore, in the case of using the US DRIs as DRIs, the maximum value of the RDA, 11 mg/day, is set as the lower limit of intake in the standard food. Meanwhile, a normalized zinc amount in each category is calculated by the aforementioned formula for the UL, and the smallest normalized zinc amount is set as the upper limit of zinc intake in the standard food. In the example of Figure 8, the upper limit of zinc in the category of males aged 21 to 25 years with physical activity level A (Active), 21.3 mg/day, is set as the upper limit of zinc intake in the standard food.

In the Chinese DRIs, the RDA of zinc for males and females aged 18 to 64 years is set to 7.5 to 12.5 mg/day. Therefore, in the case of using the Chinese DRIs as DRIs, the RDA, 12.5 mg/day, is set as the lower limit of intake in the standard food. Meanwhile, a normalized zinc amount in each category is calculated by the aforementioned formula for the UL, and the smallest normalized zinc amount is set as the upper limit of zinc intake in the standard food. In the example of Figure 12, the upper limit of zinc in the category of males aged 18 to 49 years with physical activity level III (high), 23.3 mg/day, is set as the upper limit of zinc intake in the standard food.

14. Chromium

The upper and lower limits of chromium intake in the standard food of this embodiment are calculated, as follows.

According to the "Japanese DRIs", AI and UL for those aged 18 to 64 years are set as DRIs for chromium.

The amount of the standard food in this embodiment is adjusted according to the attributes of each user. Therefore, the chromium contained in an adjusted amount of the standard food needs to be below the DRI in the categories with high EERs. Thus, a normalized chromium amount is calculated by the following formula in each category, and the smallest normalized chromium amount is set as the upper limit of chromium intake in the standard food.

$$\text{Normalized chromium amount} = \text{UL of chromium} \times (\text{standard energy amount/EER in each category})$$

Further, the maximum value (10 μg/day) of the AI of chromium in each category is set as the lower limit of chromium intake in the standard food, so that all users can take the AI or more of chromium. In the example of Figure 5, since the EER in the category of males aged 18 to 29 years with physical activity level III (high) is higher than other categories, the chromium that can be added to the standard food is 270.5 μg/day (500 μg × 1650 kcal/3050 kcal), which is smallest. Therefore, this value is set as the upper limit of chromium intake in the standard food.

In the US DRIs, the AI of chromium for males and females aged 21 to 65 years is set to 25 to 35 μg/day. Therefore, in the case of using the US DRIs as DRIs, the AI, 35 μg/day, is set as the lower limit of intake in the standard food.

In the Chinese DRIs, the RDA of chromium for males and females aged 18 to 64 years is set to 30 μg/day. Therefore, in the case of using the Chinese DRIs as DRIs, 30 μg/day is set as the lower limit of intake in the standard food.

15. Manganese

The upper and lower limits of manganese intake in the standard food of this embodiment are calculated, as follows.

According to the "Japanese DRIs", AI and UL for those aged 18 to 64 years are set as DRIs for manganese.

The amount of the standard food in this embodiment is adjusted according to the attributes of each user. Therefore, the manganese contained in an adjusted amount of the standard food needs to be below the DRI in the categories with high EERs. Thus, a normalized manganese amount is calculated by the following formula in each category, and the smallest normalized manganese amount is set as the upper limit of manganese intake in the standard

food.

$$\text{Normalized manganese amount} = \text{UL of manganese} \times (\text{standard energy amount/EER in each category})$$

Further, the maximum value (4 mg/day) of the AI of manganese in each category is set as the lower limit of manganese intake in the standard food, so that all users can take the AI or more of manganese. In the example of Figure 5, since the EER in the category of males aged 18 to 29 years with physical activity level III (high) is higher than other categories, the manganese that can be added to the standard food is 6.0 mg/day (11 mg $\times$ 1650 kcal/3050 kcal), which is smallest. Therefore, this value is set as the upper limit of manganese intake in the standard food.

In the US DRIs, the AI of manganese for males and females aged 21 to 65 years is set to 1.8 to 2.3 mg/day. Therefore, in the case of using the US DRIs as DRIs, the maximum value, 2.3 mg/day, is set as the lower limit of intake in the standard food. Meanwhile, a normalized manganese amount in each category is calculated by the aforementioned formula for the UL, and the smallest normalized manganese amount is set as the upper limit of manganese intake in the standard food. In the example of Figure 9, the upper limit of manganese in the category of males aged 21 to 35 years with physical activity level A (Active), 5.9 mg/day, is set as the upper limit of manganese intake in the standard food.

In the Chinese DRIs, the RDA of manganese for males and females aged 18 to 64 years is set to 4.5 mg/day. Therefore, in the case of using the Chinese DRIs as DRIs, 4.5 mg/day is set as the lower limit of intake in the standard food. Meanwhile, a normalized manganese amount in each category is calculated by the aforementioned formula for the UL, and the smallest normalized manganese amount is set as the upper limit of manganese intake in the standard food. In the example of Figure 13, the upper limit of manganese in the category of males aged 18 to 49 years with physical activity level III (high), 6.4 mg/day, is set as the upper limit of manganese intake in the standard food.

16. Molybdenum

The upper and lower limits of molybdenum intake in the standard food of this embodiment are calculated, as follows.

According to the "Japanese DRIs", RDA and UL are set for those aged 18 to 64 years as DRIs for molybdenum. The amount of the standard food in this embodiment is adjusted according to the attributes of each user. Therefore, the molybdenum contained in an adjusted amount of the standard food needs to be below the DRI in the categories with high EERs. Thus, a normalized molybdenum amount is calculated by the following formula in each category, and the smallest normalized molybdenum amount is set as the upper limit of molybdenum intake in the standard food.

$$\text{Normalized molybdenum amount} = \text{UL of molybdenum} \times (\text{standard energy amount/EER in each category})$$

Further, the maximum value (30 μg/day) of the RDA of molybdenum in each category is set as the lower limit of molybdenum intake in the standard food, so that all users can take the RDA or more of molybdenum. In the example of Figure 5, since the EER in the category of males aged 18 to 29 years with physical activity level III (high) is higher than other categories, the molybdenum that can be added to the standard food is 324.6 μg/day (600 μg $\times$ 1650 kcal/3050 kcal), which is smallest. Therefore, this value is set as the upper limit of molybdenum intake in the standard food.

In the US DRIs, the RDA of molybdenum for males and females aged 21 to 65 years is set to 45 μg/day. Therefore, in the case of using the US DRIs as DRIs, 45 μg/day is set as the lower limit of intake in the standard food. Meanwhile, a normalized molybdenum amount in each category is calculated by the aforementioned formula for the UL, and the smallest normalized molybdenum amount is set as the upper limit of molybdenum intake in the standard food. In the example of Figure 9, the upper limit of molybdenum in the category of males aged 21 to 35 years with physical activity level A (Active), 1067 μg/day, is set as the upper limit of molybdenum intake in the standard food.

In the Chinese DRIs, the RDA of molybdenum for males and females aged 18 to 64 years is set to 100 μg/day. Therefore, in the case of using the Chinese DRIs as DRIs, 100 μg/day is set as the lower limit of intake in the standard food. Meanwhile, a normalized molybdenum amount in each category is calculated by the aforementioned formula for the UL, and the smallest normalized molybdenum amount is set as the upper limit of molybdenum intake in the standard food. In the example of Figure 13, the upper limit of molybdenum in the category of males aged 18 to 49 years with physical activity level III (high),525 μg/day, is set as the upper limit of molybdenum intake in the standard food.

17. Chloride

In the US DRIs, the AI of chloride for males and females aged 21 to 65 years is set to 2.3 mg/day. However, if the AI, 2.3 mg/day, is set as the lower limit of intake in the standard food, the upper limit of chloride for the users belonging to the category of males aged 21 to 35 years with physical activity level A (Active) calculated by the formula of the normalized chloride amount (normalized chloride amount = UL of chloride × (standard energy amount/EER in each category)) is 1.9 g, which is below the lower limit, as shown in Figure 9. Therefore, the upper and lower limits of chloride intake are not set. However, it is possible to set the upper and lower limits of intake by limiting the age and gender.

In the Chinese DRIs, the RDA of chloride for males and females aged 18 to 64 years is set to 2300 mg/day. Therefore, in the case of using the Chinese DRIs as DRIs, 2300 mg/day is set as the lower limit of intake in the standard food.

18. Nickel

In the US DRIs, UL of nickel is set. In the case of using the US DRIs as DRIs, a normalized nickel amount in each category is calculated by the following formula for the UL, and the smallest normalized nickel amount is set as the upper limit of nickel intake in the standard food.

$$\text{Normalized nickel amount} = \text{UL of nickel} \times (\text{standard energy amount/EER in each category})$$

In the example of Figure 9, the upper limit of nickel in the category of males aged 21 to 35 years with physical activity level A (Active), 0.53 mg/day, is set as the upper limit of nickel intake in the standard food.

19. Vanadium

In the US DRIs, UL of vanadium is set. In the case of using the US DRIs as DRIs, a normalized vanadium amount in each category is calculated by the following formula for the UL, and the smallest normalized vanadium amount is set as the upper limit of vanadium intake in the standard food.

$$\text{Normalized vanadium amount} = \text{UL of vanadium} \times (\text{standard energy amount/EER in each category})$$

In the example of Figure 9, the upper limit of vanadium in the category of males aged 21 to 35 years with physical activity level A (Active), 0.96 mg/day, is set as the upper limit of vanadium intake in the standard food.

20. Boron

In the US DRIs, UL of boron is set. In the case of using the US DRIs as DRIs, a normalized boron amount in each category is calculated by the following formula for the UL, and the smallest normalized boron amount is set as the upper limit of boron intake in the standard food.

$$\text{Normalized boron amount} = \text{UL of boron} \times (\text{standard energy amount/EER in each category})$$

In the example of Figure 9, the upper limit of boron in the category of males aged 21 to 35 years with physical activity level A (Active), 10.7 mg/day, is set as the upper limit of boron intake in the standard food.

21. Fluoride

In the US DRIs, the AI of fluoride for males and females aged 21 to 65 years is set to 4 mg/day. Therefore, in the case of using the US DRIs as DRIs, 4 mg/day is set as the lower limit of intake in the standard food. Meanwhile, a normalized fluoride amount in each category is calculated by the following formula for the UL, and the smallest normalized fluoride amount is set as the upper limit of fluoride intake in the standard food.

$$\text{Normalized fluoride amount} = \text{UL of fluoride} \times (\text{standard energy amount/EER in each category})$$

In the example of Figure 9, the upper limit of fluoride in the category of males aged 21 to 35 years with physical activity level A (Active), 5.3 mg/day, is set as the upper limit of fluoride intake in the standard food.

22. Vitamin A

The upper and lower limits of vitamin A intake in the standard food of this embodiment are calculated, as follows.

According to the "Japanese DRIs", RDA and a UL for those aged 18 to 64 years are set as DRIs for vitamin A. The amount of the standard food in this embodiment is adjusted according to the attributes of each user. Therefore, the vitamin A contained in an adjusted amount of the standard food needs to be below the DRI in the categories with high EERs. Thus, a normalized vitamin A amount in each category is calculated by the following formula, and the smallest normalized vitamin A amount is set as the upper limit of vitamin A intake in the standard food.

$$\text{Normalized vitamin A amount} = \text{UL of vitamin A} \times (\text{standard energy amount/EER in each category})$$

Further, the maximum value (900 μg RE/day) of the RDA of vitamin A in each category is set as the lower limit of vitamin A intake in the standard food, so that all users can take the RDA or more of vitamin A.

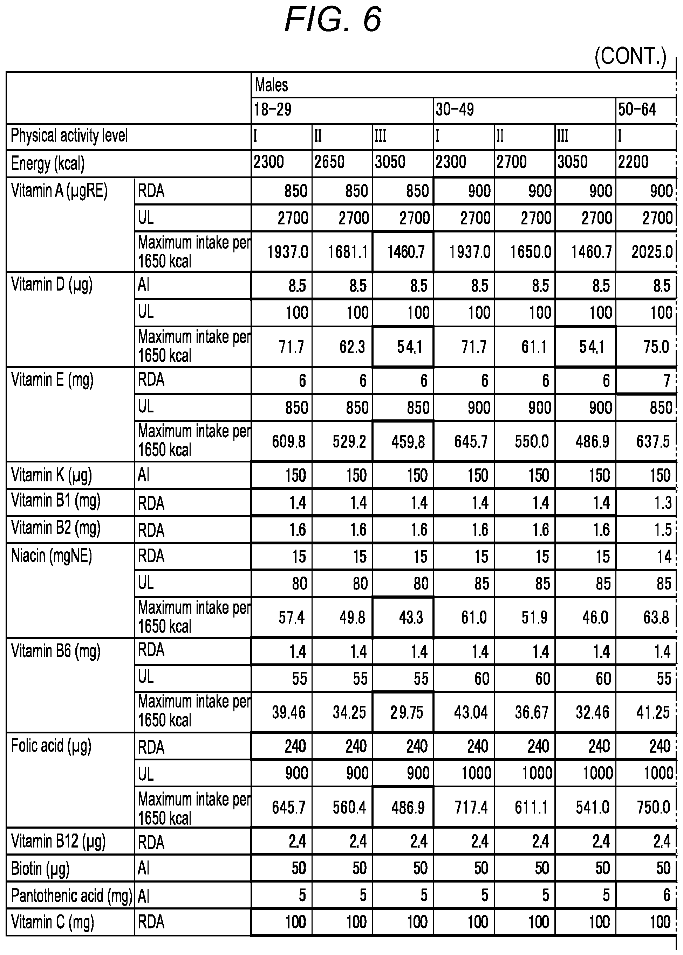

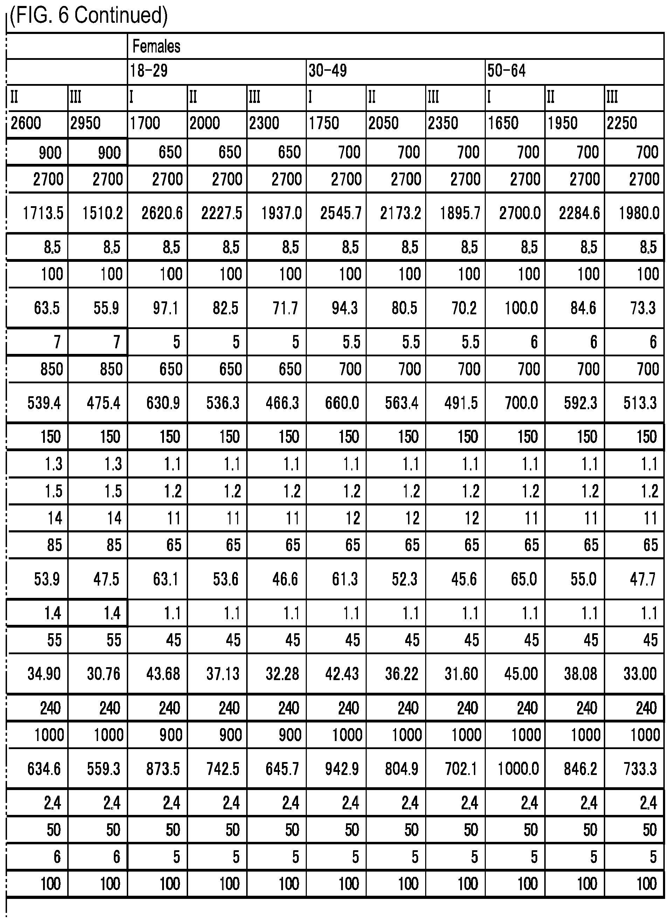

In the example of Figure 6, since the EER in the category of males aged 18 to 29 years with physical activity level III (high) is higher than other categories, the vitamin A that can be added to the standard food is 1460.7 μg RE/day (2700 μg RE × 1650 kcal/3050 kcal), which is smallest. Therefore, this value is set as the upper limit of vitamin A intake in the standard food.

There are retinol (animal) and carotenoid (vegetable) in Vitamin A. The upper limits of only retinol and added vitamin A are set in the "Japanese DRIs", and no upper limit is set for dietary carotenoid. The same applies to this embodiment.

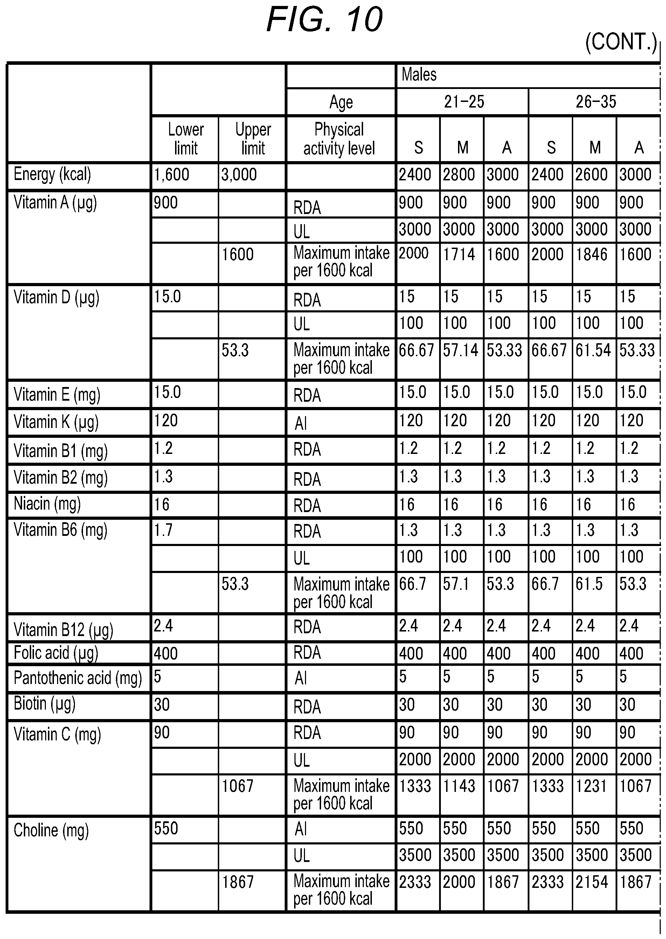

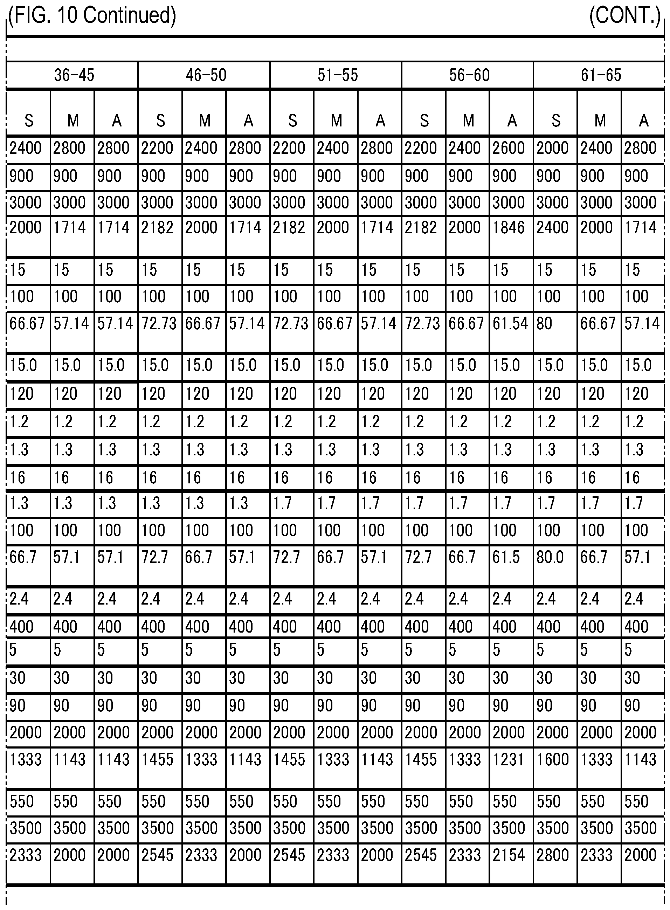

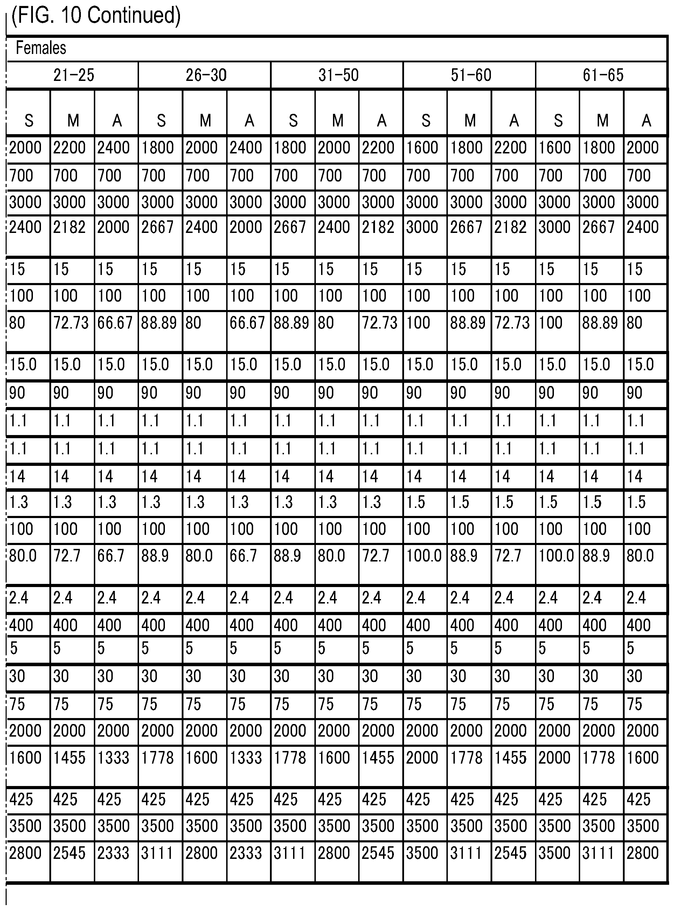

In the US DRIs, the RDA of vitamin A for males and females aged 21 to 65 years is set to 700 to 900 μg/day. Therefore, in the case of using the US DRIs as DRIs, the maximum value, 900 μg/day, is set as the lower limit of intake in the standard food. Meanwhile, a normalized vitamin A amount in each category is calculated by the aforementioned formula for the UL, and the smallest normalized vitamin A amount is set as the upper limit of vitamin A intake in the standard food. In the example of Figure 10, the upper limit of vitamin A in the category of males aged 21 to 35 years with physical activity level A (Active), 1600 μg/day, is set as the upper limit of vitamin A intake in the standard food.

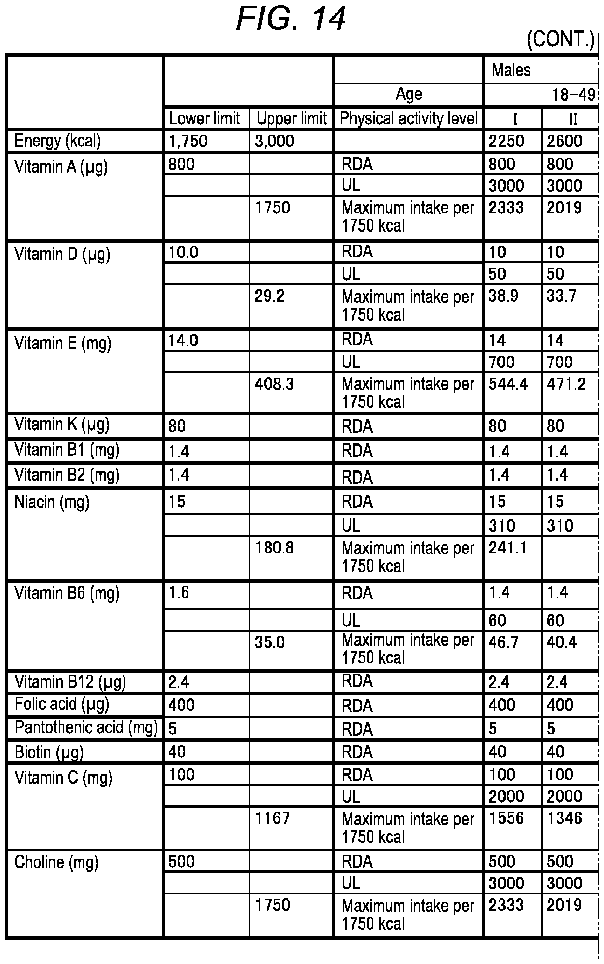

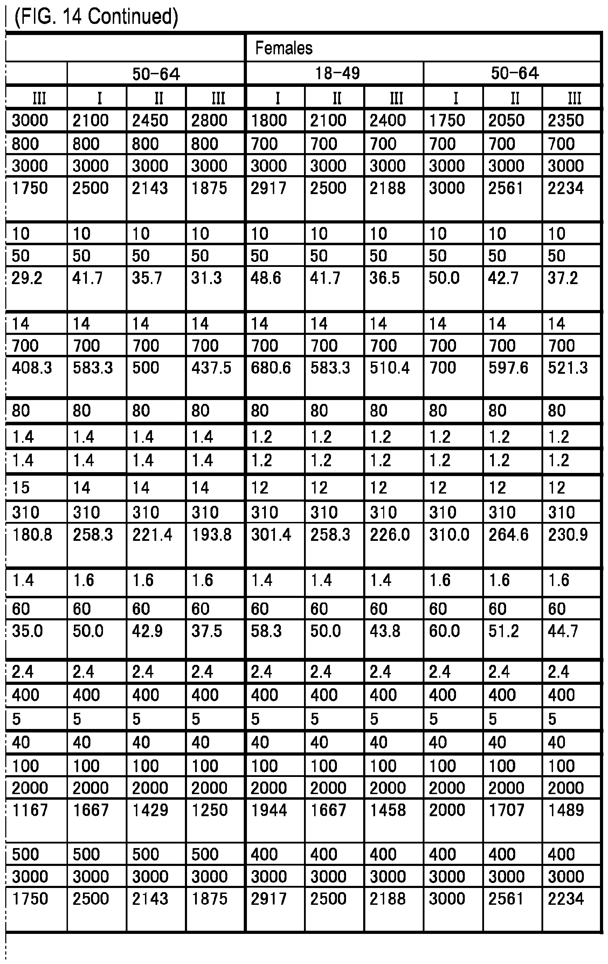

In the Chinese DRIs, the RDA of vitamin A for males and females aged 18 to 64 years is set to 700 to 800 μg/day. Therefore, in the case of using the Chinese DRIs as DRIs, 800 μg/day is set as the lower limit of intake in the standard food. Meanwhile, a normalized vitamin A amount in each category is calculated by the aforementioned formula for the UL, and the smallest normalized vitamin A amount is set as the upper limit of vitamin A intake in the standard food. In the example of Figure 14, the upper limit of vitamin A in the category of males aged 18 to 49 years with physical activity level III (high), 1750 μg/day, is set as the upper limit of vitamin A intake in the standard food.

23. Vitamin D

The upper and lower limits of vitamin D intake in the standard food of this embodiment are calculated, as follows.

According to the "Japanese DRIs", AI and UL for those aged 18 to 64 years are set as DRIs for vitamin D. The amount of the standard food in this embodiment is adjusted according to the attributes of each user. Therefore, the vitamin D contained in an adjusted amount of the standard food needs to be below the DRI in the categories with high EERs. Thus, a normalized vitamin D amount in each category is calculated by the following formula, and the smallest normalized vitamin D amount is set as the upper limit of vitamin D intake in the standard food.

$$\text{Normalized vitamin D amount} = \text{UL of vitamin D} \times (\text{standard energy amount/EER in each category})$$

Further, the maximum value (8.5 μg/day) of the AI of vitamin D in each category is set as the lower limit of vitamin D intake in the standard food, so that all users can take the AI or more of vitamin D. In the example of Figure 6, since the EER in the category of males aged 18 to 29 years with physical activity level III (high) is higher than other categories, the vitamin D that can be added to the standard food is 54.1 μg/day (100 μg × 1650 kcal/3050 kcal), which is smallest. Therefore, this value is set as the upper limit of vitamin D intake in the standard food.

In the US DRIs, the RDA of vitamin D for males and females aged 21 to 65 years is set to 15 μg/day. Therefore, in the case of using the US DRIs as DRIs, 15 μg/day is set as the lower limit of intake in the standard food. Meanwhile, a normalized vitamin D amount in each category is calculated by the aforementioned formula for the UL, and the smallest normalized vitamin D amount is set as the upper limit of vitamin D intake in the standard food. In the example of Figure 10, the upper limit of vitamin D in the category of males aged 21 to 35 years with physical activity level A (Active), 53.3 μg/day, is set as the upper limit of vitamin D intake in the standard food.

In the Chinese DRIs, the RDA of vitamin D for males and females aged 18 to 64 years is set to 10 μg/day.

Therefore, in the case of using the Chinese DRIs as DRIs, 10 μg/day is set as the lower limit of intake in the standard food. Meanwhile, a normalized vitamin D amount in each category is calculated by the aforementioned formula for the UL, and the smallest normalized vitamin D amount is set as the upper limit of vitamin D intake in the standard food. In the example of Figure 14, the upper limit of vitamin D in the category of males aged 18 to 49 years with physical activity level III (high), 29.2 μg/day, is set as the upper limit of vitamin D intake in the standard food.

24. Vitamin E

The upper and lower limits of vitamin E intake in the standard food of this embodiment are calculated, as follows.
According to the "Japanese DRIs", AI and UL for those aged 18 to 64 years are set as DRIs for vitamin E. The amount of the standard food in this embodiment is adjusted according to the attributes of each user. Therefore, the vitamin E contained in an adjusted amount of the standard food needs to be below the DRI in the categories with high EERs. Thus, a normalized vitamin E amount in each category is calculated by the following formula, and the smallest normalized vitamin E amount is set as the upper limit of vitamin E intake in the standard food.

$$\text{Normalized vitamin E amount} = \text{UL of vitamin E} \times (\text{standard energy amount/EER in each category})$$

Further, the maximum value (7.0 mg/day) of the AI of vitamin E in each category is set as the lower limit of vitamin E intake in the standard food, so that all users can take the AI or more of vitamin E.
In the example of Figure 6, since the EER in the category of males aged 18 to 29 years with physical activity level III (high) is higher than other categories, the vitamin E that can be added to the standard food is 459.8 mg/day (= 850 mg × 1650 kcal/3050 kcal), which is smallest. Therefore, this value is set as the upper limit of vitamin E intake in the standard food.
In the US DRIs, the RDA of vitamin E for males and females aged 21 to 65 years is set to 15 mg/day. Therefore, in the case of using the US DRIs as DRIs, 15 mg/day is set as the lower limit of intake in the standard food.
In the Chinese DRIs, the RDA of vitamin E for males and females aged 18 to 64 years is set to 14 mg/day. Therefore, in the case of using the Chinese DRIs as DRIs, 14 mg/day is set as the lower limit of intake in the standard food. Meanwhile, a normalized vitamin E amount in each category is calculated by the aforementioned formula for the UL, and the smallest normalized vitamin E amount is set as the upper limit of vitamin E intake in the standard food. In the example of Figure 14, the upper limit of vitamin E in the category of males aged 18 to 49 years with physical activity level III (high), 408.3 mg/day, is set as the upper limit of vitamin E intake in the standard food.

25. Vitamin K

The lower limit of vitamin K in the standard food of this embodiment is calculated, as follows.
The maximum value (150 μg/day) of the AI of vitamin K in each category is set as the lower limit of vitamin K intake in the standard food, so that all users can take the AI or more of vitamin K. According to the "Japanese DRIs", since the AI vitamin K is set to 150 μg/day regardless of the age, gender, and physical activity level, the AI vitamin K is set as the lower limit of intake in the standard food.
In the US DRIs, the AI of vitamin K for males and females aged 21 to 65 years is set to 90 to 120 μg/day. Therefore, in the case of using the US DRIs as DRIs, the maximum value, 120 μg/day, is set as the lower limit of intake in the standard food.
In the Chinese DRIs, the RDA of vitamin K for males and females aged 18 to 64 years is set to 80 μg/day. Therefore, in the case of using the Chinese DRIs as DRIs, 80 μg/day is set as the lower limit of intake in the standard food.

26. Vitamin B1

The lower limit of vitamin B1 in the standard food of this embodiment is calculated, as follows.
RDA of vitamin B1 is set in the "Japanese DRIs" and is set to 1.1 to 1.4 mg/day according to age, gender, and physical activity level. In this embodiment, the maximum value (1.4 mg/day) of the AI of vitamin B1 in each category is set as the lower limit of intake in the standard food in this embodiment, so that all users can take the AI of vitamin B1. As a result, even if a male aged 18 to 49 years suppresses the caloric intake by diet to 1650 kcal/day, for example, for the purpose of losing the weight, the AI, 1.4 mg/day, which is the DRI for vitamin B1 can be satisfied by taking the standard food.
In the US DRIs, the RDA of vitamin B1 for males and females aged 21 to 65 years is set to 1.1 to 1.2 mg/day. Therefore, in the case of using the US DRIs as DRIs, the maximum value, 1.2 mg/day, is set as the lower limit of intake in the standard food.

In the Chinese DRIs, the RDA of vitamin B1 for males and females aged 18 to 64 years is set to 1.2 to 1.4 mg/day. Therefore, in the case of using the Chinese DRIs as DRIs, the maximum value, 1.4 mg/day, is set as the lower limit of intake in the standard food.

27. Vitamin B2

The lower limit of vitamin B2 in the standard food of this embodiment is calculated, as follows.
RDA of vitamin B2 is set in the "Japanese DRIs" and is set to 1.2 to 1.6 mg/day according to age, gender, and physical activity level. In this embodiment, the maximum value (1.6 mg/day) of the AI of vitamin B2 in each category is set as the lower limit of intake in the standard food in this embodiment, so that all users can take the AI of vitamin B2. As a result, even if a male aged 18 to 49 years suppresses the caloric intake by diet to 1650 kcal/day, for example, for the purpose of losing the weight, the AI, 1.6 mg/day, which is the DRI for vitamin B1 can be satisfied by taking the standard food.
In the US DRIs, the RDA of vitamin B2 for males and females aged 21 to 65 years is set to 1.1 to 1.3 mg/day. Therefore, the maximum value, 1.3 mg/day, is set as the lower limit of intake in the standard food.
In the Chinese DRIs, the RDA of vitamin B2 for males and females aged 18 to 64 years is set to 1.2 to 1.4 mg/day. Therefore, the maximum value, 1.4 mg/day, is set as the lower limit of intake in the standard food.

28. Niacin

The upper and lower limits of niacin intake in the standard food of this embodiment are calculated, as follows.
According to the "Japanese DRIs", RDA and UL for those aged 18 to 64 years are set for niacin. Here, the UL is set only for fortified foods and supplement-derived niacin.
The amount of the standard food in this embodiment is adjusted according to the attributes of each user. Therefore, the niacin contained in an adjusted amount of the standard food needs to be below the DRI in the categories with high EERs. Thus, a normalized niacin amount in each category is calculated by the following formula, and the smallest normalized niacin amount is set as the upper limit of niacin intake in the standard food.

$$\text{Normalized niacin amount} = \text{UL of niacin} \times (\text{standard energy amount/EER in each category})$$

Further, the maximum value (15 mg NE/day) of the RDA of niacin equivalent in each category is set as the lower limit of niacin equivalent intake in the standard food, so that all users can take the RDA or more of niacin equivalent. In the example of Figure 6, since the EER in the category of males aged 18 to 29 years with physical activity level III (high) is higher than other categories, the niacin that can be added to the standard food is 43.3 mg/day (80 mg × 1650 kcal/3050 kcal), which is smallest. Therefore, this value is set as the upper limit of niacin intake in the standard food. According to the "Japanese DRIs", upper limits are set for fortified foods and supplement-derived niacin, and the same applies to this embodiment.
In the US DRIs, the RDA of niacin for males and females aged 21 to 65 years is set to 14 to 16 mg/day. Therefore, in the case of using the US DRIs as DRIs, the maximum value of the RDA, 16 mg/day, is set as the lower limit of intake in the standard food.
In the Chinese DRIs, the RDA of niacin for males and females aged 18 to 64 years is set to 12 to 15 mg/day. Therefore, in the case of using the Chinese DRIs as DRIs, the maximum value of the RDA, 15 mg/day, is set as the lower limit of intake in the standard food. Meanwhile, a normalized niacin amount in each category is calculated by the aforementioned formula for the UL, and the smallest normalized niacin amount is set as the upper limit of niacin intake in the standard food. In the example of Figure 14, the upper limit of niacin in the category of males aged 18 to 49 years with physical activity level III (high), 180.8 mg/day, is set as the upper limit of niacin intake in the standard food.

29. Vitamin B6

The upper and lower limits of vitamin B6 intake in the standard food of this embodiment are calculated, as follows.
According to the "Japanese DRIs", RDA and UL are set for those aged 18 to 64 years as DRIs for vitamin B6. The amount of the standard food in this embodiment is adjusted according to the attributes of each user. Therefore, the vitamin B6 contained in an adjusted amount of the standard food needs to be below the DRI in the categories with high EERs. Thus, a normalized vitamin B6 amount in each category is calculated by the following formula, and the smallest normalized vitamin B6 amount is set as the upper limit of vitamin B6 intake in the standard food.

$$\text{Normalized vitamin B6 amount} = \text{UL of vitamin B6} \times (\text{standard energy amount/EER in each category})$$

Further, the maximum value (1.4 mg/day) of the RDA of vitamin B6 in each category is set as the lower limit of vitamin B6 intake in the standard food, so that all users can take the RDA or more of vitamin B6. In the example of Figure 6, since the EER in the category of males aged 18 to 29 years with physical activity level III (high) is higher than other categories, the vitamin B6 that can be added to the standard food is 29.75 mg/day (55 mg $\times$ 1650 kcal/3050 kcal), which is smallest. Therefore, the upper limit of vitamin B6 intake in the standard food is set to 29.75 mg. As a result, even if a user belonging to the category of males aged 18 to 29 years with physical activity level III (high) eats 1650 kcal/day of the standard food containing vitamin B6 in an amount of less than the aforementioned upper limit of intake (for example, 29.7 mg/day), at 3050 kcal/day, the tolerable upper limit of vitamin B6, 55 mg/day, is not exceeded.

In the US DRIs, the RDA of vitamin B6 for males and females aged 21 to 65 years is set to 1.3 to 1.7 mg/day. Therefore, in the case of using the US DRIs as DRIs, the maximum value of the RDA, 1.7 mg/day, is set as the lower limit of intake in the standard food. Meanwhile, a normalized vitamin B6 amount in each category is calculated by the aforementioned formula for the UL, and the smallest normalized vitamin B6 amount is set as the upper limit of vitamin B6 intake in the standard food.

In the example of Figure 10, the upper limit of vitamin B6 in the category of males aged 21 to 35 years with physical activity level A (Active), 53.3 mg/day, is set as the upper limit of vitamin B6 intake in the standard food.

In the Chinese DRIs, the RDA of vitamin B6 for males and females aged 18 to 64 years is set to 1.4 to 1.6 mg/day. Therefore, in the case of using the Chinese DRIs as DRIs, the maximum value of the RDA, 1.6 mg/day, is set as the lower limit of intake in the standard food. Meanwhile, a normalized vitamin B6 amount in each category is calculated by the aforementioned formula for the UL, and the smallest normalized vitamin B6 amount is set as the upper limit of vitamin B6 intake in the standard food.

In the example of Figure 14, the upper limit of vitamin B6 in the category of males aged 18 to 49 years with physical activity level III (high), 35 mg/day, is set as the upper limit of vitamin B6 intake in the standard food.

30. Vitamin B12, biotin, and vitamin C

For the standard food in this embodiment, the lower limits of intake of vitamin B12, biotin, and vitamin C are calculated, as follows.

RDAs of vitamin B12 and vitamin C are set in the "Japanese DRIs", and AI of biotin is set. In this embodiment, the maximum values of the RDAs of vitamin B12 and vitamin C or the AI of biotin in each category are set as the lower limits of intake in the standard food, so that all users can take the RDAs of vitamin B12 and vitamin C or the AI of biotin. According to the "Japanese DRIs", since the RDAs of vitamin B12 and vitamin C are respectively set to 2.4 $\mu$g/day and 100 mg/day, and the AI of biotin is set to 50 $\mu$g/day, regardless of the age, gender, and physical activity level, the RDAs of vitamin B12 and vitamin C or the AI of biotin are respectively set as the lower limits of intake in the standard food.

In the US DRIs, the RDA of vitamin B12 for males and females aged 21 to 65 years is set to 2.4 $\mu$g/day, and the RDA of biotin is set to 30 $\mu$g/day. Therefore, in the case of using the US DRIs as DRIs, the RDA of vitamin B12, 2.4 $\mu$g/day, and the RDA of biotin, 30 $\mu$g/day, are respectively set as the lower limits of intake in the standard food.

In the Chinese DRIs, the RDA of vitamin B12 for males and females aged 18 to 64 years is set to 2.4 $\mu$g/day, and the RDA of biotin is set to 40 $\mu$g/day. Therefore, in the case of using the Chinese DRIs as DRIs, the RDA of vitamin B12, 2.4 $\mu$g/day, and the RDA of biotin, 40 $\mu$g/day, are set as the lower limits of intake in the standard food.

In the US DRIs, the RDA of vitamin C for males and females aged 21 to 65 years is set to 75 to 90 mg/day. Therefore, in the case of using the US DRIs as DRIs, the maximum value, 90 mg/day, is set as the lower limit of intake in the standard food. Meanwhile, a normalized vitamin C amount in each category is calculated by the following formula for the UL, and the smallest normalized vitamin C amount is set as the upper limit of vitamin C intake in the standard food.

$$\text{Normalized vitamin C amount} = \text{UL of vitamin C} \times (\text{standard energy amount/EER in each category})$$

In the example of Figure 10, the upper limit of vitamin C in the category of males aged 21 to 35 years with physical activity level A (Active), 1067 mg/day, is set as the upper limit of vitamin C intake in the standard food.

In the Chinese DRIs, the RDA of vitamin C for males and females aged 18 to 64 years is set to 100 mg/day.

Therefore, 100 mg/day is set as the lower limit of intake in the standard food. Meanwhile, a normalized vitamin C amount in each category is calculated by the aforementioned formula for the UL, and the smallest normalized vitamin C amount is set as the upper limit of vitamin C intake in the standard food. In the example of Figure 14, the upper limit of vitamin C in the category of males aged 18 to 49 years with physical activity level III (high), 1167 mg/day, is set as the upper limit of vitamin C intake in the standard food.

31. Choline

In the US DRIs, the AI of choline for males and females aged 21 to 65 years is set to 425 to 550 mg/day. Therefore, in the case of using the US DRIs as DRIs, the maximum value, 550 mg/day, is set as the lower limit of intake in the standard food. Meanwhile, a normalized choline amount in each category is calculated by the following formula for the UL, and the smallest normalized choline amount is set as the upper limit of vitamin C intake in the standard food.

$$\text{Normalized choline amount} = \text{UL of choline} \times (\text{standard energy amount/EER in each category})$$

In the example of Figure 10, the upper limit of choline in the category of males aged 21 to 35 years with physical activity level A (Active), 1867 mg/day, is set as the upper limit of choline intake in the standard food.
In the Chinese DRIs, the RDA of choline for males and females aged 18 to 64 years is set to 400 to 500 mg/day. Therefore, in the case of using the Chinese DRIs as DRIs, the maximum value, 500 mg/day, is set as the lower limit of intake in the standard food. Meanwhile, a normalized choline amount in each category is calculated by the aforementioned formula for the UL, and the smallest normalized choline amount is set as the upper limit of choline intake in the standard food. In the example of Figure 14, the upper limit of choline in the category of males aged 18 to 49 years with physical activity level III (high), 1750 mg/day, is set as the upper limit of choline intake in the standard food.

32. Pantothenic acid

For the standard food in this embodiment, the lower limit of pantothenic acid intake is calculated, as follows.
AI of pantothenic acid is set in the "Japanese DRIs" and is set to 5 to 6 mg/day according to age, gender, and physical activity level. In this embodiment, the maximum value (6 mg/day) of the AI of pantothenic acid in each category is set as the lower limit of intake in the standard food, so that all users can take the AI of pantothenic acid. As a result, all users can take the AI of pantothenic acid by eating at least the standard food.
In the US DRIs, the AI of pantothenic acid for males and females aged 21 to 65 years is set to 5 mg/day. Therefore, in the case of using the US DRIs as DRIs, 5 mg/day is set as the lower limit of intake in the standard food.
In the Chinese DRIs, the RDA of pantothenic acid for males and females aged 18 to 64 years is set to 5 mg/day. Therefore, in the case of using the Chinese DRIs as DRIs, 5 mg/day is set as the lower limit of intake in the standard food.

33. Folic acid

The upper and lower limits of folic acid intake in the standard food of this embodiment are calculated, as follows.
According to the "Japanese DRIs", RDA and UL are set for those aged 18 to 64 years as DRIs for folic acid.
The amount of the standard food in this embodiment is adjusted according to the attributes of each user. Therefore, the folic acid contained in an adjusted amount of the standard food needs to be below the DRI in the categories with high EERs. Thus, a normalized folic acid amount in each category is calculated by the following formula, and the smallest normalized folic acid amount is set as the upper limit of folic acid intake in the standard food.

$$\text{Normalized folic acid amount} = \text{UL of folic acid} \times (\text{standard energy amount/EER in each category})$$

Further, the maximum value (240 μg/day) of the RDA of folic acid in each category is set as the lower limit of folic acid intake in the standard food, so that all users can take the RDA or more of folic acid.
In the example of Figure 6, since the EER in the category of males aged 18 to 29 years with physical activity level III (high) is higher than other categories, the folic acid that can be added to the standard food is 486.9 μg/day (900 μg × 1650 kcal/3050 kcal), which is smallest. Therefore, the upper limit of folic acid intake in the standard food is set to 486.9 μg/day.
As a result, even if a user belonging to the category of males aged 18 to 29 years with physical activity level III (high) eats 1650 kcal/day of the standard food containing folic acid in an amount of less than the aforementioned upper limit of intake, at 3050 kcal/day, the tolerable upper limit of folic acid, 900 μg/day, is not exceeded.
In the "Japanese Intake Standards", the upper limit is set only for pteroylmonoglutamic acid to be added, and the same applies to this embodiment.
In the US DRIs, the RDA of folic acid for males and females aged 21 to 65 years is set to 400 μg/day. Therefore, in the case of using the US DRIs as DRIs, the lower limit of intake in the standard food is set to 400 μg/day. The UL is a value that is applied to synthetic products that can be added to supplements and fortified foods, as in Japan, and the calculation is omitted here.

In the Chinese DRIs, the RDA of folic acid for males and females aged 18 to 64 years is set to 400 μg/day. Therefore, in the case of using the Chinese DRIs as DRIs, the lower limit of intake in the standard food is set to 400 μg/day. The UL is a value that is applied to synthetic products that can be added to supplements and fortified foods, as in Japan, and the calculation is omitted here.

Here, the present invention includes not only the complete nutritious food product-providing system and the food information display, but also the nutrition calculation method for a complete nutritious food product and the nutrition calculation method program for a complete nutritious food product. Hereinafter, examples of the nutrition calculation method for a complete nutritious food product and the nutrition calculation method program for a complete nutritious food product are shown.

An example of the nutrition calculation method for a complete nutritious food product is a nutrition calculation method for a complete nutritious food product, comprising a step of acquiring information on user attributes, a step of storing the information on user attributes, a step of storing DRIs of the complete nutritious food product in which the intake standards for the first and second nutrients are set for each user attribute, and a step of calculating the intake standard for the first nutrient, which is satisfied regardless of attributes of each user, in the standard food forming the complete nutritious food product.

An example of the nutrition calculation program of a complete nutritious food product is a nutrition calculation program of a complete nutritious food product, comprising a step of acquiring information on user attributes, a step of storing the information on user attributes, a step of storing DRIs of the complete nutritious food product in which the intake standards for the first and second nutrients are set for each user attribute, and a step of calculating the intake standard for the first nutrient, which is satisfied regardless of attributes of each user, in the standard food forming the complete nutritious food product.

A complete nutritious food product-providing system 1 in this embodiment exerts the following effects.

This embodiment enables users who receive meals to easily take a complete nutritious food product, to be freed from the trouble of nutritious management, and to improve their health without awareness. Further, this embodiment enables businesses that provide the nutritious food to provide a complete nutritious food product for each user only by adjusting the amount (calories) of the nutritious food and to reduce the labor and cost of nutrition calculation. The present invention is not limited to the aforementioned embodiments, and the present invention includes modifications, improvements, etc. within the range in which the object of the present invention can be achieved.

In the present invention, the complete nutritious food product or the standard food described above can be taken in three meals (breakfast, lunch, and supper (including snacks)) in a day. In this case, since the first and second nutrients contained in the complete nutritious food product or the standard food may be unevenly contained in any of breakfast, lunch, and supper (including snacks) but are more preferably contained in proportion to the amount (calories) of each meal. In the present invention, it is also possible to select the form of taking one or two meals of the complete nutritious food product or the standard food described above among breakfast, lunch, and supper in a day.

It is further preferable to take a nutritionally calculated meal using the complete nutritious food product-providing system of the present invention, especially in a meal having a high energy intake, among breakfast, lunch, and supper. Then, a ratio of about 20:30:50 can be selected as an example of the energy intake ratio for breakfast, lunch, and supper (including snacks), but there is no limitation to this ratio.

Examples

Hereinafter, examples of the present invention will be described. The present invention is not limited to the following examples. In these examples, in order to confirm the efficacy of the standard food nutritionally calculated using the "Japanese DRIs" by the complete nutritious food product-providing system, the standard food was provided to healthy subjects as breakfast and lunch of the daily meals for a certain period. Then, the improvement in health conditions of the subjects was evaluated based on specific health indicators.

<Test method>

The standard food was taken by healthy adult males and females (age: 37.4 ± 8.8) as breakfast and lunch, and changes in their physical conditions and awareness of health were evaluated. For sodium (salt equivalent) in the standard food, each meal was adjusted to contain the range of a salt equivalent of 1.7 g or less in breakfast and less than 3.0 g in lunch, so that the subjects can continue to take meals without getting tired.

The number of subjects was 83, including 67 males and 16 females. The intake period was 17 to 19 days (about 4 weeks). Further, the standard food (breakfast and lunch) was taken only on weekdays of the week, and meals were taken freely on Saturdays, Sundays, and holidays.

The standard food nutritionally calculated using the complete nutritious food product-providing system of the present invention was taken by the subjects as breakfast and lunch, and other meals (supper and snacks) were freely taken. 5 types of breakfast and 15 types of lunch were prepared. One of the 5 types of breakfast was selected by the subjects, and two types of lunch out of the 15 types were presented to the subjects, so that one of them was selected by the subjects. The breakfast was adjusted to fall within the range of about 280 kcal to 340 kcal, and the lunch was adjusted to fall within the range of about 460 kcal to 540 kcal.

As analysis targets, only the subjects who have taken 80% or more of the meals provided during the intake period were included in the analysis. As a result, the number of subjects analyzed was 75 (62 males and 13 females). Further, a "paired t-test" and a "Wilcoxon signed-rank test" were conducted as analysis methods, and the significance level was set to 0.05.

The standard food provided as breakfast and lunch was based on a menu providing vegetables, grains, livestock meat, fish, etc., and their processed products cooked by methods such as baking, simmering, steaming, and frying, and seasonings, as required, and was supplemented with nutritional supplements, food additives, functional ingredients, various salts, etc., to compensate for the lack of various nutritional components other than above.

<Results>

(1) Anthropometry

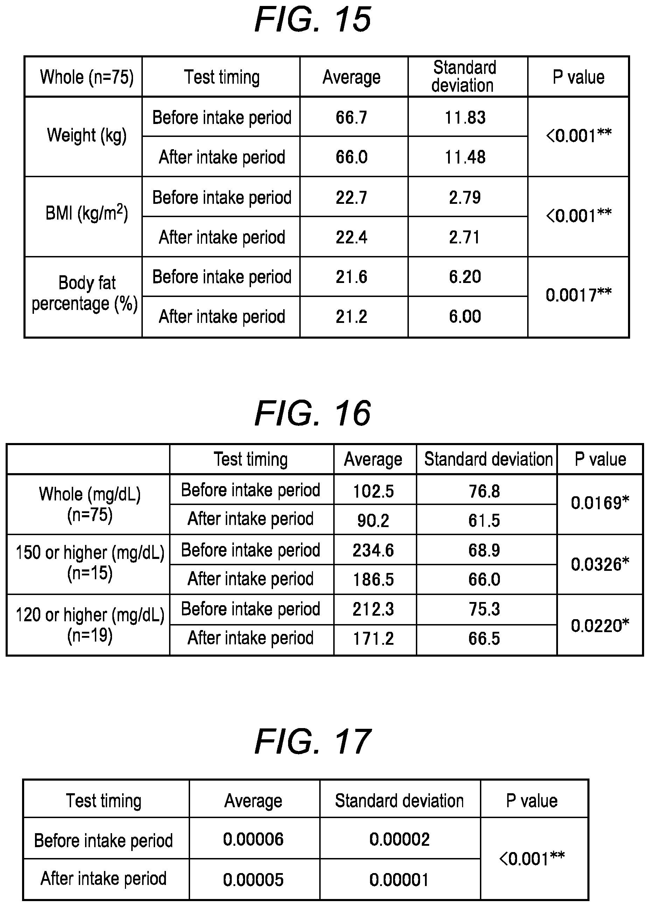

Figure 15 shows the results of anthropometry before and after the aforementioned modified food was continuously taken for a certain period. The weight, BMI, and body fat percentage significantly decreased as compared with those before the intake period.

(2) Blood triglyceride

Figure 16 below shows the analysis results of triglyceride among the blood lipid before and after the aforementioned modified food was continuously taken for a certain period.

The blood triglyceride significantly decreased as compared with that before the intake period. It is considered that the group with higher triglyceride level has a larger reduction. For the standard values in Figure 16 above, a value of 150 or more indicates hyperglyceridemia, and a value of 120 to 149 falls within a normal high range.

(3) Blood metabolome analysis

For analyzing the blood metabolome before and after the aforementioned modified food was continuously taken for a certain period, metabolites in blood (plasma) of the subjects were analyzed using a CE-TOFMS (capillary electrophoresis time-of-flight mass spectrometer), and the peak areas of 229 candidate compounds detected were compared with those before and after the intake. Further, among the compounds (40 compounds) that showed significant fluctuations, those with evidence of action on humans were investigated. As a result, 8-OHdG (8-hydroxy-2'-deoxyguanosine) significantly decreased after the intake period, as shown in Figure 9. The 8-OHdG is a compound with evidence as an oxidative stress marker for DNA damage. Figure 17 shows a relative area.

(4) Blood pressure

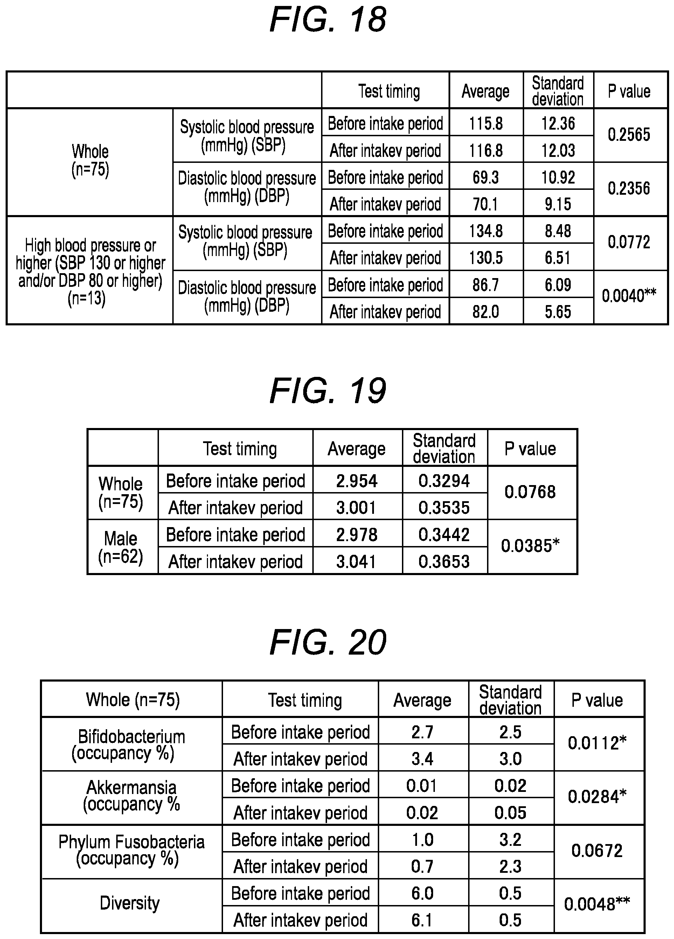

The blood pressure was measured before and after the aforementioned modified food was continuously taken for a certain period. Figure 18 shows the results. Further, a stratified analysis was also performed in the group with high blood pressure or higher. In the group with high blood pressure or higher, systolic blood pressure tended to decrease, and diastolic blood pressure significantly decreased, as compared with those before the intake period.

(5) Bone density

The bone density was measured before and after the aforementioned modified food was continuously taken for a certain period. Figure 19 shows the results. The bone density was measured using AOS-100SA (available from Hitachi, Ltd.) by OSI (osteo-sono assessment index). The bone density tended to increase overall and significantly increased in males, as compared with that before the intake period.

(6) Intestinal flora analysis

Intestinal flora analysis was performed before and after the aforementioned modified food was continuously taken for a certain period. Figure 20 shows the results. There was an improvement in the intestinal flora indicator, which is said to affect health. The occupancy and diversity scores of Bifidobacterium and Akkermansia significantly increased.

Further, the occupancy of the phylum Fusobacteria tended to decrease.

Reference Signs List

> 1: Complete nutritious food product-providing system
> 10: Terminal device
> 20: Food information display
> 30: Food production-controlling device
> 31: Food production-controlling unit
> 40: Food delivery device
> 41: Delivery instruction unit
> 100: Processor
> 110: Information acquisition unit
> 120: Nutrition calculation unit
> 130: Menu-selecting unit
> 140: Ingredient-selecting unit
> 150: Food information-providing unit
> 200: Storage unit
> 210: User information database (DB)
> 220: Dietary Reference Intake database (DB)
> 230: Menu database (DB)

**Claims**

1. A complete nutritious food product-providing system comprising:

   an information acquisition unit configured to acquire information on user attributes;
   a user information storage unit configured to store the information on user attributes;
   a Dietary Reference Intake (DRI)-storing unit configured to store DRIs of a complete nutritious food product in which intake standards for first and second nutrients are set for each user attribute; and
   a nutrition calculation unit configured to calculate the intake standard for the first nutrient in a standard food forming the complete nutritious food product, so that the standard food can satisfy the intake standard for the first nutrient of all users.

2. The complete nutritious food product-providing system according to claim 1, wherein the range of the intake standard for the first nutrient in the standard food is narrower than the range of the DRI for the first nutrient in the complete nutritious food product.

3. The complete nutritious food product-providing system according to claim 1 or 2, wherein the first nutrient is vitamins and/or minerals.

4. The complete nutritious food product-providing system according to any one of claims 1 to 3, wherein the second nutrient is protein, lipid, and/or carbohydrate.

5. The complete nutritious food product-providing system according to any one of claims 1 to 4, wherein

   the complete nutritious food product is prepared by adjusting the amount of the standard food,
   the energy amount of the complete nutritious food product is the estimated energy requirement (EER) in the category of the DRIs to which each user belongs, and the energy amount of the standard food is the EER in the category with the lowest EER among the categories of DRIs to which all users belong.

6. The complete nutritious food product-providing system according to any one of claims 1 to 5, wherein the ratio of the amount of the complete nutritious food product to the amount of the standard food is equal to the ratio of the energy amount of the complete nutritious food product to the energy amount of the standard food.

7. The complete nutritious food product-providing system according to any one of claims 3 to 6, wherein
in the case where a tolerable upper intake level (UL) of the first nutrient is set in the DRIs, a normalized nutrient amount in each category to which the user belongs is calculated by the following formula:

Normalized nutrient amount = UL of first nutrient × (energy amount of standard food/EER in each category), and the smallest normalized nutrient amount is set as the upper limit of the first nutrient intake in the standard food.

8. The complete nutritious food product-providing system according to any one of claims 3 to 7, wherein
in the case where an adequate intake (AI) or a recommended dietary allowance (RDA) of the first nutrient is set in the DRIs, the maximum value of the AI or the RDA of the first nutrient in each category of the DRIs is set as the lower limit of the first nutrient intake in the standard food.

9. The complete nutritious food product-providing system according to any one of claims 4 to 8, wherein
the upper limit of the tentative dietary goal for preventing life-style related diseases (DG) of protein in the category with the lowest EER among the categories of the food reference intakes to which the users belong is set as the upper limit of protein intake in the standard food, and the RDA with the largest value among the categories to which the users belong is set as the lower limit of protein intake in the standard food that is provided to the users.

10. The complete nutritious food product-providing system according to any one of claims 4 to 9, wherein
the upper limit of the DG of lipid in the category of standard energy amount with the lowest EER among the categories of the food reference intakes to which the users belong is set as the upper limit of lipid intake in the standard food, and the lower limit of the DG of lipid for the users belonging to the category of the "standard energy amount" is set as the lower limit of lipid intake in the standard food.

11. The complete nutritious food product-providing system according to any one of claims 4 to 10, wherein
the upper limit of the DG of carbohydrate in the category of standard energy amount with the lowest EER among the categories of the food reference intakes to which the users belong is set as the upper limit of carbohydrate intake in the standard food, and the lower limit of the DG of carbohydrate for the users belonging to the category of the standard energy amount is set as the lower limit of carbohydrate intake in the standard food.

12. The complete nutritious food product-providing system according to any one of claims 1 to 11, wherein
the information on user attributes includes at least information on gender, age, or physical activity level.

13. The complete nutritious food product-providing system according to any one of claims 3 to 12, wherein

the vitamins include one or more of vitamin A, vitamin D, vitamin E, vitamin K, vitamin $B_1$, vitamin $B_2$, niacin, vitamin $B_6$, vitamin $B_{12}$, folic acid, pantothenic acid, biotin, and vitamin C, and
the minerals include one or more of sodium, potassium, calcium, magnesium, phosphorus, iron, zinc, copper, manganese, iodine, selenium, chromium, and molybdenum.

14. The complete nutritious food product-providing system according to any one of claims 1 to 13, wherein
the DRIs of the complete nutritious food product are any of the "Dietary Reference Intakes (DRIs) for Japanese" published by the Ministry of Health, Labour and Welfare of Japan, the "Dietary Reference Intakes (DRIs)" in the US, and the "Dietary Reference Intakes for Chinese" in China.

15. The complete nutritious food product-providing system according to any one of claims 4 to 14, further comprising

a menu-selecting unit, wherein
the nutrition calculation unit outputs the energy amount of the standard food, the upper or lower limit of first nutrient intake, and the upper or lower limit of second nutrient intake to the menu-selecting unit.

16. A complete nutritious food product nutritionally calculated by the complete nutritious food product-providing system according to any one of claims 1 to 15.

17. The complete nutritious food product according to claim 16, wherein
the complete nutritious food product is composed of any one selected from the group consisting of breakfast, lunch, and supper or a combination of two or three of these.

**18.** The complete nutritious food product according to claim 16, wherein
the complete nutritious food product is a processed food product, a cooked food product, or a dish cooked based on a specific menu.

**19.** A nutrition calculation device for a complete nutritious food product, comprising:

an information acquisition unit configured to acquire information on user attributes;
a user information storage unit configured to store the information on user attributes;
a DRI-storing unit configured to store DRIs of a complete nutritious food product in which intake standards for first and second nutrients are set for each user attribute; and
a nutrition calculation unit configured to calculate the intake standard for the first nutrient, which is satisfied regardless of attributes of each user, in the standard food forming the complete nutritious food product.

**20.** A nutrition calculation program for a complete nutritious food product, comprising:

a step of acquiring information on user attributes;
a step of storing the information on user attributes;
a step of storing DRIs of the complete nutritious food product in which the intake standards for the first and second nutrients are set for each user attribute; and
a step of calculating the intake standard for the first nutrient, which is satisfied regardless of attributes of each user, in the standard food forming the complete nutritious food product.

*FIG. 1*

Food information display 20

Terminal device 10

Information input unit 11

Sensor 12

Processor 100

Information acquisition unit 110

Nutrition calculation unit 120

Menu-selecting unit 130

Ingredient-selecting unit 140

Foodinformation-providing unit 150

Storage unit 200

User information DB 210

DietaryReference intake DB 220

Menu DB 230

Food production-controlling device 30

Food production-controlling unit 31

Food delivery device 40

Delivery instruction unit 41

# FIG. 2

Start processing

Acquire user information — ST101

Acquire Food Reference Intakes — ST102

Determine standard energy amount — ST103

Determine upper/lower limits of each nutrient intake in standard food — ST104

Select menu — ST105

Select ingredients — ST106

Instruct food production — ST107

End

## FIG. 3

| Females | | | | | | | | |
|---|---|---|---|---|---|---|---|---|
| 18–29 | | | 30–49 | | | 50–64 | | |
| I | II | III | I | II | III | I | II | III |
| 1700 | 2000 | 2300 | 1750 | 2050 | 2350 | 1650 | 1950 | 2250 |
| 55.3 | 65.0 | 74.8 | 56.9 | 66.6 | 76.4 | 57.8 | 68.3 | 78.8 |
| 85.0 | 100.0 | 115.0 | 87.5 | 102.5 | 117.5 | 82.5 | 97.5 | 112.5 |
| 50.0 | 50.0 | 50.0 | 50.0 | 50.0 | 50.0 | 50.0 | 50.0 | 50.0 |
| 37.8 | 44.4 | 51.1 | 38.9 | 45.6 | 52.2 | 36.7 | 43.3 | 50.0 |
| 56.7 | 66.7 | 76.7 | 58.3 | 68.3 | 78.3 | 55.0 | 65.0 | 75.0 |
| 13.2 | 15.6 | 17.9 | 13.6 | 15.9 | 18.3 | 12.8 | 15.2 | 17.5 |
| 1.6 | 1.6 | 1.6 | 1.6 | 1.6 | 1.6 | 1.9 | 1.9 | 1.9 |
| 8 | 8 | 8 | 8 | 8 | 8 | 8 | 8 | 8 |
| 212.5 | 250.0 | 287.5 | 218.8 | 256.3 | 293.8 | 206.3 | 243.8 | 281.3 |
| 276.3 | 325.0 | 373.8 | 284.4 | 333.1 | 381.9 | 268.1 | 316.9 | 365.6 |
| 18 | 18 | 18 | 18 | 18 | 18 | 18 | 18 | 18 |

(CONT.)

EP 4 134 968 A1

EP 4 134 968 A1

| | | Males | | | | | | | | |
|---|---|---|---|---|---|---|---|---|---|---|
| | | 18–29 | | | 30–49 | | | 50–64 | | |
| Physical activity level | | I | II | III | I | II | III | I | II | III |
| Energy (kcal) | | 2300 | 2650 | 3050 | 2300 | 2700 | 3050 | 2200 | 2600 | 2950 |
| Protein (g) | Lower limit of DG | 74.8 | 86.1 | 99.1 | 74.8 | 87.8 | 99.1 | 77.0 | 91.0 | 103.3 |
| | Upper limit of DG | 115.0 | 132.5 | 152.5 | 115.0 | 135.0 | 152.5 | 110.0 | 130.0 | 147.5 |
| | RDA | **65.0** | **65.0** | **65.0** | **65.0** | **65.0** | **65.0** | **65.0** | **65.0** | **65.0** |
| Lipid (g) | Lower limit of DG | 51.1 | 58.9 | 67.8 | 51.1 | 60.0 | 67.8 | 48.9 | 57.8 | 65.6 |
| | Upper limit of DG | 76.7 | 88.3 | 101.7 | 76.7 | 90.0 | 101.7 | 73.3 | 86.7 | 98.3 |
| Saturated fatty acid (g) | DG (or less) | 17.9 | 20.6 | 23.7 | 17.9 | 21.0 | 23.7 | 17.1 | 20.2 | 22.9 |
| n-3 Fatty acid(g) | AI | 2 | 2 | 2 | 2 | 2 | 2 | 2.2 | 2.2 | 2.2 |
| n-6 Fatty acid(g) | AI | 11 | 11 | 11 | 10 | 10 | 10 | 10 | 10 | 10 |
| n-6 Fatty acid(g) | Lower limit of DG | 287.5 | 331.3 | 381.3 | 287.5 | 337.5 | 381.3 | 275.0 | 325.0 | 368.8 |
| | Upper limit of DG | 373.8 | 430.6 | 495.6 | 373.8 | 438.8 | 495.6 | 357.5 | 422.5 | |
| Dietary fiber(g) | DG | 21 | 21 | 21 | 21 | 21 | 21 | 21 | 21 | 21 |

(FIG. 3 Continued)

## FIG. 4

| Females | | | | | | | | |
|---|---|---|---|---|---|---|---|---|
| 18–29 | | | 30–49 | | | 50–64 | | |
| I | II | III | I | II | III | I | II | III |
| 1700 | 2000 | 2300 | 1750 | 2050 | 2350 | 1650 | 1950 | 2250 |
| 6.5 | 6.5 | 6.5 | 6.5 | 6.5 | 6.5 | 6.5 | 6.5 | 6.5 |
| 6.31 | 5.36 | 4.66 | 6.13 | 5.23 | 4.56 | 6.50 | 5.50 | 4.77 |
| 650 | 650 | 650 | 650 | 650 | 650 | 650 | 650 | 650 |
| 2500 | 2500 | 2500 | 2500 | 2500 | 2500 | 2500 | 2500 | 2500 |
| 2426.5 | 2062.5 | 1793.5 | 2357.1 | 2012.2 | 1755.3 | 2500.0 | 2115.4 | 1833.3 |
| 10.5 | 10.5 | 10.5 | 10.5 | 10.5 | 10.5 | 10.5 | 10.5 | 10.5 |
| 40 | 40 | 40 | 40 | 40 | 40 | 40 | 40 | 40 |
| 38.8 | 33.0 | 28.7 | 37.7 | 32.2 | 28.1 | 40.0 | 33.8 | 29.3 |
| 800 | 800 | 800 | 800 | 800 | 800 | 800 | 800 | 800 |
| 3000 | 3000 | 3000 | 3000 | 3000 | 3000 | 3000 | 3000 | 3000 |
| 2911.8 | 2475.0 | 2152.2 | 2828.6 | 2414.6 | 2106.4 | 3000.0 | 2538.5 | 2200.0 |
| 270 | 270 | 270 | 290 | 290 | 290 | 290 | 290 | 290 |
| 350 | 350 | 350 | 350 | 350 | 350 | 350 | 350 | 350 |
| 339.7 | 288.8 | 251.1 | 330.0 | 281.7 | 245.7 | 350.0 | 296.2 | 256.7 |
| 2000 | 2000 | 2000 | 2000 | 2000 | 2000 | 2000 | 2000 | 2000 |
| 0.7 | 0.7 | 0.7 | 0.7 | 0.7 | 0.7 | 0.7 | 0.7 | 0.7 |
| 7 | 7 | 7 | 7 | 7 | 7 | 7 | 7 | 7 |
| 6.8 | 5.8 | 5.0 | 6.6 | 5.6 | 4.9 | 7.0 | 5.9 | 5.1 |

(CONT.)

EP 4 134 968 A1

| | | Males | | | | | | | | |
|---|---|---|---|---|---|---|---|---|---|---|
| | | 18–29 | | | 30–49 | | | 50–64 | | |
| Physical activity level | | I | II | III | I | II | III | I | II | III |
| Energy (kcal) | | 2300 | 2650 | 3050 | 2300 | 2700 | 3050 | 2200 | 2600 | 2950 |
| Sodium Salt equivalent (g) | DG (less than) | 7.5 | 7.5 | 7.5 | 7.5 | 7.5 | 7.5 | 7.5 | 7.5 | 7.5 |
| | Maximum intake per 1650 kcal | 5.38 | 4.67 | 4.06 | 5.38 | 4.58 | 4.06 | 5.63 | 4.76 | 4.19 |
| Calcium (mg) | RDA | 800 | 800 | 800 | 750 | 750 | 750 | 750 | 750 | 750 |
| | UL | 2500 | 2500 | 2500 | 2500 | 2500 | 2500 | 2500 | 2500 | 2500 |
| | Maximum intake per 1650 kcal | 1793.5 | 1556.6 | 1352.5 | 1793.5 | 1527.8 | 1352.5 | 1875.0 | 1586.5 | 1398.3 |
| Iron (mg) | RDA (menstruation for females) | 7 | 7 | 7 | 7.5 | 7.5 | 7.5 | 7.5 | 7.5 | 7.5 |
| | UL | 50 | 50 | 50 | 55 | 55 | 55 | 50 | 50 | 50 |
| | Maximum intake per 1650 kcal | 35.9 | 31.1 | 27.0 | 39.5 | 33.6 | 29.8 | 37.5 | 31.7 | 28.0 |
| Phosphorous (mg) | AI | 1000 | 1000 | 1000 | 1000 | 1000 | 1000 | 1000 | 1000 | 1000 |
| | UL | 3000 | 3000 | 3000 | 3000 | 3000 | 3000 | 3000 | 3000 | 3000 |
| | Maximum intake per 1650 kcal | 2152.2 | 1867.9 | 1623.0 | 2152.2 | 1833.3 | 1623.0 | 2250.0 | 1903.8 | 1678.0 |
| Magnesium (mg) | RDA | 340 | 340 | 340 | 370 | 370 | 370 | 370 | 370 | 370 |
| | UL | 350 | 350 | 350 | 350 | 350 | 350 | 350 | 350 | 350 |
| | Maximum intake per 1650 kcal | 251.1 | 217.9 | 189.3 | 251.1 | 213.9 | 189.3 | 262.5 | 222.1 | 195.8 |
| Potassium (mg) | AI | 2500 | 2500 | 2500 | 2500 | 2500 | 2500 | 2500 | 2500 | 2500 |
| Copper (mg) | RDA | 0.9 | 0.9 | 0.9 | 0.9 | 0.9 | 0.9 | 0.9 | 0.9 | 0.9 |
| | UL | 7 | 7 | 7 | 7 | 7 | 7 | 7 | 7 | 7 |
| | Maximum intake per 1650 kcal | 5.0 | 4.4 | 3.8 | 5.0 | 4.3 | 3.8 | 5.3 | 4.4 | 3.9 |

FIG. 4 Continued)

FIG. 5

| Females | | | | | | | | |
|---|---|---|---|---|---|---|---|---|
| 18–29 | | | 30–49 | | | 50–64 | | |
| I | II | III | I | II | III | I | II | III |
| 1700 | 2000 | 2300 | 1750 | 2050 | 2350 | 1650 | 1950 | 2250 |
| 130 | 130 | 130 | 130 | 130 | 130 | 130 | 130 | 130 |
| 3000 | 3000 | 3000 | 3000 | 3000 | 3000 | 3000 | 3000 | 3000 |
| 2911.8 | 2475.0 | 2152.2 | 2828.6 | 2414.6 | 2106.4 | 3000.0 | 2538.5 | 2200.0 |
| 25 | 25 | 25 | 25 | 25 | 25 | 25 | 25 | 25 |
| 350 | 350 | 350 | 350 | 350 | 350 | 350 | 350 | 350 |
| 339.7 | 288.8 | 251.1 | 330.0 | 281.7 | 245.7 | 350.0 | 296.2 | 256.7 |
| 8 | 8 | 8 | 8 | 8 | 8 | 8 | 8 | 8 |
| 35 | 35 | 35 | 35 | 35 | 35 | 35 | 35 | 35 |
| 34.0 | 28.9 | 25.1 | 33.0 | 28.2 | 24.6 | 35.0 | 29.6 | 25.7 |
| 10 | 10 | 10 | 10 | 10 | 10 | 10 | 10 | 10 |
| 500 | 500 | 500 | 500 | 500 | 500 | 500 | 500 | 500 |
| 485.3 | 412.5 | 358.7 | 471.4 | 402.4 | 351.1 | 500.0 | 423.1 | 366.7 |
| 3.5 | 3.5 | 3.5 | 3.5 | 3.5 | 3.5 | 3.5 | 3.5 | 3.5 |
| 11 | 11 | 11 | 11 | 11 | 11 | 11 | 11 | 11 |
| 10.7 | 9.1 | 7.9 | 10.4 | 8.9 | 7.7 | 11.0 | 9.3 | 8.1 |
| 25 | 25 | 25 | 25 | 25 | 25 | 25 | 25 | 25 |
| 500 | 500 | 500 | 500 | 500 | 500 | 500 | 500 | 500 |
| 485.3 | 412.5 | 358.7 | 471.4 | 402.4 | 351.1 | 500.0 | 423.1 | 366.7 |

(CONT.)

|  | | Males | | | | | | | | |
|---|---|---|---|---|---|---|---|---|---|---|
|  | | 18–29 | | | 30–49 | | | 50–64 | | |
| Physical activity level | | I | II | III | I | II | III | I | II | III |
| Energy (kcal) | | 2300 | 2650 | 3050 | 2300 | 2700 | 3050 | 2200 | 2600 | 2950 |
| Iodine (µg) | RDA | 130 | 130 | 130 | 130 | 130 | 130 | 130 | 130 | 130 |
| | UL | 3000 | 3000 | 3000 | 3000 | 3000 | 3000 | 3000 | 3000 | 3000 |
| | Maximum intake per 1650 kcal | 2152.2 | 1867.9 | 1623.0 | 2152.2 | 1833.3 | 1623.0 | 2250.0 | 1903.8 | 1678.0 |
| Selenium (µg) | RDA | 30 | 30 | 30 | 30 | 30 | 30 | 30 | 30 | 30 |
| | UL | 450 | 450 | 450 | 450 | 450 | 450 | 450 | 450 | 450 |
| | Maximum intake per 1650 kcal | 322.8 | 280.2 | 243.4 | 322.8 | 275.0 | 243.4 | 337.5 | 285.6 | 251.7 |
| Zinc (mg) | RDA | 11 | 11 | 11 | 11 | 11 | 11 | 11 | 11 | 11 |
| | UL | 40 | 40 | 40 | 45 | 45 | 45 | 45 | 45 | 45 |
| | Maximum intake per 1650 kcal | 28.7 | 24.9 | 21.6 | 32.3 | 27.5 | 24.3 | 33.8 | 28.6 | 25.2 |
| Chromium (µg) | AI | 10 | 10 | 10 | 10 | 10 | 10 | 10 | 10 | 10 |
| | UL | 500 | 500 | 500 | 500 | 500 | 500 | 500 | 500 | 500 |
| | Maximum intake per 1650 kcal | 358.7 | 311.3 | 270.5 | 358.7 | 305.6 | 270.5 | 375.0 | 317.3 | 279.7 |
| Manganese (mg) | AI | 4 | 4 | 4 | 4 | 4 | 4 | 4 | 4 | 4 |
| | UL | 11 | 11 | 11 | 11 | 11 | 11 | 11 | 11 | 11 |
| | Maximum intake per 1650 kcal | 7.9 | 6.8 | 6.0 | 7.9 | 6.7 | 6.0 | 8.3 | 7.0 | 6.2 |
| Molybdenum (µg) | RDA | 30 | 30 | 30 | 30 | 30 | 30 | 30 | 30 | 30 |
| | UL | 600 | 600 | 600 | 600 | 600 | 600 | 600 | 600 | 600 |
| | Maximum intake per 1650 kcal | 430.4 | 373.6 | 324.6 | 430.4 | 366.7 | 324.6 | 450.0 | 380.8 | 335.6 |

(FIG. 5 Continued)

# FIG. 6

(CONT.)

| | | Males | | | | | | 50-64 |
|---|---|---|---|---|---|---|---|---|
| | | 18-29 | | | 30-49 | | | |
| Physical activity level | | I | II | III | I | II | III | I |
| Energy (kcal) | | 2300 | 2650 | 3050 | 2300 | 2700 | 3050 | 2200 |
| Vitamin A (μgRE) | RDA | 850 | 850 | 850 | 900 | 900 | 900 | 900 |
| | UL | 2700 | 2700 | 2700 | 2700 | 2700 | 2700 | 2700 |
| | Maximum intake per 1650 kcal | 1937.0 | 1681.1 | 1460.7 | 1937.0 | 1650.0 | 1460.7 | 2025.0 |
| Vitamin D (μg) | AI | 8.5 | 8.5 | 8.5 | 8.5 | 8.5 | 8.5 | 8.5 |
| | UL | 100 | 100 | 100 | 100 | 100 | 100 | 100 |
| | Maximum intake per 1650 kcal | 71.7 | 62.3 | 54.1 | 71.7 | 61.1 | 54.1 | 75.0 |
| Vitamin E (mg) | RDA | 6 | 6 | 6 | 6 | 6 | 6 | 7 |
| | UL | 850 | 850 | 850 | 900 | 900 | 900 | 850 |
| | Maximum intake per 1650 kcal | 609.8 | 529.2 | 459.8 | 645.7 | 550.0 | 486.9 | 637.5 |
| Vitamin K (μg) | AI | 150 | 150 | 150 | 150 | 150 | 150 | 150 |
| Vitamin B1 (mg) | RDA | 1.4 | 1.4 | 1.4 | 1.4 | 1.4 | 1.4 | 1.3 |
| Vitamin B2 (mg) | RDA | 1.6 | 1.6 | 1.6 | 1.6 | 1.6 | 1.6 | 1.5 |
| Niacin (mgNE) | RDA | 15 | 15 | 15 | 15 | 15 | 15 | 14 |
| | UL | 80 | 80 | 80 | 85 | 85 | 85 | 85 |
| | Maximum intake per 1650 kcal | 57.4 | 49.8 | 43.3 | 61.0 | 51.9 | 46.0 | 63.8 |
| Vitamin B6 (mg) | RDA | 1.4 | 1.4 | 1.4 | 1.4 | 1.4 | 1.4 | 1.4 |
| | UL | 55 | 55 | 55 | 60 | 60 | 60 | 55 |
| | Maximum intake per 1650 kcal | 39.46 | 34.25 | 29.75 | 43.04 | 36.67 | 32.46 | 41.25 |
| Folic acid (μg) | RDA | 240 | 240 | 240 | 240 | 240 | 240 | 240 |
| | UL | 900 | 900 | 900 | 1000 | 1000 | 1000 | 1000 |
| | Maximum intake per 1650 kcal | 645.7 | 560.4 | 486.9 | 717.4 | 611.1 | 541.0 | 750.0 |
| Vitamin B12 (μg) | RDA | 2.4 | 2.4 | 2.4 | 2.4 | 2.4 | 2.4 | 2.4 |
| Biotin (μg) | AI | 50 | 50 | 50 | 50 | 50 | 50 | 50 |
| Pantothenic acid (mg) | AI | 5 | 5 | 5 | 5 | 5 | 5 | 6 |
| Vitamin C (mg) | RDA | 100 | 100 | 100 | 100 | 100 | 100 | 100 |

## (FIG. 6 Continued)

| Females | | | | | | | | | | |
|---|---|---|---|---|---|---|---|---|---|---|
| | | 18-29 | | | 30-49 | | | 50-64 | | |
| II | III | I | II | III | I | II | III | I | II | III |
| 2600 | 2950 | 1700 | 2000 | 2300 | 1750 | 2050 | 2350 | 1650 | 1950 | 2250 |
| 900 | 900 | 650 | 650 | 650 | 700 | 700 | 700 | 700 | 700 | 700 |
| 2700 | 2700 | 2700 | 2700 | 2700 | 2700 | 2700 | 2700 | 2700 | 2700 | 2700 |
| 1713.5 | 1510.2 | 2620.6 | 2227.5 | 1937.0 | 2545.7 | 2173.2 | 1895.7 | 2700.0 | 2284.6 | 1980.0 |
| 8.5 | 8.5 | 8.5 | 8.5 | 8.5 | 8.5 | 8.5 | 8.5 | 8.5 | 8.5 | 8.5 |
| 100 | 100 | 100 | 100 | 100 | 100 | 100 | 100 | 100 | 100 | 100 |
| 63.5 | 55.9 | 97.1 | 82.5 | 71.7 | 94.3 | 80.5 | 70.2 | 100.0 | 84.6 | 73.3 |
| 7 | 7 | 5 | 5 | 5 | 5.5 | 5.5 | 5.5 | 6 | 6 | 6 |
| 850 | 850 | 650 | 650 | 650 | 700 | 700 | 700 | 700 | 700 | 700 |
| 539.4 | 475.4 | 630.9 | 536.3 | 466.3 | 660.0 | 563.4 | 491.5 | 700.0 | 592.3 | 513.3 |
| 150 | 150 | 150 | 150 | 150 | 150 | 150 | 150 | 150 | 150 | 150 |
| 1.3 | 1.3 | 1.1 | 1.1 | 1.1 | 1.1 | 1.1 | 1.1 | 1.1 | 1.1 | 1.1 |
| 1.5 | 1.5 | 1.2 | 1.2 | 1.2 | 1.2 | 1.2 | 1.2 | 1.2 | 1.2 | 1.2 |
| 14 | 14 | 11 | 11 | 11 | 12 | 12 | 12 | 11 | 11 | 11 |
| 85 | 85 | 65 | 65 | 65 | 65 | 65 | 65 | 65 | 65 | 65 |
| 53.9 | 47.5 | 63.1 | 53.6 | 46.6 | 61.3 | 52.3 | 45.6 | 65.0 | 55.0 | 47.7 |
| 1.4 | 1.4 | 1.1 | 1.1 | 1.1 | 1.1 | 1.1 | 1.1 | 1.1 | 1.1 | 1.1 |
| 55 | 55 | 45 | 45 | 45 | 45 | 45 | 45 | 45 | 45 | 45 |
| 34.90 | 30.76 | 43.68 | 37.13 | 32.28 | 42.43 | 36.22 | 31.60 | 45.00 | 38.08 | 33.00 |
| 240 | 240 | 240 | 240 | 240 | 240 | 240 | 240 | 240 | 240 | 240 |
| 1000 | 1000 | 900 | 900 | 900 | 1000 | 1000 | 1000 | 1000 | 1000 | 1000 |
| 634.6 | 559.3 | 873.5 | 742.5 | 645.7 | 942.9 | 804.9 | 702.1 | 1000.0 | 846.2 | 733.3 |
| 2.4 | 2.4 | 2.4 | 2.4 | 2.4 | 2.4 | 2.4 | 2.4 | 2.4 | 2.4 | 2.4 |
| 50 | 50 | 50 | 50 | 50 | 50 | 50 | 50 | 50 | 50 | 50 |
| 6 | 6 | 5 | 5 | 5 | 5 | 5 | 5 | 5 | 5 | 5 |
| 100 | 100 | 100 | 100 | 100 | 100 | 100 | 100 | 100 | 100 | 100 |

FIG. 7

| Females | | | | | | | | | | | | | | | | | | | | |
|---|---|---|---|---|---|---|---|---|---|---|---|---|---|---|---|---|---|---|---|---|
| 56–60 | | | 61–65 | | | 21–25 | | | 26–30 | | | 31–50 | | | 51–60 | | | 61–65 | | |
| S | M | A | S | M | A | S | M | A | S | M | A | S | M | A | S | M | A | S | M | A |
| 2200 | 2400 | 2600 | 2000 | 2400 | 2800 | 2000 | 2200 | 2400 | 1800 | 2000 | 2400 | 1800 | 2000 | 2200 | 1600 | 1800 | 2200 | 1600 | 1800 | 2000 |
| 55.0 | 60.0 | 65.0 | 50.0 | 60.0 | 70.0 | 50.0 | 55.0 | 60.0 | 45.0 | 50.0 | 60.0 | 45.0 | 50.0 | 55.0 | 40.0 | 45.0 | 55.0 | 40.0 | 45.0 | 50.0 |
| 193 | 210 | 228 | 175 | 210 | 245 | 175 | 193 | 210 | 158 | 175 | 210 | 158 | 175 | 193 | 140 | 158 | 193 | 140 | 158 | 175 |
| 56.0 | 56.0 | 56.0 | 56.0 | 56.0 | 56.0 | 46.0 | 46.0 | 46.0 | 46.0 | 46.0 | 46.0 | 46.0 | 46.0 | 46.0 | 46.0 | 46.0 | 46.0 | 46.0 | 46.0 | 46.0 |
| 48.9 | 53.3 | 57.8 | 44.4 | 53.3 | 62.2 | 44.4 | 48.9 | 53.3 | 40.0 | 44.4 | 53.3 | 40.0 | 44.4 | 48.9 | 35.6 | 40.0 | 48.9 | 35.6 | 40.0 | 44.4 |
| 85.6 | 93.3 | 101.1 | 77.8 | 93.3 | 108.9 | 77.8 | 85.6 | 93.3 | 70.0 | 77.8 | 93.3 | 70.0 | 77.8 | 85.6 | 62.2 | 70.0 | 85.6 | 62.2 | 70.0 | 77.8 |
| 1.6 | 1.6 | 1.6 | 1.6 | 1.6 | 1.6 | 1.1 | 1.1 | 1.1 | 1.1 | 1.1 | 1.1 | 1.1 | 1.1 | 1.1 | 1.1 | 1.1 | 1.1 | 1.1 | 1.1 | 1.1 |
| 14 | 14 | 14 | 14 | 14 | 14 | 12 | 12 | 12 | 12 | 12 | 12 | 12 | 12 | 12 | 11 | 11 | 11 | 11 | 11 | 11 |
| 248 | 270 | 293 | 225 | 270 | 315 | 225.0 | 247.5 | 270.0 | 202.5 | 225.0 | 270.0 | 202.5 | 225.0 | 247.5 | 180 | 203 | 248 | 180 | 203 | 225 |
| 130 | 130 | 130 | 130 | 130 | 130 | 130 | 130 | 130 | 130 | 130 | 130 | 130 | 130 | 130 | 130 | 130 | 130 | 130 | 130 | 130 |
| 358 | 390 | 423 | 325 | 390 | 455 | 325.0 | 357.5 | 390.0 | 292.5 | 325.0 | 390.0 | 292.5 | 325.0 | 357.5 | 260 | 293 | 358 | 260 | 293 | 325 |
| 30 | 30 | 30 | 30 | 30 | 30 | 25 | 25 | 25 | 25 | 25 | 25 | 25 | 25 | 25 | 21 | 21 | 21 | 21 | 21 | 21 |

(CONT.)

EP 4 134 968 A1

| | Lower limit | Upper limit | Physical activity level | Males | | | | | | | | | | | | | | |
|---|---|---|---|---|---|---|---|---|---|---|---|---|---|---|---|---|---|---|
| | | | Age | 21–25 | | | 26–35 | | | 36–45 | | | 46–50 | | | 51–55 | | |
| | | | | S | M | A | S | M | A | S | M | A | S | M | A | S | M | A |
| Energy (kcal) | 1,600 | 3,000 | | 2400 | 2800 | 3000 | 2400 | 2600 | 3000 | 2400 | 2800 | 2800 | 2200 | 2400 | 2800 | 2200 | 2400 | 2800 |
| Protein (g) | | | Lower limit of DG | 60.0 | 70.0 | 75.0 | 60.0 | 65.0 | 75.0 | 60.0 | 70.0 | 70.0 | 55.0 | 60.0 | 70.0 | 55.0 | 60.0 | 70.0 |
| | | 140 | Upper limit of DG | 210 | 245 | 263 | 210 | 228 | 263 | 210 | 245 | 245 | 193 | 210 | 245 | 193 | 210 | 245 |
| | 56.0 | | RDA | 56.0 | 56.0 | 56.0 | 56.0 | 56.0 | 56.0 | 56.0 | 56.0 | 56.0 | 56.0 | 56.0 | 56.0 | 56.0 | 56.0 | 56.0 |
| Lipid (g) | 35.6 | | Lower limit of DG | 53.3 | 62.2 | 66.7 | 53.3 | 57.8 | 66.7 | 53.3 | 62.2 | 62.2 | 48.9 | 53.3 | 62.2 | 48.9 | 53.3 | 62.2 |
| | | 62.2 | Upper limit of DG | 93.3 | 108.9 | 116.7 | 93.3 | 101.1 | 116.7 | 93.3 | 108.9 | 108.9 | 85.6 | 93.3 | 108.9 | 85.6 | 93.3 | 108.9 |
| α-Linolenic acid (g) | 1.6 | | AI | 1.6 | 1.6 | 1.6 | 1.6 | 1.6 | 1.6 | 1.6 | 1.6 | 1.6 | 1.6 | 1.6 | 1.6 | 1.6 | 1.6 | 1.6 |
| Linoleic acid (g) | 17.0 | | AI | 17 | 17 | 17 | 17 | 17 | 17 | 17 | 17 | 17 | 17 | 17 | 17 | 14 | 14 | 14 |
| Carbohydrate (g) | | | Lower limit of DG | 270.0 | 315.0 | 337.5 | 270.0 | 292.5 | 337.5 | 270.0 | 315.0 | 315.0 | 248 | 270 | 315 | 248 | 270 | 315 |
| | 130.0 | | RDA | | | | | | | | | | | | | | 130 | 130 |
| | | 260.0 | Upper limit of DG | 390.0 | 455.0 | 487.5 | 390.0 | 422.5 | 487.5 | 390.0 | 455.0 | 455.0 | 358 | 390 | 455 | 358 | 390 | 455 |
| Dietary fiber (g) | 38 | | AI | 38 | 38 | 38 | 38 | 38 | 38 | 38 | 38 | 38 | 38 | 38 | 38 | 30 | 30 | 30 |

(FIG. 7 Continued)

FIG. 8

| Females | | | | | | | | | | | | | | | | | | | | |
| 56–60 | | | 61–65 | | | 21–25 | | | 26–30 | | | 31–50 | | | 51–60 | | | 61–65 | | |
| S | M | A | S | M | A | S | M | A | S | M | A | S | M | A | S | M | A | S | M | A |
|---|---|---|---|---|---|---|---|---|---|---|---|---|---|---|---|---|---|---|---|---|
| 2200 | 2400 | 2600 | 2000 | 2400 | 2800 | 2000 | 2200 | 2400 | 1800 | 2000 | 2400 | 1800 | 2000 | 2200 | 1600 | 1800 | 2200 | 1600 | 1800 | 2000 |
| 1500 | 1500 | 1500 | 1500 | 1500 | 1500 | 1500 | 1500 | 1500 | 1500 | 1500 | 1500 | 1500 | 1500 | 1500 | 1500 | 1500 | 1500 | 1500 | 1500 | 1500 |
| 3400 | 3400 | 3400 | 3400 | 3400 | 3400 | 2600 | 2600 | 2600 | 2600 | 2600 | 2600 | 2600 | 2600 | 2600 | 2600 | 2600 | 2600 | 2600 | 2600 | 2600 |
| 1000 | 1000 | 1000 | 1000 | 1000 | 1000 | 1000 | 1000 | 1000 | 1000 | 1000 | 1000 | 1000 | 1000 | 1000 | 1200 | 1200 | 1200 | 1200 | 1200 | 1200 |
| 2000 | 2000 | 2000 | 2000 | 2000 | 2000 | 2500 | 2500 | 2500 | 2500 | 2500 | 2500 | 2500 | 2500 | 2500 | 2000 | 2000 | 2000 | 2000 | 2000 | 2000 |
| 1455 | 1333 | 1231 | 1600 | 1333 | 1143 | 2000 | 1818 | 1667 | 2222 | 2000 | 1667 | 2222 | 2000 | 1818 | 2000 | 1778 | 1455 | 2000 | 1778 | 1600 |
| 420 | 420 | 420 | 420 | 420 | 420 | 310 | 310 | 310 | 310 | 310 | 310 | 320 | 320 | 320 | 320 | 320 | 320 | 320 | 320 | 320 |
| 700 | 700 | 700 | 700 | 700 | 700 | 700 | 700 | 700 | 700 | 700 | 700 | 700 | 700 | 700 | 700 | 700 | 700 | 700 | 700 | 700 |
| 4000 | 4000 | 4000 | 3000 | 3000 | 3000 | 4000 | 4000 | 4000 | 4000 | 4000 | 4000 | 4000 | 4000 | 4000 | 3000 | 3000 | 3000 | 3000 | 3000 | 3000 |
| 2909 | 2667 | 2462 | 2400 | 2000 | 1714 | 3200 | 2909 | 2667 | 3556 | 3200 | 2667 | 3556 | 3200 | 2909 | 3000 | 2667 | 2182 | 3000 | 2667 | 2400 |
| 8.0 | 8.0 | 8.0 | 8.0 | 8.0 | 8.0 | 18.0 | 18.0 | 18.0 | 18.0 | 18.0 | 18.0 | 18.0 | 18.0 | 18.0 | 8.0 | 8.0 | 8.0 | 8.0 | 8.0 | 8.0 |
| 45 | 45 | 45 | 45 | 45 | 45 | 45 | 45 | 45 | 45 | 45 | 45 | 45 | 45 | 45 | 45 | 45 | 45 | 45 | 45 | 45 |
| 32.7 | 30.0 | 27.7 | 36.0 | 30.0 | 25.7 | 36.0 | 32.7 | 30.0 | 40.0 | 36.0 | 30.0 | 40.0 | 36.0 | 32.7 | 45.0 | 40.0 | 32.7 | 45.0 | 40.0 | 36.0 |
| 11 | 11 | 11 | 11 | 11 | 11 | 8 | 8 | 8 | 8 | 8 | 8 | 8 | 8 | 8 | 8 | 8 | 8 | 8 | 8 | 8 |
| 40 | 40 | 40 | 40 | 40 | 40 | 40 | 40 | 40 | 40 | 40 | 40 | 40 | 40 | 40 | 40 | 40 | 40 | 40 | 40 | 40 |
| 29.1 | 26.7 | 24.6 | 32.0 | 26.7 | 22.9 | 32.0 | 29.1 | 26.7 | 35.6 | 32.0 | 26.7 | 35.6 | 32.0 | 29.1 | 40.0 | 35.6 | 29.1 | 40.0 | 35.6 | 32.0 |
| 0.9 | 0.9 | 0.9 | 0.9 | 0.9 | 0.9 | 0.9 | 0.9 | 0.9 | 0.9 | 0.9 | 0.9 | 0.9 | 0.9 | 0.9 | 0.9 | 0.9 | 0.9 | 0.9 | 0.9 | 0.9 |
| 10 | 10 | 10 | 10 | 10 | 10 | 10 | 10 | 10 | 10 | 10 | 10 | 10 | 10 | 10 | 10 | 10 | 10 | 10 | 10 | 10 |
| 7.3 | 6.7 | 6.2 | 8.0 | 6.7 | 5.7 | 8.0 | 7.3 | 6.7 | 8.9 | 8.0 | 6.7 | 8.9 | 8.0 | 7.3 | 10.0 | 8.9 | 7.3 | 10.0 | 8.9 | 8.0 |

(CONT.)

EP 4 134 968 A1

EP 4 134 968 A1

| | Lower limit | Upper limit | Physical activity level | Males | | | | | | | | | | | | | | |
|---|---|---|---|---|---|---|---|---|---|---|---|---|---|---|---|---|---|---|
| | | | Age | 21–25 | | | 26–35 | | | 36–45 | | | 46–50 | | | 51–55 | | |
| | | | | S | M | A | S | M | A | S | M | A | S | M | A | S | M | A |
| Energy (kcal) | 1,600 | 3,000 | | 2400 | 2800 | 3000 | 2400 | 2600 | 3000 | 2400 | 2800 | 2800 | 2200 | 2400 | 2800 | 2200 | 2400 | 2800 |
| Sodium (mg) | 1500.0 | | AI | | | | | | | | | | 1500 | 1500 | 1500 | 1500 | 1500 | 1500 |
| Potassium (mg) | 3400 | | AI | 3400 | 3400 | 3400 | 3400 | 3400 | 3400 | 3400 | 3400 | 3400 | 3400 | 3400 | 3400 | 3400 | 3400 | 3400 |
| Calcium (mg) | (1200) | | RDA | 1000 | 1000 | 1000 | 1000 | 1000 | 1000 | 1000 | 1000 | 1000 | 1000 | 1000 | 1000 | 1000 | 1000 | 1000 |
| | | | UL | 2500 | 2500 | 2500 | 2500 | 2500 | 2500 | 2500 | 2500 | 2500 | 2500 | 2500 | 2500 | 2000 | 2000 | 2000 |
| | | (1143) | Maximum intake per 1600 kcal | 1667 | 1429 | 1333 | 1667 | 1538 | 1333 | 1667 | 1429 | 1429 | 1818 | 1667 | 1429 | 1455 | 1333 | 1143 |
| Magnesium | 420 | | AI | 400 | 400 | 400 | 400 | 400 | 400 | 420 | 420 | 420 | 420 | 420 | 420 | 420 | 420 | 420 |
| Phosphorous (mg) | 700 | | AI | 700 | 700 | 700 | 700 | 700 | 700 | 700 | 700 | 700 | 700 | 700 | 700 | 700 | 700 | 700 |
| | | | RDA | 3000 | 3000 | 3000 | 3000 | 3000 | 3000 | 3000 | 3000 | 4000 | 4000 | 4000 | 4000 | 4000 | 4000 | 4000 |
| | | 1600 | Maximum intake per 1600 kcal | 2000 | 1714 | 1600 | 2000 | 1846 | 1600 | 2000 | 1714 | 2286 | 2909 | 2667 | 2286 | 2909 | 2667 | 2286 |
| Iron (mg) | 18.0 | | RDA (menstruation for some females) | 8.0 | 8.0 | 8.0 | 8.0 | 8.0 | 8.0 | 8.0 | 8.0 | 8.0 | 8.0 | 8.0 | 8.0 | 8.0 | 8.0 | 8.0 |
| | | | UL | 50 | 50 | 50 | 55 | 45 | 45 | 45 | 45 | 45 | 45 | 45 | 45 | 45 | 45 | 45 |
| | | 24.0 | Maximum intake per 1600 kcal | 33.3 | 28.6 | 26.7 | 36.7 | 27.7 | 24.0 | 30.0 | 25.7 | 25.7 | 32.7 | 30.0 | 25.7 | 32.7 | 30.0 | 25.7 |
| Zinc (mg) | 11 | | RDA | 11 | 11 | 11 | 11 | 11 | 11 | 11 | 11 | 11 | 11 | 11 | 11 | 11 | 11 | 11 |
| | | | UL | 40 | 40 | 40 | 45 | 45 | 45 | 45 | 45 | 45 | 40 | 40 | 40 | 40 | 40 | 40 |
| | | 21.3 | Maximum intake per 1600 kcal | 26.7 | 22.9 | 21.3 | 30.0 | 27.7 | 24.0 | 30.0 | 25.7 | 25.7 | 29.1 | 26.7 | 22.9 | 29.1 | 26.7 | 22.9 |
| Copper (mg) | 0.9 | | RDA | 0.9 | 0.9 | 0.9 | 0.9 | 0.9 | 0.9 | 0.9 | 0.9 | 0.9 | 0.9 | 0.9 | 0.9 | 0.9 | 0.9 | 0.9 |
| | | | UL | 10 | 10 | 10 | 10 | 10 | 10 | 10 | 10 | 10 | 10 | 10 | 10 | 10 | 10 | 10 |
| | | 5.3 | Maximum intake per 1600 kcal | 6.7 | 5.7 | 5.3 | 6.7 | 6.2 | 5.3 | 6.7 | 5.7 | 5.7 | 7.3 | 6.7 | 5.7 | 7.3 | 6.7 | 5.7 |

FIG. 8 Continued

## FIG. 9

(CONT.)

| | Lower limit | Upper limit | Physical activity level | Males | | | | | |
|---|---|---|---|---|---|---|---|---|---|
| | | | Age | 21-25 | | | 26-35 | | |
| | | | | S | M | A | S | M | A |
| Energy (kcal) | 1,600 | 3,000 | | 2400 | 2800 | 3000 | 2400 | 2600 | 3000 |
| Manganese (mg) | 2.3 | | AI | 2.3 | 2.3 | 2.3 | 2.3 | 2.3 | 2.3 |
| | | | UL | 11 | 11 | 11 | 11 | 11 | 11 |
| | | 5.9 | Maximum intake per 1600 kcal | 7.3 | 6.3 | 5.9 | 7.3 | 6.8 | 5.9 |
| Iodine (µg) | 150 | | RDA | 150 | 150 | 150 | 150 | 150 | 150 |
| | | | UL | 1100 | 1100 | 1100 | 1100 | 1100 | 1100 |
| | | 586.7 | Maximum intake per 1600 kcal | 733 | 629 | 587 | 733 | 677 | 587 |
| Selenium (µg) | 55 | | RDA | 55 | 55 | 55 | 55 | 55 | 55 |
| | | | UL | 400 | 400 | 400 | 400 | 400 | 400 |
| | | 213 | Maximum intake per 1600 kcal | 267 | 229 | 213 | 267 | 246 | 213 |
| Chromium (µg) | 35 | | AI | 35 | 35 | 35 | 35 | 35 | 35 |
| Molybdenum | 45 | | RDA | 45 | 45 | 45 | 45 | 45 | 45 |
| | | | UL | 2000 | 2000 | 2000 | 2000 | 2000 | 2000 |
| | | 1067 | Maximum intake per 1600 kcal | 1333 | 1143 | 1067 | 1333 | 1231 | 1067 |
| Nickel (mg) | | | UL | 1.0 | 1.0 | 1.0 | 1.0 | 1.0 | 1.0 |
| | | 0.53 | Maximum intake per 1600 kcal | 0.67 | 0.57 | 0.53 | 0.67 | 0.62 | 0.53 |
| Vanadium (mg) | | | UL | 1.8 | 1.8 | 1.8 | 1.8 | 1.8 | 1.8 |
| | | 0.96 | Maximum intake per 1600 kcal | 1.20 | 1.03 | 0.96 | 1.20 | 1.11 | 0.96 |
| Boron (mg) | | | UL | 20 | 20 | 20 | 20 | 20 | 20 |
| | | 10.7 | Maximum intake per 1600 kcal | 13.3 | 11.4 | 10.7 | 13.3 | 12.3 | 10.7 |
| Fluoride (mg) | 4 | | AI | 4 | 4 | 4 | 4 | 4 | 4 |
| | | | UL | 10 | 10 | 10 | 10 | 10 | 10 |
| | | 5.3 | Maximum intake per 1600 kcal | 6.7 | 5.7 | 5.3 | 6.7 | 6.2 | 5.3 |
| Chloride (g) | (2.3) | | AI | 2.3 | 2.3 | 2.3 | 2.3 | 2.3 | 2.3 |
| | | | UL | 3.6 | 3.6 | 3.6 | 3.6 | 3.6 | 3.6 |
| | | (1.9) | Maximum intake per 1600 kcal | 2.4 | 2.1 | 1.9 | 2.4 | 2.2 | 1.9 |

(FIG. 9 Continued)  (CONT.)

| 36–45 | | | 46–50 | | | 51–55 | | | 56–60 | | | 61–65 | | |
|---|---|---|---|---|---|---|---|---|---|---|---|---|---|---|
| S | M | A | S | M | A | S | M | A | S | M | A | S | M | A |
| 2400 | 2800 | 2800 | 2200 | 2400 | 2800 | 2200 | 2400 | 2800 | 2200 | 2400 | 2600 | 2000 | 2400 | 2800 |
| 2.3 | 2.3 | 2.3 | 2.3 | 2.3 | 2.3 | 2.3 | 2.3 | 2.3 | 2.3 | 2.3 | 2.3 | 2.3 | 2.3 | 2.3 |
| 11 | 11 | 11 | 11 | 11 | 11 | 11 | 11 | 11 | 11 | 11 | 11 | 11 | 11 | 11 |
| 7.3 | 6.3 | 6.3 | 8.0 | 7.3 | 6.3 | 8.0 | 7.3 | 6.3 | 8.0 | 7.3 | 6.8 | 8.8 | 7.3 | 6.3 |
| 150 | 150 | 150 | 150 | 150 | 150 | 150 | 150 | 150 | 150 | 150 | 150 | 150 | 150 | 150 |
| 1100 | 1100 | 1100 | 1100 | 1100 | 1100 | 1100 | 1100 | 1100 | 1100 | 1100 | 1100 | 1100 | 1100 | 1100 |
| 733 | 629 | 629 | 800 | 733 | 629 | 800 | 733 | 629 | 800 | 733 | 677 | 880 | 733 | 629 |
| 55 | 55 | 55 | 55 | 55 | 55 | 55 | 55 | 55 | 55 | 55 | 55 | 55 | 55 | 55 |
| 400 | 400 | 400 | 400 | 400 | 400 | 400 | 400 | 400 | 400 | 400 | 400 | 400 | 400 | 400 |
| 267 | 229 | 229 | 291 | 267 | 229 | 291 | 267 | 229 | 291 | 267 | 246 | 320 | 267 | 229 |
| 35 | 35 | 35 | 35 | 35 | 35 | 30 | 30 | 30 | 30 | 30 | 30 | 30 | 30 | 30 |
| 45 | 45 | 45 | 45 | 45 | 45 | 45 | 45 | 45 | 45 | 45 | 45 | 45 | 45 | 45 |
| 2000 | 2000 | 2000 | 2000 | 2000 | 2000 | 2000 | 2000 | 2000 | 2000 | 2000 | 2000 | 2000 | 2000 | 2000 |
| 1333 | 1143 | 1143 | 1455 | 1333 | 1143 | 1455 | 1333 | 1143 | 1455 | 1333 | 1231 | 1600 | 1333 | 1143 |
| 1.0 | 1.0 | 1.0 | 1.0 | 1.0 | 1.0 | 1.0 | 1.0 | 1.0 | 1.0 | 1.0 | 1.0 | 1.0 | 1.0 | 1.0 |
| 0.67 | 0.57 | 0.57 | 0.73 | 0.67 | 0.57 | 0.73 | 0.67 | 0.57 | 0.73 | 0.67 | 0.62 | 0.80 | 0.67 | 0.57 |
| 1.8 | 1.8 | 1.8 | 1.8 | 1.8 | 1.8 | 1.8 | 1.8 | 1.8 | 1.8 | 1.8 | 1.8 | 1.8 | 1.8 | 1.8 |
| 1.20 | 1.03 | 1.03 | 1.31 | 1.20 | 1.03 | 1.31 | 1.20 | 1.03 | 1.31 | 1.20 | 1.11 | 1.44 | 1.20 | 1.03 |
| 20 | 20 | 20 | 20 | 20 | 20 | 20 | 20 | 20 | 20 | 20 | 20 | 20 | 20 | 20 |
| 13.3 | 11.4 | 11.4 | 14.5 | 13.3 | 11.4 | 14.5 | 13.3 | 11.4 | 14.5 | 13.3 | 12.3 | 16.0 | 13.3 | 11.4 |
| 4 | 4 | 4 | 4 | 4 | 4 | 4 | 4 | 4 | 4 | 4 | 4 | 4 | 4 | 4 |
| 10 | 10 | 10 | 10 | 10 | 10 | 10 | 10 | 10 | 10 | 10 | 10 | 10 | 10 | 10 |
| 6.7 | 5.7 | 5.7 | 7.3 | 6.7 | 5.7 | 7.3 | 6.7 | 5.7 | 7.3 | 6.7 | 6.2 | 8.0 | 6.7 | 5.7 |
| 2.3 | 2.3 | 2.3 | 2.3 | 2.3 | 2.3 | 2.3 | 2.3 | 2.3 | 2.3 | 2.3 | 2.3 | 2.3 | 2.3 | 2.3 |
| 3.6 | 3.6 | 3.6 | 3.6 | 3.6 | 3.6 | 3.6 | 3.6 | 3.6 | 3.6 | 3.6 | 3.6 | 3.6 | 3.6 | 3.6 |
| 2.4 | 2.1 | 2.1 | 2.6 | 2.4 | 2.1 | 2.6 | 2.4 | 2.1 | 2.6 | 2.4 | 2.2 | 2.9 | 2.4 | 2.1 |

(FIG. 9 Continued)

| Females | | | | | | | | | | | | | | |
|---|---|---|---|---|---|---|---|---|---|---|---|---|---|---|
| 21–25 | | | 26–30 | | | 31–50 | | | 51–60 | | | 61–65 | | |
| S | M | A | S | M | A | S | M | A | S | M | A | S | M | A |
| 2000 | 2200 | 2400 | 1800 | 2000 | 2400 | 1800 | 2000 | 2200 | 1600 | 1800 | 2200 | 1600 | 1800 | 2000 |
| 1.8 | 1.8 | 1.8 | 1.8 | 1.8 | 1.8 | 1.8 | 1.8 | 1.8 | 1.8 | 1.8 | 1.8 | 1.8 | 1.8 | 1.8 |
| 11 | 11 | 11 | 11 | 11 | 11 | 11 | 11 | 11 | 11 | 11 | 11 | 11 | 11 | 11 |
| 8.8 | 8.0 | 7.3 | 9.8 | 8.8 | 7.3 | 9.8 | 8.8 | 8.0 | 11.0 | 9.8 | 8.0 | 11.0 | 9.8 | 8.8 |
| 150 | 150 | 150 | 150 | 150 | 150 | 150 | 150 | 150 | 150 | 150 | 150 | 150 | 150 | 150 |
| 1100 | 1100 | 1100 | 1100 | 1100 | 1100 | 1100 | 1100 | 1100 | 1100 | 1100 | 1100 | 1100 | 1100 | 1100 |
| 880 | 800 | 733 | 978 | 880 | 733 | 978 | 880 | 800 | 1100 | 978 | 800 | 1100 | 978 | 880 |
| 55 | 55 | 55 | 55 | 55 | 55 | 55 | 55 | 55 | 55 | 55 | 55 | 55 | 55 | 55 |
| 400 | 400 | 400 | 400 | 400 | 400 | 400 | 400 | 400 | 400 | 400 | 400 | 400 | 400 | 400 |
| 320 | 291 | 267 | 356 | 320 | 267 | 356 | 320 | 291 | 400 | 356 | 291 | 400 | 356 | 320 |
| 25 | 25 | 25 | 25 | 25 | 25 | 25 | 25 | 25 | 20 | 20 | 20 | 20 | 20 | 20 |
| 45 | 45 | 45 | 45 | 45 | 45 | 45 | 45 | 45 | 45 | 45 | 45 | 45 | 45 | 45 |
| 2000 | 2000 | 2000 | 2000 | 2000 | 2000 | 2000 | 2000 | 2000 | 2000 | 2000 | 2000 | 2000 | 2000 | 2000 |
| 1600 | 1455 | 1333 | 1778 | 1600 | 1333 | 1778 | 1600 | 1455 | 2000 | 1778 | 1455 | 2000 | 1778 | 1600 |
| 1.0 | 1.0 | 1.0 | 1.0 | 1.0 | 1.0 | 1.0 | 1.0 | 1.0 | 1.0 | 1.0 | 1.0 | 1.0 | 1.0 | 1.0 |
| 0.80 | 0.73 | 0.67 | 0.89 | 0.80 | 0.67 | 0.89 | 0.80 | 0.73 | 1.00 | 0.89 | 0.73 | 1.00 | 0.89 | 0.80 |
| 1.8 | 1.8 | 1.8 | 1.8 | 1.8 | 1.8 | 1.8 | 1.8 | 1.8 | 1.8 | 1.8 | 1.8 | 1.8 | 1.8 | 1.8 |
| 1.44 | 1.31 | 1.20 | 1.60 | 1.44 | 1.20 | 1.60 | 1.44 | 1.31 | 1.80 | 1.60 | 1.31 | 1.80 | 1.60 | 1.44 |
| 20 | 20 | 20 | 20 | 20 | 20 | 20 | 20 | 20 | 20 | 20 | 20 | 20 | 20 | 20 |
| 16.0 | 14.5 | 13.3 | 17.8 | 16.0 | 13.3 | 17.8 | 16.0 | 14.5 | 20.0 | 17.8 | 14.5 | 20.0 | 17.8 | 16.0 |
| 3 | 3 | 3 | 3 | 3 | 3 | 3 | 3 | 3 | 3 | 3 | 3 | 3 | 3 | 3 |
| 10 | 10 | 10 | 10 | 10 | 10 | 10 | 10 | 10 | 10 | 10 | 10 | 10 | 10 | 10 |
| 8.0 | 7.3 | 6.7 | 8.9 | 8.0 | 6.7 | 8.9 | 8.0 | 7.3 | 10.0 | 8.9 | 7.3 | 10.0 | 8.9 | 8.0 |
| 2.3 | 2.3 | 2.3 | 2.3 | 2.3 | 2.3 | 2.3 | 2.3 | 2.3 | 2.3 | 2.3 | 2.3 | 2.3 | 2.3 | 2.3 |
| 3.6 | 3.6 | 3.6 | 3.6 | 3.6 | 3.6 | 3.6 | 3.6 | 3.6 | 3.6 | 3.6 | 3.6 | 3.6 | 3.6 | 3.6 |
| 2.9 | 2.6 | 2.4 | 3.2 | 2.9 | 2.4 | 3.2 | 2.9 | 2.6 | 3.6 | 3.2 | 2.6 | 3.6 | 3.2 | 2.9 |

# FIG. 10

(CONT.)

| | Lower limit | Upper limit | Physical activity level | Males | | | | | |
|---|---|---|---|---|---|---|---|---|---|
| | | | Age | 21–25 | | | 26–35 | | |
| | | | | S | M | A | S | M | A |
| Energy (kcal) | 1,600 | 3,000 | | 2400 | 2800 | 3000 | 2400 | 2600 | 3000 |
| Vitamin A (µg) | 900 | | RDA | 900 | 900 | 900 | 900 | 900 | 900 |
| | | | UL | 3000 | 3000 | 3000 | 3000 | 3000 | 3000 |
| | | 1600 | Maximum intake per 1600 kcal | 2000 | 1714 | 1600 | 2000 | 1846 | 1600 |
| Vitamin D (µg) | 15.0 | | RDA | 15 | 15 | 15 | 15 | 15 | 15 |
| | | | UL | 100 | 100 | 100 | 100 | 100 | 100 |
| | | 53.3 | Maximum intake per 1600 kcal | 66.67 | 57.14 | 53.33 | 66.67 | 61.54 | 53.33 |
| Vitamin E (mg) | 15.0 | | RDA | 15.0 | 15.0 | 15.0 | 15.0 | 15.0 | 15.0 |
| Vitamin K (µg) | 120 | | AI | 120 | 120 | 120 | 120 | 120 | 120 |
| Vitamin B1 (mg) | 1.2 | | RDA | 1.2 | 1.2 | 1.2 | 1.2 | 1.2 | 1.2 |
| Vitamin B2 (mg) | 1.3 | | RDA | 1.3 | 1.3 | 1.3 | 1.3 | 1.3 | 1.3 |
| Niacin (mg) | 16 | | RDA | 16 | 16 | 16 | 16 | 16 | 16 |
| Vitamin B6 (mg) | 1.7 | | RDA | 1.3 | 1.3 | 1.3 | 1.3 | 1.3 | 1.3 |
| | | | UL | 100 | 100 | 100 | 100 | 100 | 100 |
| | | 53.3 | Maximum intake per 1600 kcal | 66.7 | 57.1 | 53.3 | 66.7 | 61.5 | 53.3 |
| Vitamin B12 (µg) | 2.4 | | RDA | 2.4 | 2.4 | 2.4 | 2.4 | 2.4 | 2.4 |
| Folic acid (µg) | 400 | | RDA | 400 | 400 | 400 | 400 | 400 | 400 |
| Pantothenic acid (mg) | 5 | | AI | 5 | 5 | 5 | 5 | 5 | 5 |
| Biotin (µg) | 30 | | RDA | 30 | 30 | 30 | 30 | 30 | 30 |
| Vitamin C (mg) | 90 | | RDA | 90 | 90 | 90 | 90 | 90 | 90 |
| | | | UL | 2000 | 2000 | 2000 | 2000 | 2000 | 2000 |
| | | 1067 | Maximum intake per 1600 kcal | 1333 | 1143 | 1067 | 1333 | 1231 | 1067 |
| Choline (mg) | 550 | | AI | 550 | 550 | 550 | 550 | 550 | 550 |
| | | | UL | 3500 | 3500 | 3500 | 3500 | 3500 | 3500 |
| | | 1867 | Maximum intake per 1600 kcal | 2333 | 2000 | 1867 | 2333 | 2154 | 1867 |

(FIG. 10 Continued)                                                    (CONT.)

| 36−45 | | | 46−50 | | | 51−55 | | | 56−60 | | | 61−65 | | |
|---|---|---|---|---|---|---|---|---|---|---|---|---|---|---|
| S | M | A | S | M | A | S | M | A | S | M | A | S | M | A |
| 2400 | 2800 | 2800 | 2200 | 2400 | 2800 | 2200 | 2400 | 2800 | 2200 | 2400 | 2600 | 2000 | 2400 | 2800 |
| 900 | 900 | 900 | 900 | 900 | 900 | 900 | 900 | 900 | 900 | 900 | 900 | 900 | 900 | 900 |
| 3000 | 3000 | 3000 | 3000 | 3000 | 3000 | 3000 | 3000 | 3000 | 3000 | 3000 | 3000 | 3000 | 3000 | 3000 |
| 2000 | 1714 | 1714 | 2182 | 2000 | 1714 | 2182 | 2000 | 1714 | 2182 | 2000 | 1846 | 2400 | 2000 | 1714 |
| 15 | 15 | 15 | 15 | 15 | 15 | 15 | 15 | 15 | 15 | 15 | 15 | 15 | 15 | 15 |
| 100 | 100 | 100 | 100 | 100 | 100 | 100 | 100 | 100 | 100 | 100 | 100 | 100 | 100 | 100 |
| 66.67 | 57.14 | 57.14 | 72.73 | 66.67 | 57.14 | 72.73 | 66.67 | 57.14 | 72.73 | 66.67 | 61.54 | 80 | 66.67 | 57.14 |
| 15.0 | 15.0 | 15.0 | 15.0 | 15.0 | 15.0 | 15.0 | 15.0 | 15.0 | 15.0 | 15.0 | 15.0 | 15.0 | 15.0 | 15.0 |
| 120 | 120 | 120 | 120 | 120 | 120 | 120 | 120 | 120 | 120 | 120 | 120 | 120 | 120 | 120 |
| 1.2 | 1.2 | 1.2 | 1.2 | 1.2 | 1.2 | 1.2 | 1.2 | 1.2 | 1.2 | 1.2 | 1.2 | 1.2 | 1.2 | 1.2 |
| 1.3 | 1.3 | 1.3 | 1.3 | 1.3 | 1.3 | 1.3 | 1.3 | 1.3 | 1.3 | 1.3 | 1.3 | 1.3 | 1.3 | 1.3 |
| 16 | 16 | 16 | 16 | 16 | 16 | 16 | 16 | 16 | 16 | 16 | 16 | 16 | 16 | 16 |
| 1.3 | 1.3 | 1.3 | 1.3 | 1.3 | 1.3 | 1.7 | 1.7 | 1.7 | 1.7 | 1.7 | 1.7 | 1.7 | 1.7 | 1.7 |
| 100 | 100 | 100 | 100 | 100 | 100 | 100 | 100 | 100 | 100 | 100 | 100 | 100 | 100 | 100 |
| 66.7 | 57.1 | 57.1 | 72.7 | 66.7 | 57.1 | 72.7 | 66.7 | 57.1 | 72.7 | 66.7 | 61.5 | 80.0 | 66.7 | 57.1 |
| 2.4 | 2.4 | 2.4 | 2.4 | 2.4 | 2.4 | 2.4 | 2.4 | 2.4 | 2.4 | 2.4 | 2.4 | 2.4 | 2.4 | 2.4 |
| 400 | 400 | 400 | 400 | 400 | 400 | 400 | 400 | 400 | 400 | 400 | 400 | 400 | 400 | 400 |
| 5 | 5 | 5 | 5 | 5 | 5 | 5 | 5 | 5 | 5 | 5 | 5 | 5 | 5 | 5 |
| 30 | 30 | 30 | 30 | 30 | 30 | 30 | 30 | 30 | 30 | 30 | 30 | 30 | 30 | 30 |
| 90 | 90 | 90 | 90 | 90 | 90 | 90 | 90 | 90 | 90 | 90 | 90 | 90 | 90 | 90 |
| 2000 | 2000 | 2000 | 2000 | 2000 | 2000 | 2000 | 2000 | 2000 | 2000 | 2000 | 2000 | 2000 | 2000 | 2000 |
| 1333 | 1143 | 1143 | 1455 | 1333 | 1143 | 1455 | 1333 | 1143 | 1455 | 1333 | 1231 | 1600 | 1333 | 1143 |
| 550 | 550 | 550 | 550 | 550 | 550 | 550 | 550 | 550 | 550 | 550 | 550 | 550 | 550 | 550 |
| 3500 | 3500 | 3500 | 3500 | 3500 | 3500 | 3500 | 3500 | 3500 | 3500 | 3500 | 3500 | 3500 | 3500 | 3500 |
| 2333 | 2000 | 2000 | 2545 | 2333 | 2000 | 2545 | 2333 | 2000 | 2545 | 2333 | 2154 | 2800 | 2333 | 2000 |

(FIG. 10 Continued)

| Females | | | | | | | | | | | | | | |
|---|---|---|---|---|---|---|---|---|---|---|---|---|---|---|
| 21-25 | | | 26-30 | | | 31-50 | | | 51-60 | | | 61-65 | | |
| S | M | A | S | M | A | S | M | A | S | M | A | S | M | A |
| 2000 | 2200 | 2400 | 1800 | 2000 | 2400 | 1800 | 2000 | 2200 | 1600 | 1800 | 2200 | 1600 | 1800 | 2000 |
| 700 | 700 | 700 | 700 | 700 | 700 | 700 | 700 | 700 | 700 | 700 | 700 | 700 | 700 | 700 |
| 3000 | 3000 | 3000 | 3000 | 3000 | 3000 | 3000 | 3000 | 3000 | 3000 | 3000 | 3000 | 3000 | 3000 | 3000 |
| 2400 | 2182 | 2000 | 2667 | 2400 | 2000 | 2667 | 2400 | 2182 | 3000 | 2667 | 2182 | 3000 | 2667 | 2400 |
| 15 | 15 | 15 | 15 | 15 | 15 | 15 | 15 | 15 | 15 | 15 | 15 | 15 | 15 | 15 |
| 100 | 100 | 100 | 100 | 100 | 100 | 100 | 100 | 100 | 100 | 100 | 100 | 100 | 100 | 100 |
| 80 | 72.73 | 66.67 | 88.89 | 80 | 66.67 | 88.89 | 80 | 72.73 | 100 | 88.89 | 72.73 | 100 | 88.89 | 80 |
| 15.0 | 15.0 | 15.0 | 15.0 | 15.0 | 15.0 | 15.0 | 15.0 | 15.0 | 15.0 | 15.0 | 15.0 | 15.0 | 15.0 | 15.0 |
| 90 | 90 | 90 | 90 | 90 | 90 | 90 | 90 | 90 | 90 | 90 | 90 | 90 | 90 | 90 |
| 1.1 | 1.1 | 1.1 | 1.1 | 1.1 | 1.1 | 1.1 | 1.1 | 1.1 | 1.1 | 1.1 | 1.1 | 1.1 | 1.1 | 1.1 |
| 1.1 | 1.1 | 1.1 | 1.1 | 1.1 | 1.1 | 1.1 | 1.1 | 1.1 | 1.1 | 1.1 | 1.1 | 1.1 | 1.1 | 1.1 |
| 14 | 14 | 14 | 14 | 14 | 14 | 14 | 14 | 14 | 14 | 14 | 14 | 14 | 14 | 14 |
| 1.3 | 1.3 | 1.3 | 1.3 | 1.3 | 1.3 | 1.3 | 1.3 | 1.3 | 1.5 | 1.5 | 1.5 | 1.5 | 1.5 | 1.5 |
| 100 | 100 | 100 | 100 | 100 | 100 | 100 | 100 | 100 | 100 | 100 | 100 | 100 | 100 | 100 |
| 80.0 | 72.7 | 66.7 | 88.9 | 80.0 | 66.7 | 88.9 | 80.0 | 72.7 | 100.0 | 88.9 | 72.7 | 100.0 | 88.9 | 80.0 |
| 2.4 | 2.4 | 2.4 | 2.4 | 2.4 | 2.4 | 2.4 | 2.4 | 2.4 | 2.4 | 2.4 | 2.4 | 2.4 | 2.4 | 2.4 |
| 400 | 400 | 400 | 400 | 400 | 400 | 400 | 400 | 400 | 400 | 400 | 400 | 400 | 400 | 400 |
| 5 | 5 | 5 | 5 | 5 | 5 | 5 | 5 | 5 | 5 | 5 | 5 | 5 | 5 | 5 |
| 30 | 30 | 30 | 30 | 30 | 30 | 30 | 30 | 30 | 30 | 30 | 30 | 30 | 30 | 30 |
| 75 | 75 | 75 | 75 | 75 | 75 | 75 | 75 | 75 | 75 | 75 | 75 | 75 | 75 | 75 |
| 2000 | 2000 | 2000 | 2000 | 2000 | 2000 | 2000 | 2000 | 2000 | 2000 | 2000 | 2000 | 2000 | 2000 | 2000 |
| 1600 | 1455 | 1333 | 1778 | 1600 | 1333 | 1778 | 1600 | 1455 | 2000 | 1778 | 1455 | 2000 | 1778 | 1600 |
| 425 | 425 | 425 | 425 | 425 | 425 | 425 | 425 | 425 | 425 | 425 | 425 | 425 | 425 | 425 |
| 3500 | 3500 | 3500 | 3500 | 3500 | 3500 | 3500 | 3500 | 3500 | 3500 | 3500 | 3500 | 3500 | 3500 | 3500 |
| 2800 | 2545 | 2333 | 3111 | 2800 | 2333 | 3111 | 2800 | 2545 | 3500 | 3111 | 2545 | 3500 | 3111 | 2800 |

## FIG. 11

| | Lower limit | Upper limit | Age | Males | | | | | | Females | | | | | |
|---|---|---|---|---|---|---|---|---|---|---|---|---|---|---|---|
| | | | | 18–49 | | | 50–64 | | | 18–49 | | | 50–64 | | |
| | | | Physical activity level | I | II | III | I | II | III | I | II | III | I | II | III |
| Energy (kcal) | 1,750 | 3,000 | | 2250 | 2600 | 3000 | 2100 | 2450 | 2800 | 1800 | 2100 | 2400 | 1750 | 2050 | 2350 |
| Protein (g) | 65.0 | | RDA | 65.0 | 65.0 | 65.0 | 65.0 | 65.0 | 65.0 | 55.0 | 55.0 | 55.0 | 55.0 | 55.0 | 55.0 |
| Lipid (g) | 38.9 | | Lower limit of DG | 50.0 | 57.8 | 66.7 | 46.7 | 54.4 | 62.2 | 40.0 | 46.7 | 53.3 | 38.9 | 45.6 | 52.2 |
| | | 58.3 | Upper limit of DG | 75.0 | 86.7 | 100.0 | 70.0 | 81.7 | 93.3 | 60.0 | 70.0 | 80.0 | 58.3 | 68.3 | 78.3 |
| Saturated fatty acid (g) | | 19.4 | DG (or less) | 25.0 | 28.9 | 33.3 | 23.3 | 27.2 | 31.1 | 20.0 | 23.3 | 26.7 | 19.4 | 22.8 | 26.1 |
| α-Linolenic acid | 2.0 | | RDA | 1.5 | 1.7 | 2.0 | 1.4 | 1.6 | 1.9 | 1.2 | 1.4 | 1.6 | 1.2 | 1.4 | 1.6 |
| Linoleic acid | 13.3 | | RDA | 10.0 | 11.6 | 13.3 | 9.3 | 10.9 | 12.4 | 8.0 | 9.3 | 10.7 | 7.8 | 9.1 | 10.4 |
| Carbohydrate (g) | 218.8 | | Lower limit of DG | 281.3 | 325.0 | 375.0 | 262.5 | 306.3 | 350.0 | 225.0 | 262.5 | 300.0 | 218.8 | 256.3 | 293.8 |
| | | 284.4 | Upper limit of DG | 365.6 | 422.5 | 487.5 | 341.3 | 398.1 | 455.0 | 292.5 | 341.3 | 390.0 | 284.4 | 333.1 | 381.9 |
| Added sugar (g) | 75 | 43.75 | DG | 56.3 | 65.0 | 75.0 | 52.5 | 61.3 | 70.0 | 45.0 | 52.5 | 60.0 | 43.8 | 51.3 | 58.8 |

EP 4 134 968 A1

## FIG. 12

| | Lower limit | Upper limit | Physical activity level | Males 18–49 I | II | III | Males 50–64 I | II | III | Females 18–49 I | II | III | Females 50–64 I | II | III |
|---|---|---|---|---|---|---|---|---|---|---|---|---|---|---|---|
| Energy (kcal) | 1,750 | 3,000 | | 2250 | 2600 | 3000 | 2100 | 2450 | 2800 | 1800 | 2100 | 2400 | 1750 | 2050 | 2350 |
| Sodium (mg) | 1500.0 | | RDA | 1500 | 1500 | 1500 | 1400 | 1400 | 1400 | 1500 | 1500 | 1500 | 1400 | 1400 | 1400 |
| Potassium (mg) | 2000 | | RDA | 2000 | 2000 | 2000 | 2000 | 2000 | 2000 | 2000 | 2000 | 2000 | 2000 | 2000 | 2000 |
| Calcium (mg) | 1000 | | RDA | 800 | 800 | 800 | 1000 | 1000 | 1000 | 800 | 800 | 800 | 1000 | 1000 | 1000 |
| | | | UL | 2000 | 2000 | 2000 | 2000 | 2000 | 2000 | 2000 | 2000 | 2000 | 2000 | 2000 | 2000 |
| | | 1167 | Maximum intake per 1750 kcal | 1556 | 1346 | 1167 | 1667 | 1429 | 1250 | 1944 | 1667 | 1458 | 2000 | 1707 | 1489 |
| Magnesium (mg) | 330 | | RDA | 330 | 330 | 330 | 330 | 330 | 330 | 330 | 330 | 330 | 330 | 330 | 330 |
| Phosphorus (mg) | 720 | | RDA | 720 | 720 | 720 | 720 | 720 | 720 | 720 | 720 | 720 | 720 | 720 | 720 |
| | | | UL | 3500 | 3500 | 3500 | 3500 | 3500 | 3500 | 3500 | 3500 | 3500 | 3500 | 3500 | 3500 |
| | | 2042 | Maximum intake per 1750 kcal | 2722 | 2356 | 2042 | 2917 | 2500 | 2188 | 3403 | 2917 | 2552 | 3500 | 2988 | 2606 |
| Iron (mg) | 20.0 | | RDA (menstruation for some females) | 12.0 | 12.0 | 12.0 | 12.0 | 12.0 | 12.0 | 20.0 | 20.0 | 20.0 | 12.0 | 12.0 | 12.0 |
| | | | UL | 42 | 42 | 42 | 42 | 42 | 42 | 42 | 42 | 42 | 42 | 42 | 42 |
| | | 24.5 | Maximum intake per 1750 kcal | 32.7 | 28.3 | 24.5 | 35.0 | 30.0 | 26.3 | 40.8 | 35.0 | 30.6 | 42.0 | 35.9 | 31.3 |
| Zinc (mg) | 12.5 | | RDA | 12.5 | 12.5 | 12.5 | 12.5 | 12.5 | 12.5 | 7.5 | 7.5 | 7.5 | 7.5 | 7.5 | 7.5 |
| | | | UL | 40 | 40 | 40 | 40 | 40 | 40 | 40 | 40 | 40 | 40 | 40 | 40 |
| | | 23.3 | Maximum intake per 1750 kcal | 31.1 | 26.9 | 23.3 | 33.3 | 28.6 | 25.0 | 38.9 | 33.3 | 29.2 | 40.0 | 34.1 | 29.8 |
| Copper (mg) | 0.8 | | RDA | 0.8 | 0.8 | 0.8 | 0.8 | 0.8 | 0.8 | 0.8 | 0.8 | 0.8 | 0.8 | 0.8 | 0.8 |
| | | | UL | 8 | 8 | 8 | 8 | 8 | 8 | 8 | 8 | 8 | 8 | 8 | 8 |
| | | 4.7 | Maximum intake per 1750 kcal | 6.2 | 5.4 | 4.7 | 6.7 | 5.7 | 5.0 | 7.8 | 6.7 | 5.8 | 8.0 | 6.8 | 6.0 |

# FIG. 13

| | Lower limit | Upper limit | Physical activity level | Males | | | | | | Females | | | | | |
|---|---|---|---|---|---|---|---|---|---|---|---|---|---|---|---|
| | | | Age | 18–49 | | | 50–64 | | | 18–49 | | | 50–64 | | |
| | | | | I | II | III | I | II | III | I | II | III | I | II | III |
| Energy (kcal) | 1,750 | 3,000 | | 2250 | 2600 | 3000 | 2100 | 2450 | 2800 | 1800 | 2100 | 2400 | 1750 | 2050 | 2350 |
| Manganese (mg) | 4.5 | | RDA | 4.5 | 4.5 | 4.5 | 4.5 | 4.5 | 4.5 | 4.5 | 4.5 | 4.5 | 4.5 | 4.5 | 4.5 |
| | | | UL | 11 | 11 | 11 | 11 | 11 | 11 | 11 | 11 | 11 | 11 | 11 | 11 |
| | | 6.4 | Maximum intake per 1750 kcal | 8.6 | 7.4 | 6.4 | 9.2 | 7.9 | 6.9 | 10.7 | 9.2 | 8.0 | 11.0 | 9.4 | 8.2 |
| Iodine (µg) | 120 | | RDA | 120 | 120 | 120 | 120 | 120 | 120 | 120 | 120 | 120 | 120 | 120 | 120 |
| | | | UL | 600 | 600 | 600 | 600 | 600 | 600 | 600 | 600 | 600 | 600 | 600 | 600 |
| | | 350.0 | Maximum intake per 1750 kcal | 466.7 | 403.8 | 350 | 500 | 428.6 | 375 | 583.3 | 500 | 437.5 | 600 | 512.2 | 446.8 |
| Selenium (µg) | 60 | | RDA | 60 | 60 | 60 | 60 | 60 | 60 | 60 | 60 | 60 | 60 | 60 | 60 |
| | | | UL | 400 | 400 | 400 | 400 | 400 | 400 | 400 | 400 | 400 | 400 | 400 | 400 |
| | | 233 | Maximum intake per 1750 kcal | 311 | 269 | 233 | 333 | 286 | 250 | 389 | 333 | 292 | 400 | 341 | 298 |
| Chromiumv (µg) | 30 | | RDA | 30 | 30 | 30 | 30 | 30 | 30 | 30 | 30 | 30 | 30 | 30 | 30 |
| Molybdenum (µg) | 100 | | RDA | 100 | 100 | 100 | 100 | 100 | 100 | 100 | 100 | 100 | 100 | 100 | 100 |
| | | | UL | 900 | 900 | 900 | 900 | 900 | 900 | 900 | 900 | 900 | 900 | 900 | 900 |
| | | 525.0 | Maximum intake per 1750 kcal | 700 | 605.8 | 525 | 750 | 642.9 | 562.5 | 875 | 750 | 656.3 | 900 | 768.3 | 670.2 |
| Fluoride (mg) | 1.5 | | RDA | 1.5 | 1.5 | 1.5 | 1.5 | 1.5 | 1.5 | 1.5 | 1.5 | 1.5 | 1.5 | 1.5 | 1.5 |
| | | | UL | 3.5 | 3.5 | 3.5 | 3.5 | 3.5 | 3.5 | 3.5 | 3.5 | 3.5 | 3.5 | 3.5 | 3.5 |
| | | 2.0 | Maximum intake per 1750 kcal | 2.72 | 2.36 | 2.04 | 2.92 | 2.50 | 2.19 | 3.40 | 2.92 | 2.55 | 3.50 | 2.99 | 2.61 |
| Chloride (mg) | 2300 | | RDA | 2300 | 2300 | 2300 | 2200 | 2200 | 2200 | 2300 | 2300 | 2300 | 2200 | 2200 | 2200 |

EP 4 134 968 A1

## FIG. 14

| | | | | Males | |
|---|---|---|---|---|---|
| | | | Age | | 18-49 |
| | Lower limit | Upper limit | Physical activity level | I | II |
| Energy (kcal) | 1,750 | 3,000 | | 2250 | 2600 |
| Vitamin A (μg) | 800 | | RDA | 800 | 800 |
| | | | UL | 3000 | 3000 |
| | | 1750 | Maximum intake per 1750 kcal | 2333 | 2019 |
| Vitamin D (μg) | 10.0 | | RDA | 10 | 10 |
| | | | UL | 50 | 50 |
| | | 29.2 | Maximum intake per 1750 kcal | 38.9 | 33.7 |
| Vitamin E (mg) | 14.0 | | RDA | 14 | 14 |
| | | | UL | 700 | 700 |
| | | 408.3 | Maximum intake per 1750 kcal | 544.4 | 471.2 |
| Vitamin K (μg) | 80 | | RDA | 80 | 80 |
| Vitamin B1 (mg) | 1.4 | | RDA | 1.4 | 1.4 |
| Vitamin B2 (mg) | 1.4 | | RDA | 1.4 | 1.4 |
| Niacin (mg) | 15 | | RDA | 15 | 15 |
| | | | UL | 310 | 310 |
| | | 180.8 | Maximum intake per 1750 kcal | 241.1 | |
| Vitamin B6 (mg) | 1.6 | | RDA | 1.4 | 1.4 |
| | | | UL | 60 | 60 |
| | | 35.0 | Maximum intake per 1750 kcal | 46.7 | 40.4 |
| Vitamin B12 (μg) | 2.4 | | RDA | 2.4 | 2.4 |
| Folic acid (μg) | 400 | | RDA | 400 | 400 |
| Pantothenic acid (mg) | 5 | | RDA | 5 | 5 |
| Biotin (μg) | 40 | | RDA | 40 | 40 |
| Vitamin C (mg) | 100 | | RDA | 100 | 100 |
| | | | UL | 2000 | 2000 |
| | | 1167 | Maximum intake per 1750 kcal | 1556 | 1346 |
| Choline (mg) | 500 | | RDA | 500 | 500 |
| | | | UL | 3000 | 3000 |
| | | 1750 | Maximum intake per 1750 kcal | 2333 | 2019 |

| (FIG. 14 Continued)

| Females | | | | | | | | | |
|---|---|---|---|---|---|---|---|---|---|
| 50–64 | | | | 18–49 | | | 50–64 | | |
| III | I | II | III | I | II | III | I | II | III |
| 3000 | 2100 | 2450 | 2800 | 1800 | 2100 | 2400 | 1750 | 2050 | 2350 |
| 800 | 800 | 800 | 800 | 700 | 700 | 700 | 700 | 700 | 700 |
| 3000 | 3000 | 3000 | 3000 | 3000 | 3000 | 3000 | 3000 | 3000 | 3000 |
| 1750 | 2500 | 2143 | 1875 | 2917 | 2500 | 2188 | 3000 | 2561 | 2234 |
| 10 | 10 | 10 | 10 | 10 | 10 | 10 | 10 | 10 | 10 |
| 50 | 50 | 50 | 50 | 50 | 50 | 50 | 50 | 50 | 50 |
| 29.2 | 41.7 | 35.7 | 31.3 | 48.6 | 41.7 | 36.5 | 50.0 | 42.7 | 37.2 |
| 14 | 14 | 14 | 14 | 14 | 14 | 14 | 14 | 14 | 14 |
| 700 | 700 | 700 | 700 | 700 | 700 | 700 | 700 | 700 | 700 |
| 408.3 | 583.3 | 500 | 437.5 | 680.6 | 583.3 | 510.4 | 700 | 597.6 | 521.3 |
| 80 | 80 | 80 | 80 | 80 | 80 | 80 | 80 | 80 | 80 |
| 1.4 | 1.4 | 1.4 | 1.4 | 1.2 | 1.2 | 1.2 | 1.2 | 1.2 | 1.2 |
| 1.4 | 1.4 | 1.4 | 1.4 | 1.2 | 1.2 | 1.2 | 1.2 | 1.2 | 1.2 |
| 15 | 14 | 14 | 14 | 12 | 12 | 12 | 12 | 12 | 12 |
| 310 | 310 | 310 | 310 | 310 | 310 | 310 | 310 | 310 | 310 |
| 180.8 | 258.3 | 221.4 | 193.8 | 301.4 | 258.3 | 226.0 | 310.0 | 264.6 | 230.9 |
| 1.4 | 1.6 | 1.6 | 1.6 | 1.4 | 1.4 | 1.4 | 1.6 | 1.6 | 1.6 |
| 60 | 60 | 60 | 60 | 60 | 60 | 60 | 60 | 60 | 60 |
| 35.0 | 50.0 | 42.9 | 37.5 | 58.3 | 50.0 | 43.8 | 60.0 | 51.2 | 44.7 |
| 2.4 | 2.4 | 2.4 | 2.4 | 2.4 | 2.4 | 2.4 | 2.4 | 2.4 | 2.4 |
| 400 | 400 | 400 | 400 | 400 | 400 | 400 | 400 | 400 | 400 |
| 5 | 5 | 5 | 5 | 5 | 5 | 5 | 5 | 5 | 5 |
| 40 | 40 | 40 | 40 | 40 | 40 | 40 | 40 | 40 | 40 |
| 100 | 100 | 100 | 100 | 100 | 100 | 100 | 100 | 100 | 100 |
| 2000 | 2000 | 2000 | 2000 | 2000 | 2000 | 2000 | 2000 | 2000 | 2000 |
| 1167 | 1667 | 1429 | 1250 | 1944 | 1667 | 1458 | 2000 | 1707 | 1489 |
| 500 | 500 | 500 | 500 | 400 | 400 | 400 | 400 | 400 | 400 |
| 3000 | 3000 | 3000 | 3000 | 3000 | 3000 | 3000 | 3000 | 3000 | 3000 |
| 1750 | 2500 | 2143 | 1875 | 2917 | 2500 | 2188 | 3000 | 2561 | 2234 |

## FIG. 15

| Whole (n=75) | Test timing | Average | Standard deviation | P value |
|---|---|---|---|---|
| Weight (kg) | Before intake period | 66.7 | 11.83 | <0.001** |
| | After intake period | 66.0 | 11.48 | |
| BMI (kg/m$^2$) | Before intake period | 22.7 | 2.79 | <0.001** |
| | After intake period | 22.4 | 2.71 | |
| Body fat percentage (%) | Before intake period | 21.6 | 6.20 | 0.0017** |
| | After intake period | 21.2 | 6.00 | |

## FIG. 16

| | Test timing | Average | Standard deviation | P value |
|---|---|---|---|---|
| Whole (mg/dL) (n=75) | Before intake period | 102.5 | 76.8 | 0.0169* |
| | After intake period | 90.2 | 61.5 | |
| 150 or higher (mg/dL) (n=15) | Before intake period | 234.6 | 68.9 | 0.0326* |
| | After intake period | 186.5 | 66.0 | |
| 120 or higher (mg/dL) (n=19) | Before intake period | 212.3 | 75.3 | 0.0220* |
| | After intake period | 171.2 | 66.5 | |

## FIG. 17

| Test timing | Average | Standard deviation | P value |
|---|---|---|---|
| Before intake period | 0.00006 | 0.00002 | <0.001** |
| After intake period | 0.00005 | 0.00001 | |

## FIG. 18

| | | Test timing | Average | Standard deviation | P value |
|---|---|---|---|---|---|
| Whole (n=75) | Systolic blood pressure (mmHg) (SBP) | Before intake period | 115.8 | 12.36 | 0.2565 |
| | | After intakev period | 116.8 | 12.03 | |
| | Diastolic blood pressure (mmHg) (DBP) | Before intake period | 69.3 | 10.92 | 0.2356 |
| | | After intakev period | 70.1 | 9.15 | |
| High blood pressure or higher (SBP 130 or higher and/or DBP 80 or higher) (n=13) | Systolic blood pressure (mmHg) (SBP) | Before intake period | 134.8 | 8.48 | 0.0772 |
| | | After intakev period | 130.5 | 6.51 | |
| | Diastolic blood pressure (mmHg) (DBP) | Before intake period | 86.7 | 6.09 | 0.0040** |
| | | After intakev period | 82.0 | 5.65 | |

## FIG. 19

| | Test timing | Average | Standard deviation | P value |
|---|---|---|---|---|
| Whole (n=75) | Before intake period | 2.954 | 0.3294 | 0.0768 |
| | After intakev period | 3.001 | 0.3535 | |
| Male (n=62) | Before intake period | 2.978 | 0.3442 | 0.0385* |
| | After intakev period | 3.041 | 0.3653 | |

## FIG. 20

| Whole (n=75) | Test timing | Average | Standard deviation | P value |
|---|---|---|---|---|
| Bifidobacterium (occupancy %) | Before intake period | 2.7 | 2.5 | 0.0112* |
| | After intakev period | 3.4 | 3.0 | |
| Akkermansia (occupancy % | Before intake period | 0.01 | 0.02 | 0.0284* |
| | After intakev period | 0.02 | 0.05 | |
| Phylum Fusobacteria (occupancy %) | Before intake period | 1.0 | 3.2 | 0.0672 |
| | After intakev period | 0.7 | 2.3 | |
| Diversity | Before intake period | 6.0 | 0.5 | 0.0048** |
| | After intakev period | 6.1 | 0.5 | |

## INTERNATIONAL SEARCH REPORT

International application No.

PCT/JP2021/012053

A. CLASSIFICATION OF SUBJECT MATTER
G16H 20/60(2018.01)i; A23L 33/00(2016.01)i; A23L 33/10(2016.01)i
FI: G16H20/60; A23L33/10; A23L33/00

According to International Patent Classification (IPC) or to both national classification and IPC

B. FIELDS SEARCHED

Minimum documentation searched (classification system followed by classification symbols)
G06Q10/00-99/00; G16H10/00-80/00; A23L33/00; A23L33/10

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y<br>A | JP 2013-134650 A (YOSHIKAWA, Hiroyuki) 08 July 2013 (2013-07-08) paragraphs [0006], [0027]-[0030], [0033]-[0034], [0042]-[0046], [0049], [0055], [0059]-[0071], fig. 1-5, 12 | 1-6, 12-20<br>7-11 |
| Y<br>A | JP 2019-140952 A (COMP INC.) 29 August 2019 (2019-08-29) paragraphs [0011]-[0012], [0016]-[0032], table 1 | 1-6, 12-20<br>7-11 |
| Y<br>A | WO 2015/001595 A1 (HITACHI, LTD.) 08 January 2015 (2015-01-08) paragraphs [0013], [0015], [0017], [0042], [0046] | 15, 18<br>7-11 |
| A | JP 2009-159873 A (NISSIN FOODS HOLDINGS CO., LTD.) 23 January 2009 (2009-01-23) paragraphs [0018]-[0020], [0023]-[0027], [0030] | 1-20 |

☒ Further documents are listed in the continuation of Box C.    ☒ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 15 June 2021 (15.06.2021) | 22 June 2021 (22.06.2021) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

## INTERNATIONAL SEARCH REPORT

International application No.

PCT/JP2021/012053

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2012-203834 A (NTT DATA CORPORATION) 22 October 2012 (2012-10-22) paragraphs [0059]-[0064] | 1-20 |

Form PCT/ISA/210 (continuation of second sheet) (January 2015)

**INTERNATIONAL SEARCH REPORT**
Information on patent family members

International application No.

PCT/JP2021/012053

| Patent Documents referred in the Report | Publication Date | Patent Family | Publication Date |
|---|---|---|---|
| JP 2013-134650 A | 08 Jul. 2013 | (Family: none) | |
| JP 2019-140952 A | 29 Aug. 2019 | (Family: none) | |
| WO 2015/001595 A1 | 08 Jan. 2015 | GB 2529593 A paragraphs [0013], [0015], [0017], [0042], [0046] | |
| JP 2009-159873 A | 23 Jul. 2009 | (Family: none) | |
| JP 2012-203834 A | 22 Oct. 2012 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019140952 A

**Non-patent literature cited in the description**

- Development Study Group Report, the "Dietary Reference Intakes (DRIs) for Japanese. 2020
- Japanese DRIs. Ministry of Health, Labour and Welfare, 2020
- Dietary Reference Intakes (DRIs). Food and Nutrition Board of the Institute of Medicine
- Dietary Reference Values for nutrients. European Food Safety Authority, 2017
- Dietary Reference Intakes for Chinese. Chinese Nutrition Society, 2013
- Dietary Reference Intakes for Koreans. Ministry of Health and Welfare and The Korean Nutrition Society, 2015
- Dietary Guidelines for Indians. National Institute of Nutrition, 2011
- Nutritional Requirements during Spaceflight and on Earth. *Aerospace Environmental Medicine,* 2008, vol. 45 (3), 75-97